(19) Europäisches Patentamt
European Patent Office
Office européen des brevets

(11) **EP 2 260 054 B1**

(12) **EUROPEAN PATENT SPECIFICATION**

(45) Date of publication and mention of the grant of the patent:
**03.06.2015 Bulletin 2015/23**

(51) Int Cl.:
**C07K 14/715** *(2006.01)*    **C07K 1/20** *(2006.01)*
**C12N 15/62** *(2006.01)*    **C07K 1/16** *(2006.01)*

(21) Application number: **09716313.3**

(22) Date of filing: **27.02.2009**

(86) International application number:
**PCT/US2009/035581**

(87) International publication number:
**WO 2009/111347 (11.09.2009 Gazette 2009/37)**

(54) **PURIFIED IMMUNOGLOBULIN FUSION PROTEINS AND METHODS OF THEIR PURIFICATION**

AUFGEREINIGTE IMMUNOGLOBULINFUSIONSPROTEINE UND VERFAHREN ZU IHRER AUFREINIGUNG

PROTÉINES HYBRIDES PURIFIÉES D IMMUNOGLOBULINE ET LEURS PROCÉDÉS DE PURIFICATION

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **29.02.2008 US 67852**
**19.06.2008 US 74102**

(43) Date of publication of application:
**15.12.2010 Bulletin 2010/50**

(60) Divisional application:
**13159051.5 / 2 615 112**

(73) Proprietor: **Biogen Idec MA Inc.**
**Weston, MA 02493 (US)**

(72) Inventors:
• **MCCUE, Justin**
  **Belmont, MA 02478 (US)**
• **ROMERO, Jonathan**
  **Somerville**
  **MA 02143 (US)**
• **MEIER, Werner**
  **Burlington**
  **MA 01803 (US)**
• **NOTARNICOLA, Stephen**
  **Medford**
  **MA 02155 (US)**
• **EVANS, David**
  **Melrose, MA 02176 (US)**
• **MACNIVEN, Richard**
  **Somerville**
  **MA 02145 (US)**

(74) Representative: **Adams, Harvey Vaughan John**
**Mathys & Squire LLP**
**The Shard**
**32 London Bridge Street**
**London SE1 9SG (GB)**

(56) References cited:
**WO-A2-00/36092**    **WO-A2-2008/112325**

• **BROWNING J L ET AL: "Characterization of lymphotoxin-alpha beta complexes on the surface of mouse lymphocytes." JOURNAL OF IMMUNOLOGY (BALTIMORE, MD. : 1950) 1 OCT 1997, vol. 159, no. 7, 1 October 1997 (1997-10-01), pages 3288-3298, XP002535405 ISSN: 0022-1767**
• **GOMMERMAN JENNIFER L ET AL: "A role for surface lymphotoxin in experimental autoimmune encephalomyelitis independent of LIGHT." JOURNAL OF CLINICAL INVESTIGATION, vol. 112, no. 5, September 2003 (2003-09), pages 755-767, XP002535406 ISSN: 0021-9738**

- DEGLI-ESPOSTI M A ET AL: "Activation of the lymphotoxin beta receptor by cross-linking induces chemokine production and growth arrest in A375 melanoma cells" JOURNAL OF IMMUNOLOGY, AMERICAN ASSOCIATION OF IMMUNOLOGISTS, US, vol. 158, no. 4, 15 February 1997 (1997-02-15), pages 1756-1762, XP002141941 ISSN: 0022-1767
- JENNISSEN HERBERT P: "Hydrophobic interaction chromatography: harnessing multivalent protein-surface interactions for purification procedures." METHODS IN MOLECULAR BIOLOGY (CLIFTON, N.J.) 2005, vol. 305, 2005, pages 81-99, XP008108104 ISSN: 1064-3745

- BROWNING J L ET AL: "SIGNALING THROUGH THE LYMPHOTOXIN BETA RECEPTOR INDUCES THE DEATH OF SOME ADENOCARCINOMA TUMOR LINES", THE JOURNAL OF EXPERIMENTAL MEDICINE, ROCKEFELLER UNIVERSITY PRESS, US, vol. 183, no. 3, 1 March 1996 (1996-03-01) , pages 867-878, XP000571445, ISSN: 0022-1007, DOI: 10.1084/JEM.183.3.867

**Description**

## BACKGROUND OF THE INVENTION

Given the increasing acceptance of biologies as approved therapeutics, detailed characterization of these kinds of drugs is important. Unforeseen product breakdown or presence of impurities can jeopardize therapeutic efficacy as well as patient safety. Examples of impurities found during biologic manufacturing can include aggregates of the product. In pharmaceutical compositions, aggregates are undesirable as they may be immunogenic and present safety concerns for *in vivo* use. Aggregates may also decrease the *in vivo* stability of the product due to neutralizing antibodies produced in the patient.

Moreover, inactive proteins may also be made during manufacturing. Inactive proteins may result from incorrect folding during the manufacturing process. For example, US 2002/0039580 (Browning et al.) describes two forms, an active and inactive form of the lymphotoxin β receptor immunoglobulin (LTβR-Ig) fusion protein when expressed in mammalian cells. US 2002/0039580 also teaches that by reducing the temperature of the cell culture during Ig fusion protein production, the percentage of inactive molecule in a preparation can be decreased. That publication describes only one inactive form of LTβR-Ig, which has no ability to bind ligand.

WO 00/36092 relates to methods for the expression of active LT-β-R-Ig chimeric proteins, and also their purification.

WO 2008/112325 discloses LT-β-R-Ig fusion proteins.

Browning et al. (J. Immunol. (1997) 159(7): 3288-3298) characterizes the appearance of surface LT alpha beta complexes and LT beta R on several common murine cell lines.

In biologic manufacturing, such product related impurities can present purification challenges. These impurities are often difficult to identify as they often have comparable characteristics to the active form of the therapeutic protein of interest and, therefore, can be difficult to detect. Moreover, even when such impurities are identified, separation of the impurity from the drug product can be difficult. Thus, there remains a need for not only identifying product-related impurities which may be present during biologic production and purification processes, but also for methods for removing the impurities once they have been identified.

## SUMMARY OF THE INVENTION

The present invention addresses the problem of purifying an antibody-based biologic, *e.g.*, LT-β-R-Ig fusion protein, from impurities that arise during expression of the biologic in mammalian cell culture, *e.g.* CHO cells, such that the resulting composition is suitable for pharmaceutical use, both in terms of manufacturability of large amounts of the protein and purity.

As described herein, expression of LT-β-R-Ig fusion proteins in mammalian cells yields two inactive forms of LT-β-R-Ig and LT-β-R-Ig aggregates. Such impurities must be separated from the biologically active, monomeric form of LT-β-R-Ig in order to provide sufficient purity for use in patients. Inactive forms of LT-β-R-Ig present a unique challenge in the purification process. For example, the two inactive forms of LT-β-R-Ig described herein closely resemble the active, monomeric form of the fusion protein, e.g., with respect to size and charge. In addition, when production of LT-β-RIg is scaled up to increase yield (as is required for manufacture of a therapeutic), high levels of aggregated LT-β-R-Ig are produced. Thus, in order to produce active, monomeric LT-β-R-Ig fusion proteins at sufficient concentration and of the purity needed for clinical use, a process that minimizes the aggregation, minimizes the presence of inactive forms of the protein, and minimizes LT-β-R-Ig fusion protein loss was needed. The present invention provides a solution to this complex problem. More specifically, the present invention provides a method for separating biologically active lymphotoxin-β receptor immunoglobulin (LT-β-R-Ig) fusion proteins from biologically inactive LT-β-R-Ig fusion proteins inclusive of the first inactive form of the LT-β-R-Ig fusion protein (inactive-1) and the second inactive form of the LT-β-R-Ig fusion protein (inactive-2), comprising

loading a mixture comprising biologically active LT-β-R-Ig fusion proteins and biologically inactive LT-β-R-Ig fusion proteins onto a butyl hydrophobic interaction chromatography (HIC) resin and

contacting the resin with a solution comprising a salt gradient to obtain a first eluate comprising biologically inactive-1 LT-β-R-Ig fusion proteins and a second eluate comprising biologically active LT-β-R-Ig fusion proteins, and

wherein the inactive-1 LT-β-R-Ig fusion protein elutes from the butyl hydrophobic interaction chromatography (HIC) resin at a higher salt concentration than the biologically active LT-β-R-Ig fusion protein, and

wherein the inactive-2 LT-β-R-Ig fusion protein remains bound to the butyl resin until stripped off with water.

In a related aspect, the invention provides a method for separating biologically active lymphotoxin-β receptor immunoglobulin (LT-β-R-Ig) fusion proteins from biologically inactive LT-β-R-Ig fusion proteins inclusive of the first inactive form of the LT-β-R-Ig fusion protein (inactive-1) and the second inactive form of the LT-β-R-Ig fusion protein (inactive-2), said method comprising the steps of:

i) loading a mixture comprising biologically active LT-β-R-Ig fusion proteins and biologically inactive LT-β-R-Ig fusion proteins onto a butyl hydrophobic interaction chromatography (HIC) resin under conditions that allow the biologically active LT-β-R-Ig fusion proteins to bind the butyl resin;

ii) contacting the butyl HIC resin with a solution comprising a salt gradient to obtain a first eluate enriched for the presence of biologically active LT-β-R-Ig fusion proteins;

iii) loading the first eluate of step ii) onto a second HIC resin under conditions that allow the biologically active LT-β-R-Ig fusion proteins to bind the second HIC resin; and

iv) contacting the second HIC resin with a solution to obtain a second eluate further enriched for the presence of biologically active LT-β-R-Ig fusion proteins,

to thereby separate the biologically active LT-β-R-Ig fusion proteins from the biologically inactive LT-β-R-Ig fusion proteins, and

wherein the inactive-1 LT-β-R-Ig fusion protein elutes from the butyl hydrophobic interaction chromatography (HIC) resin at a higher salt concentration than the biologically active LT-β-R-Ig fusion protein, and

wherein the inactive-2 LT-β-R-Ig fusion protein remains bound to the butyl resin until stripped off with water.

In a further aspect, the invention provides a method for separating biologically active LT-β-R-Ig fusion proteins from biologically inactive LT-β-R-Ig fusion proteins, said method comprising the steps of:

i) loading a mixture comprising biologically active LT-β-R-Ig fusion proteins and biologically inactive LT-β-R-Ig fusion proteins onto a mixed mode resin under conditions that allow the biologically active LT-β-R-Ig fusion proteins to bind the mixed mode resin;

ii) eluting the biologically active LT-β-R-Ig fusion proteins from the mixed mode resin of step i) with a solution to obtain a first eluate enriched for the presence of biologically active LT-β-R-Ig fusion proteins;

iii) loading the first eluate of step ii) onto a hydrophobic interaction chromatography (HIC) resin under conditions that allow the biologically active LT-β-R-Ig fusion proteins to bind the HIC resin; and

iv) eluting the biologically active LT-β-R-Ig fusion proteins of step iii) with a solution to obtain a second eluate further enriched for the presence of biologically active LT-β-R-Ig fusion proteins,

to thereby separate biologically active LT-β-R-Ig fusion proteins from biologically inactive LT-β-R-Ig fusion proteins.

In a further aspect, the invention provides a method for separating unaggregated, biologically active LT-β-R-Ig fusion proteins from aggregated LT-β-R-Ig fusion proteins the method comprising the steps of:

i) loading a mixture comprising unaggregated, biologically active LT-β-R-Ig fusion proteins and aggregated LT-β-R-Ig fusion proteins, wherein more than about 30% or about 30% of LT-p-R-Ig fusion proteins in the mixture are aggregated, onto a mixed mode resin under conditions that allow the biologically active LT-β-R-Ig fusion proteins to bind the mixed mode resin;

ii) eluting the biologically active LT-β-R-Ig fusion proteins from the mixed mode resin of step i) using a solution to obtain a first eluate enriched for the presence of biologically active LT-β-R-Ig fusion proteins;

iii) loading the first eluate of step ii) onto a hydrophobic interaction chromatography (HIC) resin under conditions that allow the biologically active Ig fusion proteins to bind the HIC resin; and

iv) eluting the biologically active LT-β-R-Ig fusion proteins of step iii) using a solution to obtain a second eluate further enriched for the presence of biologically active LT-β-R-Ig fusion proteins,

thereby separating the unaggregated biologically active LT-β-R-Ig fusion proteins from the aggregated LT-β-R-Ig fusion proteins.

In a further aspect, the invention provides method for removing at least 97% aggregated LT-β-R-Ig fusion proteins from a mixture comprising unaggregated, biologically active LT-β-R-Ig fusion proteins and aggregated LT-β-R-Ig fusion proteins, said method comprising the steps of:

i) loading the mixture onto a mixed mode resin under conditions that allow the unaggregated, biologically active LT-β-R-Ig fusion proteins to bind the mixed mode resin;

ii) eluting the unaggregated, biologically active LT-β-R-Ig fusion proteins from the mixed mode resin of step i) with a solution to obtain a first eluate enriched for the presence of unaggregated, biologically active LT-β-R-Ig fusion proteins;

iii) loading the first eluate of step ii) onto a hydrophobic interaction chromatography (HIC) resin under conditions that allow the unaggregated, biologically active LT-β-R-Ig fusion proteins to bind the HIC resin; and

iv) eluting the unaggregated, biologically active LT-β-R-Ig fusion proteins of step

iii) with a solution to obtain a second eluate further enriched for the presence of unaggregated, biologically active LT-β-R-Ig fusion proteins,

to thereby remove at least 97% aggregated LT-β-R-Ig fusion proteins from the mixture comprising unaggregated, biologically active LT-β-R-Ig fusion proteins and aggregated LT-β-R-Ig fusion proteins.

In yet another aspect, the invention provides a method for preparing a composition comprising biologically active LT-β-R-Ig fusion proteins, wherein the composition comprises less than 1% of a first inactive form of the LT-β-R-Ig fusion protein (Inactive-1), less than 1% of a second inactive form of the LT-β-R-Ig fusion protein (Inactive-2), and less than 1% aggregated LT-β-R-Ig fusion proteins, said method comprising the steps of

i) obtaining a cell culture supernatant from a mammalian cell culture system comprising a mammalian host cell transformed with DNA encoding the LT-β-R-Ig-fusion protein;

ii) contacting the cell culture supernatant with recombinant Protein A (rProtein A) to obtain an LT-β-R-Ig fusion protein mixture;

iii) loading the LT-β-R-Ig fusion protein mixture of ii) onto a mixed mode resin at a pH of about 5.0 under conditions such that the LT-β-R-Ig fusion proteins bind the mixed mode resin;

iv) washing the mixed mode resin with a buffer having a pH of about 7.0 to 7.2;

v) contacting the mixed mode resin of step iii) with a solution to obtain a first eluate enriched for the presence of biologically active LT-β-R-Ig fusion protein;

vi) loading the first eluate of step iv) onto a hydrophobic interaction chromatography (HIC) resin under conditions that allow the LT-β-R-Ig fusion protein to bind the HIC resin; and

vii) contacting the HIC resin of step vi) with a solution to obtain a second eluate further enriched for the presence of biologically active LT-β-R-Ig fusion protein,

wherein the second eluate comprises less than 1% of the first inactive form of the LT-β-R-Ig fusion protein (Inactive-1), less than 1% of the second inactive form of the LT-β-R-Ig fusion protein (Inactive 2), and less than 1% aggregated LT-β-R-Ig fusion proteins.

The present invention and embodiments thereof are set out in the appended claims.

## BRIEF DESCRIPTION OF THE FIGURES

*Figure 1* provides an overview of the process of Cycle-1-1.

*Figure 2* shows the results of the HIC-1 butyl resin step of Cycle-1-1, including the conditions under which inactive forms 1 and 2 of the LTβR-Ig fusion protein are eluted.

*Figure 3* describes an overview of process intermediates using HPLC.

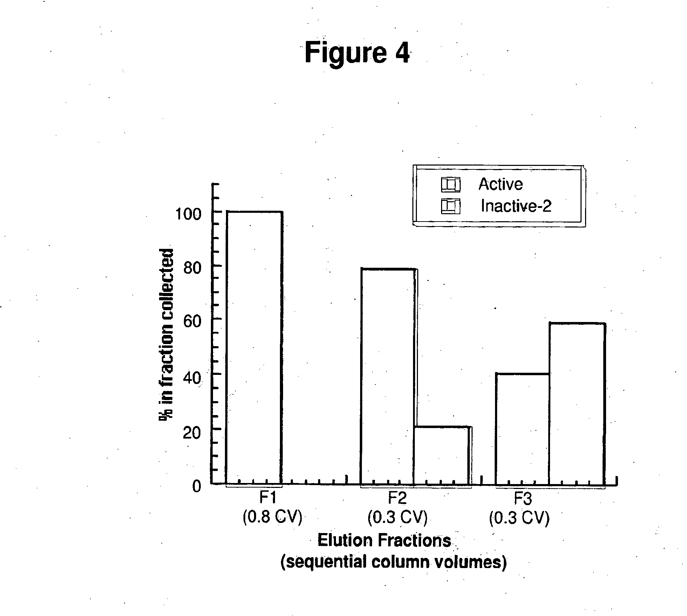

*Figure. 4* graphically depicts active and Inactive-2 percentages in Elution Fractions of Butyl-650M LTβR-Ig Purification (Cycle 1-1). LTβR-Ig purification at load and wash concentrations of 1.65 M NaCl. Columns were loaded in duplicate to 8 mg/mL resin. Elution was performed using 0.7 M NaCl and elution fractions were collected in sequence (bar graph shows data from each fraction, not cumulative) and LTβR-Ig forms measured.

*Figure 5* provides an overview of the process of Cycle-1-2.

*Figure 6* describes the step and gradient wash strategy of Cycle 1-2.

*Figure 7* provides a table which summarizes the improvements in impurity resolution following the three separation methods of Cycle-1, *i.e.,* protein A, HIC-1 (butyl), and HIC-2 (phenyl).

*Figure 8* describes aggregate clearance by the HIC-2 (phenyl) step, including effect of load, bed height, and flow velocity. Figure 8A describes the effect of load on the percentage of aggregate and active LTβR-Ig fusion protein following the phenyl step. Figure 8B describes the effect of bed height and velocity on percentage of active LTβR-Ig (active monomer) yield from development of the Cycle-2 HIC (phenyl) step.

*Figure 9* provides an overview of the process of Cycle-2.

*Figure 10* provides a schematic of the LTβR-Ig fusion protein.

*Figures 11A and 11B* describes the operating conditions for the mixed mode column.

*Figure 12* graphically depicts the chromatography conditions for the 3rd column of cycle-2.

*Figure 13* provides a table describing the key performance features of the modified downstream process in 15000 manufacturing.

## DETAILED DESCRIPTION OF THE INVENTION

In order that the invention herein described may be fully understood, the following detailed description is set forth.

## I. Definitions

The terms "active" or "biologically active," used interchangeably herein, refer to the form of a protein which is capable of binding to its cognate binding partner (e.g., to a ligand for a receptor) and blocking the biological effect of the binding of cell-associated LTβR with LT. Not included in the definition of biologically active is a biological or immunological response *to* the protein. Biological activity may be determined according to the type of protein being characterized. For example a protein which is a receptor (or a soluble form thereof) may be shown to be biologically active if it can bind to its respective ligand. Alternatively, the protein may be bound by an antibody specific for the active form of the protein. A biological effect of the protein may be measured using cell-based assay. Such assays are well known in the art (*e.g.*, LTβR activity may be determined using a standard IL-8 inhibition assay).

The term "inactive" or "biologically inactive" refers to a protein which is unable to bind to its cognate binding partner and, therefore, cannot modify a biological response in a system or which binds with insufficient affinity to cause a biological response. For example in the case of a receptor or soluble form thereof, an inactive form of the receptor is unable to bind to a ligand and, thereby unable to mediate the biological activity of the active form. Alternatively, an inactive form of a receptor may bind to ligand, e.g., with reduced affinity, but does not cause an appropriate biological response, or (when in soluble form) does not fully block the biological signal transduced upon binding of cell-based forms of the receptor with its ligand.

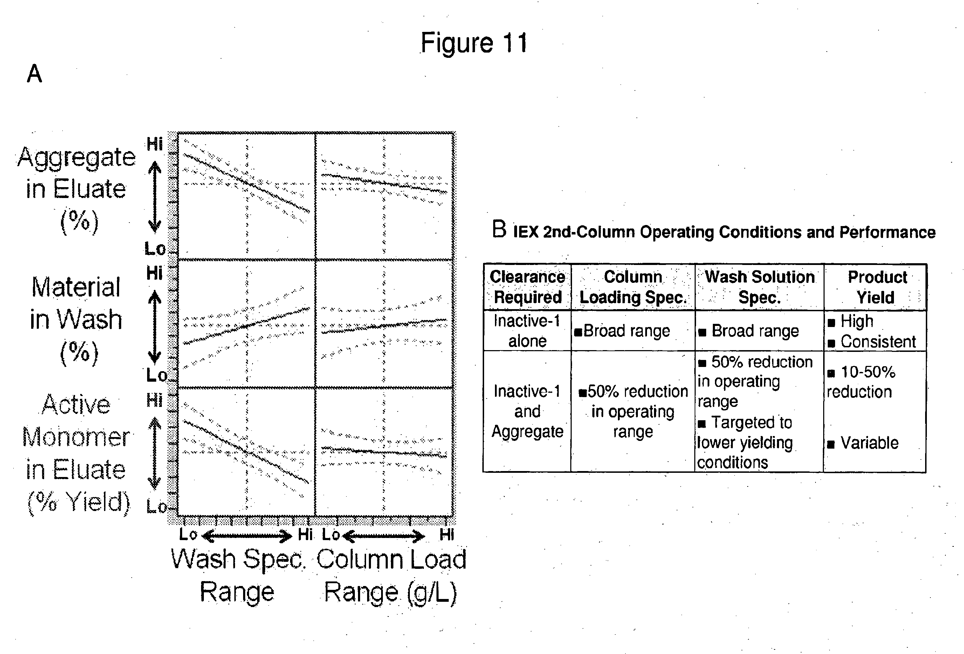

As used herein, the phrase "active monomer" refers to a biologically active LT-β-R-Ig fusion protein which is non aggregated and comprises two chains of LT-β-R-Ig linked through disulfide bonds. An example of an active monomer is provided in Figure 11. Thus, a monomer is a two chain Fc fusion protein which is not aggregated.

The term "aggregate" refers to a misfolded, rigid protein grouping or a high molecular weight material (including multimers of a protein). In one embodiment, an aggregate refers to a multimeric form of an LT-β-R-Ig monomer which comprises more than two chains of an Fc fusion protein, *e.g.*, a dimeric or higher order multimeric form (tetrameric, octameric). Aggregates may be biologically active or inactive.

The terms "protein" and "polypeptide" are used herein interchangeably, and refer to amino acid sequences of a variety of lengths, either in their neutral (uncharged) forms or as salts, and either unmodified or modified by glycosylation, side chain oxidation, or phosphorylation.

The term "fusion protein" refers to a molecule comprising two or more proteins or fragments thereof linked by a covalent bond via their individual peptide backbones, most preferably generated through genetic expression of a polynucleotide molecule encoding those proteins. In a preferred instance, the fusion protein includes an immunoglobulin domain.

The term "lymphotoxin-β receptor" or "LTβR" refers to a member of the TNF family of receptors which binds to surface lymphotoxin (LT) which is composed of a trimeric complex of lymphotoxin alpha and beta chains (Crowe et al, 1994) as well as to the ligand LIGHT (homologous to lymphotoxins, exhibits inducible expression, and competes with herpes simplex virus glycoprotein D for HVEM, a receptor expressed by T lymphocytes). LT-β-R is involved in the development of the peripheral immune system and the regulation of events in the lymph nodes and spleen in the mature immune system (Ware et al, 1995; Mackay et al, 1997; Rennert et al, 1996; Rennert et al, 1997; Chaplin and Fu, 1998).

In one embodiment, an LTβR-immunoglobulin (LTβR-Ig) fusion protein comprises the ligand-binding portion of the extracellular domain of LTβR and an IgG constant region, *e.g.*, Fc portion of an Ig. An LTβR-Ig fusion protein can block signaling between the surface LT ligand and LTβR with consequences on the functional state of follicular dendritic cells (Mackay and Browning 1998). This blocking can furthermore lead to diminished autoimmune disease in rodent models (Mackay et al, 1998, U.S. Ser. No. 08/505,606 filed Jul. 21, 1995 and U.S. Ser. No. 60/029,060 filed Oct. 26,1996). The sequence of LTβR is provided in SEQ ID NO: 1:

HUMAN LTβR SEQ. (PUBMED AAH26262 AND P36941)

```
  1   MLLPWATSAP  GLAWGPLVLG  LFGLLAASQP  QAVPPYASEN  QTCRDQEKEY  YEPQHRICCS
 61   RCPPGTYVSA  KCSRIRDTVC  ATCAENSYNE  HWNYLTICQL  CRPCDPVMGL  EEIAPCTSKR
121   KTQCRCQPGM  FCAAWALECT  HCELLSDCPP  GTEAELKDEV  GKGNNHCVPC  KAGHFQNTSS
181   PSARCQPHTR  CENQGLVEAA  PGTAQSDTTC  KNPLEPLPPE  MSGTMLMLAV  LLPLAFFLLL
241   ATVFSCIWKS  HPSLCRKLGS  LLKRRPQGEG  PNPVAGSWEP  PKAHPYFPDL  VQPLLPISGD
301   VSPVSTGLPA  APVLEAGVPQ  QQSPLDLTRE  PQLEPGEQSQ  VAHGTNGIHV  TGGSMTITGN
361   IYIYNGPVLG  GPPGPGDLPA  TPEPPYPIPE  EGDPGPPGLS  TPHQEDGKAW  HLAETEHCGA
421   TPSNRGPRNQ  FITHD  (SEQ ID NO: 1)
```

In one embodiment, an LT-β-R-Ig fusion protein can include all or a fragment of the extracellular domain of human LT-β-R-Ig (e.g., it can include residues 40-200, 35-200, 40-210; 35-220, 32-225, or 28-225 of human LT-β-R. In some embodiments, an LT-β-Rfusion protein includes the extracellular region of the LT-β-Rmolecule as represented by residues 32-225 of SEQ ID NO:1.

An example of an LTβR-Ig fusion protein that may be included in the methods of the invention is set forth below (SEQ ID NO:2):

> *MLLPWATSAPGLAWGPLVLGLFGLLAA*AVPPYASENQTCRDQEKEYYEPQHRICCSRCPPGTYVSA
> KCSRIRDTVCATCAENSYNEHWNYLTICQLCRPCDPVMGLEEIAPCTSKRKTQCRCQPGMFCAA
> WALECTHCELLSDCPPGTEAELKDEVGKGNNHCVPCKAGHFQNTSSPSARCQPHTRCENQGLVE
> AAPGTAQSDTTCKNPLEPLPPEMSGTM**VDKTHTCPPCPAPELLGGPSVFLFPPKPKDTLMISRT**
> **PEVTCVVVDVSHEDPEVKFNWYVDGVEVHNAKTKPREEQYNSTYRVVSVLTVLHQDWLN**
> **GKEYKCKVSNKALPAPIEKTISKAKGQPREPQVYTLPPSRDELTKNQVSLTCLVKGFYPSDI**
> **AVEWESNGQPENNYKTTPPVLDSDGSFFLYSKLTVDKSRWQQGNVFSCSVMHEALHNHYT**
> **QKSLSLSPG**

(Amino acids in italics indicate signal sequence; underlined amino acids indicate sequence derived from the extracellular region of LT-β-R; and amino acids in bold indicate IgG Fc sequence. A valine linking the LT-β-R sequence with the IgG-Fc sequence is artificial, and derived neither from the LT-β-R or the IgG-Fc sequence. The underlined sequence is a substantial part of the extracellular domain of LT-β-R and corresponds to amino acids 32 to 225 of SEQ ID NO:1.) In one embodiment, the LT-β-R fusion proteins used in the methods of the invention includes the amino acid sequence set forth in SEQ ID NO:2 without the signal sequence.

"Surface LT ligand" refers to a surface LT complex or derivative thereof that can specifically bind to the LTβR.

The term "ligand binding domain" or "ligand binding portion" as used herein refers to a native receptor (e.g., cell surface receptor) or region or derivative thereof retraining at least a qualitative ligand binding ability, and preferably the biological activity of a corresponding native receptor.

The term "immunoglobulin fusion protein" refers to an LT-B-R-Ig fusion protein. The portions of the Ig molecule may derive from any of the various immunoglobulin isotypes, including, for example, IgG1, IgG2, IgM, IgA etc.

The generic term "immunoglobulin" comprises five distinct classes of antibody that can be distinguished biochemically. All five classes of antibodies are clearly within the scope of the present invention, the following discussion will generally be directed to the IgG class of immunoglobulin molecules

The phrase "TNF family of receptors" refers to a receptor, whether naturally membrane bound or secreted (as in the case of osteoprotegerin), which has the canonical TNF family cysteine bridging patterns or any receptor which binds to a defined member of the TNF family of ligands (e.g. Banner *et al* 1993).

The terms "approximately" and "about", as used herein in reference to a number generally includes numbers that fall within a range of 10% in either direction of the number (greater than or less than the number) unless otherwise stated or otherwise evident from the context (except where such number would exceed 100% of a possible value).

II. Separation of different forms of inactive and/or aggregated Ig fusion proteins

The invention concerns the ability to produce purified active forms of immunoglobulin (Ig) fusion proteins, including LTβR-Ig fusion proteins, by separating active forms of the protein from inactive forms and/or by separating active forms of the protein from aggregates.

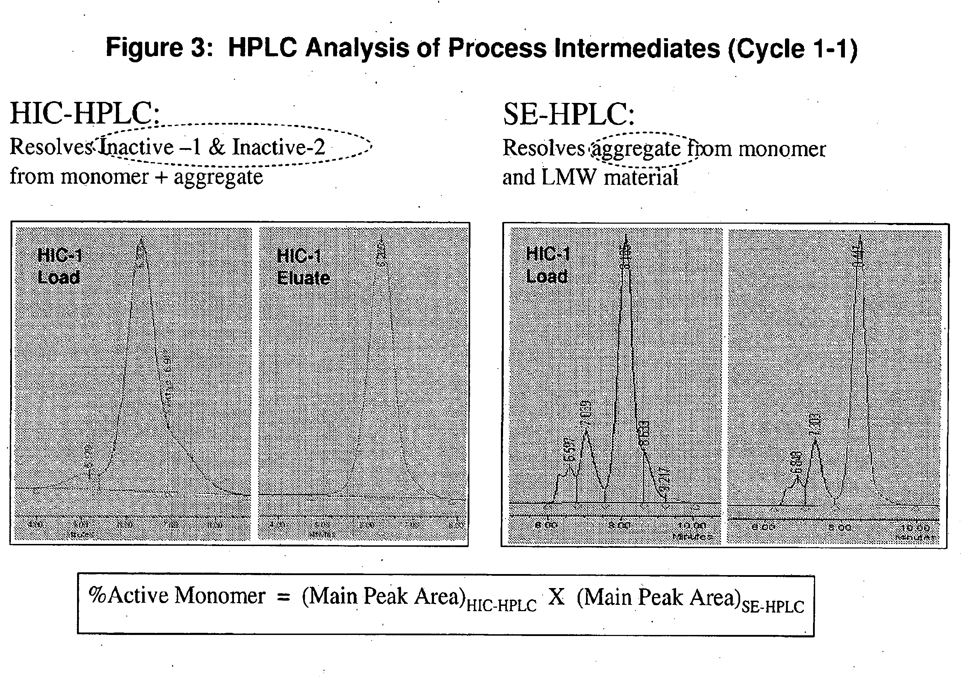

While it was known that expression of Ig fusion proteins in mammalian cells may result in multiple forms of inactive Ig fusion proteins, the extent of contamination and the means of adequately purifying the fusion proteins was not known. More specifically, the art taught that LTβR-Ig fusion protein is expressed in two forms in monkey cos cells or Chinese hamster ovary cells. One form was taught to bind ligand with high affinity, the "active" form whereas the other, the "inactive" form was taught not to bind ligand (US 20020039580). However the realization that there are in fact two different inactive forms of the LTβR-Ig fusion protein (Inactive-1 and Inactive-2) was not previously known and, therefore, separation methods to minimize the presence of both of these inactive forms (Inactive-1 and Inactive-2) in a preparation had not previously been developed. For example, as described in the Examples below, mammalian expression of LTβR-Ig fusion proteins results in a mixture of active LTβR-Ig fusion proteins, the mixture comprising a first inactive form of the LTβR-Ig fusion protein (referred to herein as "Inactive-1" and which can be seen as the first shoulder before the main peak in the HIC-1 load HIC-HPLC panel of Figure 3), and a second inactive form of the LTβR-Ig fusion protein (referred to herein as "Inactive-2" which can be seen as the shoulder after the main peak of the HIC-1 load HIC-HPLC panel of Figure 3), and aggregated forms of LTβR-Ig.

The invention pertains to separation of the desired active, monomeric form of LTβR-Ig from multiple forms of inactive and/or Ig fusion proteins, as well as protein aggregates. The method of the invention relies on combinations of different types of chromatography, *e.g.*, hydrophobic interaction chromatography (HIC), to effectively reduce the percentage of inactive (including both forms) and/or aggregate Ig fusion proteins from a mixture.

The invention provides a method for separating a mixture containing biologically active immunoglobulin (Ig) fusion proteins, inactive Ig fusion proteins, and aggregates. In one aspect, the separation is achieved by first step comprising a mixed mode resin followed by a phenyl HIC step. The first step in the process of the invention involves contacting the mixture with a mixed mode resin under conditions that allow the biologically active Ig fusion proteins to bind the mixed mode resin. It should be noted that conditions which are conducive to the mixed mode resin binding the active Ig fusion protein may also be conducive to impurity binding. As such, wash and elution conditions may be used to separate the impurities from the biologically active In fusion proteins. For example, for the purification of active LT-β-R-Ig, following the loading of the mixed mode resin with the protein mixture, certain pre-elution washes and elution conditions may be used to separate active LT-β-R-Ig from Inactive 1. Examples of such wash/elution conditions are provided in the Examples below. The biologically active Ig fusion proteins are then eluted from the first mixed mode resin step, such that an eluate is obtained.

The second step in the purification process comprises contacting the eluate of the first step with a hydrophobic interaction chromatography (HIC) resin under conditions that allow the biologically active Ig fusion proteins to bind the HIC resin. The active Ig fusion proteins are then eluted. The resulting solution contains biologically active Ig fusion proteins which have been separated from both inactive forms of the Ig fusion protein and protein aggregates. In a preferred embodiment, the HIC resin is a phenyl resin, *e.g.,* phenyl sepharose. With respect to purifying LT-β-R-Ig fusion proteins using the phenyl step, as described in more detail in the Examples below, the product (active LT-β-R-Ig fusion proteins), aggregates, and Inactive-2 all bind to the phenyl column upon loading, but are separated using appropriate wash/elution conditions. The elution conditions may result in the aggregate and Inactive-2 remaining preferentially on the column, while the LT-β-R-Ig product elutes in a pure form.

One method for separating active Ig fusion proteins from inactive forms and/or aggregates includes contacting a mixture comprising biologically active Ig fusion proteins and biologically inactive Ig fusion proteins with a butyl hydrophobic interaction chromatography (HIC) resin under conditions that allow the biologically active Ig fusion proteins to bind the butyl resin. It should be noted that conditions which are conducive to the butyl resin binding the active Ig fusion protein may also be conducive to impurity binding. As such, wash and elution conditions may be used to separate the impurities from the biologically active Ig fusion proteins. For example, for the purification of active LT-β-R-Ig, following the loading of the butyl resin with the protein mixture, certain pre-elution washes and elution conditions may be used to separate active LT-β-R-Ig from the inactive forms. Inactive-1 may be washed off by lowering the NaCl concentration. The active LT-β-R-Ig and aggregates may be eluted by further lowering the NaCl concentration, while Inactive-2 remains bound to the column until is stripped off with water (no NaCl). Examples of such wash/elution conditions are provided in the Examples below.

Following elution of the biologically active Ig fusion proteins, the eluate is subsequently contacted with a second HIC resin, such that the biologically active Ig fusion proteins to bind the second HIC resin. Following elution of the biologically active Ig fusion proteins, the resulting solution contains biologically active Ig fusion proteins separated from inactive forms and/or aggregates. In one embodiment, the butyl HIC resin comprises a hydroxylated methacrylate backbone

Alternatively, the purification method of the invention may be achieved using a combination of phenyl HIC steps. With respect to LT-β-R-Ig fusion proteins, the biologically active LT-β-R-Ig, inactive forms (*i.e.*, Inactive-1 and Inactive-2), and aggregates each bind to phenyl resin. As such, the active LT-β-R-Ig may be purified using appropriate combinations of wash and elution buffers,

An important aspect of the invention is the purification of biologically active LTβR-Ig fusion proteins from the two inactive forms of the protein, as well as protein aggregates. Such impurities result from production of the LT-β-R-Ig fusion protein, particularly production in mammalian cell culture, but can be reduced in accordance with the purification steps described herein.

The invention provides a method for separating a mixture containing biologically active LTβR-Ig fusion proteins, inactive LTβR-Ig fusion proteins, and aggregates. In one aspect, the separation is achieved by first step comprising a mixed mode resin followed by a phenyl HIC step. The first step in the process involves contacting the mixture with a mixed mode resin under conditions that allow the biologically active LTβR-Ig fusion proteins to bind the mixed mode resin. It should be noted that conditions which are conducive to the mixed mode resin binding the active Ig fusion protein may also be conducive to impurity binding. As such, wash and elution conditions may be used to separate the impurities from the biologically active Ig fusion proteins. The biologically active LTβR-Ig fusion proteins are then eluted from the first mixed mode resin step, such that an eluate is obtained. In one embodiment, the eluate from the mixed mode resin has a significantly reduced amount of inactive-1 form of LTβR-Ig fusion protein, *i.e.*, the first eluate contains less than 2%, less than 1%, or less than 0.5% of the inactive 1 form of LTβR-Ig fusion protein. In addition aggregate may be reduced from 28% in the load to 8% in the eluate depending on the chromatography conditions. The second step in the purification process comprises contacting the eluate of the first step with a hydrophobic interaction chromatography (HIC) resin under conditions that allow the biologically active LTβR-Ig fusion proteins (which may also include impurities) to bind the HIC resin, The active LTβR-Ig fusion proteins are then eluted, such that both the inactive-2 form of the LTβR-Ig fusion protein and protein aggregates are significantly reduced. In one embodiment, the eluate from the HIC resin has a significantly reduced amount of inactive-2 form of LTβR-Ig fusion protein, *i.e.,* the first eluate contains less than 2%, less than 1%, or less than 0.5% of the inactive 2 form of LTβR-Ig fusion protein. The resulting solution contains biologically active LTβR-Ig fusion proteins which have been separated from both inactive forms of the LTβR-Ig fusion protein and protein aggregates. In a preferred embodiment, the HIC resin is a phenyl resin, *e.g.*, phenyl sepharose.

Another method for separating active Ig fusion proteins from inactive forms and/or aggregates includes contacting a mixture comprising biologically active Ig fusion proteins and biologically inactive Ig fusion proteins with a butyl hydrophobic interaction chromatography (HIC) resin under conditions that allow the biologically active Ig fusion proteins to bind the butyl resin. The biologically active Ig fusion proteins are then eluted and collected in the first eluate. The eluate is subsequently contacted with a second HIC resin, such that the biologically active Ig fusion proteins to bind the second HIC resin. Following elution of the biologically active Ig fusion proteins, the resulting solution contains biologically active Ig fusion proteins separated from inactive forms and/or aggregates. In one embodiment, the butyl HIC resin comprises a hydroxylated methacrylate backbone.

Also featured is a method for removing at least 97% of protein aggregates from a mixture comprising biologically active LT-β-R-Ig fusion proteins and protein aggregates. The method is based on the above combinations of chromatography steps, *e.g.,* HIC phenyl or mixed mode / HIC Phenyl. In one embodiment, the method includes contacting the mixture with a phenyl HIC resin or a mixed mode resin under conditions that allow the biologically active LT-β-R-Ig fusion proteins to bind the phenyl HIC or mixed mode resin, and subsequently eluting the biologically active LT-β-R-Ig fusion proteins from the phenyl HIC or mixed mode resin to obtain a first eluate. The first eluate has reduced amounts of protein aggregate, including less than 25% protein aggregates, less than 20% protein aggregates, less than 15% protein aggregates, or about 10% protein aggregate. In one embodiment, following the first HIC step, the eluate may be contacted with a second resin, *e.g.*, phenyl HIC, under conditions that allow the biologically active LT-β-R-Ig fusion proteins to bind the HIC resin. Elution of the biologically active LT-β-R-Ig fusion proteins to obtain a second eluate which contains less than 2% protein aggregate, or less than 1% protein aggregate. This method results in removal of at least 97% of the protein aggregates from the initial mixture comprising biologically active LT-β-R-Ig fusion proteins and protein aggregates.

A particular advantage of the methods described herein, is that the various processes may be used to purify product, *e.g.*, LT-β-R-Ig fusion protein, when 50% of the starting material comprises unwanted inactive and aggregate species. In one embodiment, the mixed mode resin (*e.g.,* Capto MMC) step is capable of reducing aggregate from 28 to 8% and the cycle-2 Phenyl column is capable of reducing aggregate from 24% to <1.0%.

Another aspect of the invention is a method for preparing a composition comprising biologically active LT-β-R-Ig fusion proteins. As set forth herein above, in one illustrative embodiment the composition comprises less than 1% of a first inactive form of the LT-β-R-Ig fusion protein (Inactive-1 protein), less than 1% of a second inactive form of the LT-β-R-Ig fusion protein (Inactive 2 protein), and less than 1% protein aggregate. The method is based on certain combinations of chromatography steps which provide for removal of the inactive Ig fusion proteins and protein aggregates. The method includes obtaining a cell culture supernatant from a mammalian cell culture system comprising a mammalian host cell transformed with DNA encoding the LT-β-R-Ig-fusion protein. Following the expression, the cell culture supernatant is contacted with recombinant Protein A (rProtein A) to obtain an LT-β-R-Ig fusion protein mixture. The LT-β-R-Ig fusion mixture is contacted with a hydrophobic interaction chromatography (HIC) resin under conditions that allow the active LT-β-R-Ig fusion protein to bind the resin. Following elution of the active LT-β-R-Ig fusion protein, the eluate is contacted with a second hydrophobic interaction chromatography (HIC) resin under conditions that allow the active LT-β-R-Ig fusion protein to bind the HIC resin. In one embodiment, following elution of the active LT-β-R-Ig fusion proteins, the eluate will contain less than 1% of the first inactive form of the LT-β-R-Ig fusion protein (Inactive-1 protein), less than 1% of the second inactive form of the LT-β-R-Ig fusion protein (Inactive 2 protein), and less than 1% protein aggregate. In one embodiment, the invention pertains to a composition comprising less than 1% of the first inactive form of the LT-β-R-Ig fusion protein (Inactive-1 protein), less than 1% of the second inactive form of the LT-β-R-Ig fusion protein (Inactive 2 protein), and less than 1% protein aggregate.

In one embodiment, the Phenyl based purification of LT-β-R-Ig fusion proteins expressed in cell culture includes step elution, whereby the column is loaded at 1 M ammonium sulfate and eluted at 0.44 M. Aggregates and Inactive-2 form of LTBR-Ig elute "later" than the product.

In one embodiment, the Butyl based purification of LT-β-R-Ig fusion proteins expressed in cell culture includes washing the Inactive-1 LT-β-R-Ig fusion protein following column load by stepping down the NaCl concentration. Then, for the Butyl elution, NaCl concentration is further stepped down, causing product (and aggregate) to elute but not Inactive-2. Finally Inactive-2 is stripped from the Butyl column by deceasing the NaCl salt concentration to zero.

Active and inactive forms of the LT-β-R-Ig fusion protein may be determined according to any number of parameters known in the art, including binding to the LT ligand. In one embodiment, the affinity of the binding interaction may be measured. In another embodiment, *in vitro* assays known in the art that measure the ability of an LT-β-R molecule to cause a biological response may be used to distinguish biologically active from biologically inactive LT-β-R-Ig fusion proteins, such as the IL-8 inhibition assay.

For example, in one embodiment an IL-8 release assay is based on the secretion of IL-8, which is observed after soluble recombinant human lymphotoxin α1 β2 binds to cell surface lymphotoxin beta receptor on A375 cells (human melanoma cell line). The IL-8 release assay measures the ability of a binding molecule (e.g., LT-β-R-Ig) to block this IL-8 secretion by binding to soluble lymphotoxin α1β2, preventing it from binding to the lymphotoxin beta receptor. The IL-8 that is secreted into the media supernatant is then measured with an ELISA assay. The binding molecule is diluted to the appropriate concentrations and incubated with soluble recombinant human lymphotoxin α1β2 (170ng/ml) for 1 hour at room temperature in a 96-well microtiter plate. The concentration of lymphotoxin α1β2 is optimized by titration experiments that determine the maximum amount of IL-8 release. Twenty thousand A375 cells are then added to each well, and the plate is incubated for 17 hours at 37°C 5% $CO_2$, At the end of the incubation period, the plate is centrifuged and the supernatant is harvested. The supernatants are tested for IL-8 concentration with a standard sandwich ELISA assay. The IL-8 concentrations are plotted versus antibody concentrations, and an IC50 is determined from a 4-parameter curve fit of the data.

Generally, LT-β-R-Ig fusion protein is considered active in the IL-8 inhibition assay if the percent inhibition is between 75%-135% of a control reference. Alternatively, active and inactive forms of LT-β-R-Ig fusion proteins may be determined using antibodies that are specific for one form or the other. Examples of antibodies specific for the active or functional form (antibodies that fail to bind the inactive form) of LT-β-R-Ig fusion protein include AGH1 (produced by hybridoma AG.H1.5.1; ATCC Accession No. HB11796) and BDA8 (hybridoma cell line (BD.A8.AB9) which produces the mouse anti-human LT-β-R mAb BDA8 was deposited on Jan. 12, 995 with the American Type Culture Collection (ATCC) (Rockville, Md.) according to the provisions of the Budapest Treaty, and was assigned the ATCC accession number HB11798) which are each described in US 20020197254, US 20020039580, and US Patent No. 7,001,598. In addition, active and inactive proteins may be characterized by their elution properties in accordance with the chromatography steps described herein. For example, using the butyl HIC column the inactive-1 form of the LT-β-R-Ig fusion protein is eluted in the first wash of 1.55 M NaCl. The LT-β-R-Ig fusion protein and protein aggregates are eluted from the column using 0.7M NaCl as an elution buffer, and the inactive-2 form of LT-β-R-Ig fusion protein is eluted using the strip wash of pure water. As described in more detail in the Examples below, similar purification may be achieved using step gradients of sodium sulfate instead of sodium chloride.

It should be noted that the above methods may be performed using large scale manufacturing processes. For example, the methods described herein may be used to effectively separate impurities from the mixture having a volume of at least 5000 L, at least 10000L, at least 15000L, 15000 L, or greater than 15000 L.

In addition, certain column parameters may be adjusted to maximize protein recovery. In one embodiment, the second HIC step of the method includes a column having a height of between 25-35 centimeters, 29 to 30 cm, or about 30 centimeters.

It should be understood that compositions obtained using the methods described herein are also encompassed by the disclosure. For example, in one instance, the disclosure provides a composition comprising biologically active Ig fusion proteins, e.g., LT-β-R-Ig fusion protein, wherein less than 10% of the Ig fusion proteins are biologically inactive. In another instance, less than 5% of the Ig fusion proteins are biologically inactive; less than 1% of the Ig fusion proteins are biologically inactive; or less than 0.5% of the Ig fusion proteins are biologically inactive. Compositions containing reduced levels of aggregates are also described, including a composition comprising biologically active LT-β-R-Ig fusion proteins and less than 2% protein aggregates.

III. Chromatography Techniques

The separation method of the invention can be performed on an unpurified population of polypeptides (e.g., culture supernatants or preparations of Ig fusion proteins isolated from prokaryotic inclusion bodies). Methods for obtaining such culture supernatants are described in more detail below in section IV. Alternatively, the instant separation methods can be used on polypeptide mixtures obtained after one or more initial selection or purification steps, e.g., after a preparation comprising inactive and active forms have been eluted from an affinity matrix, e.g., Protein A.

In a preferred embodiment, the chromatographic steps described herein can be applied to mixtures that have been partially purified by other protein purification procedures. The term "partially purified" as used herein includes a protein preparation in which the Ig fusion protein of interest is present at a concentration of at least 5 % by weight, more preferably at least 10% and most preferably at least 45% by weight. Initial or subsequent purification steps can be used to remove, e.g., immunoglobulin aggregates, misfolded species, host cell protein, residue material from preceding chromatographic steps (such as Protein A when employed). Accordingly, the application of the specific chromatography steps described herein can also be appreciated in the context of an overall purification protocol. Exemplary purification steps that can be used prior to or subsequent to purification include: affinity chromatography (for example, PROSEP-A® (BioProcessing Ltd., U.K.) which consists of Protein A covalently coupled to controlled pore glass or Protein A SEPHAROSE® Fast Flow (Pharmacia) or TOYOPEARL 650M Protein A (TosoHaas)). Protein A is preferred for human $\gamma 1$, $\gamma 2$, or $\gamma 4$ heavy chains and protein G for mouse isotypes. Bakerbond ABXtm resin can be used if the molecule comprises a CH3 domain.

One example of chromatography which may be used in the processes of the invention is mixed mode chromatography. A preferred mixed mode resin is one which has a dynamic binding capacity of more than 45 mg BSA/ml medium at 30 mS/cm and is a multimodal weak cation exchanger. An example of a mixed mode resin which may be used is Capto MMC (GE Healthcare).

An example of chromatography which may be used is hydrophobic interaction chromatography or HIC. The term "HIC," as used herein, refers to hydrophobic interaction chromatography which employs a hydrophobic interaction between the column and the Ig fusion protein to separate the inactive forms of the protein and other contaminants, such as aggregates, from the refolded product, *i.e.*, biologically active Ig fusion protein.

Hydrophobic interaction chromatography was first developed following the observation that proteins could be retained on affinity gels which comprised hydrocarbon spacer arms but lacked the affinity ligand. Elution from HIC supports can be effected by alterations in solvent, pH, ionic strength, or by the addition of chaotropic agents or organic modifiers, such as ethylene or propylene glycol. A description of the general principles of hydrophobic interaction chromatography can be found e.g., in U. S. Patent 3,917,527 and in U. S. Patent 4,000,098. HIC in the context of high performance liquid chromatography (HPLC) has been used to separate antibody fragments lacking heavy chain portions (e.g., $F(ab')_2$) from intact antibody molecules in a single step protocol. (Morimoto, K. et al., L Biochem. Biophys. Meth. 24: 107 (1992)).

Ion exchange chromatography may also be employed in the methods of the invention. In this regard various anionic or cationic substituents, may be attached to matrices in order to form anionic or cationic supports for chromatography. Anionic exchange substituents include diethylaminoethyl(DEAE), quaternary aminoethyl(QAE) and quaternary amine(Q) groups. Cationic exchange substituents. include carboxymethyl (CM), sulfoethyl(SE), sulfopropyl(SP), phosphate(P) and sulfonate(S). Cellulose ion exchange resins such as DE23, DE32, DE52, CM-23, CM-32 and CM-52 are available from Whatman Ltd. Maidstone, Kent, U.K. SEPHADEX®-based and low cross-linked ion exchangers are also known. For example, DEAE-, QAE-, CM-, and SP-SEPHADEX® and DEAE-, Q-, CM-and S-SEPHAROSE® and SEPHAROSE® Fast Flow are all available from Pharmacia AB. Further, both DEAE and CM derivitized ethylene glycol-methacrylate copolymer such as TOYOPEARL DEAE-650S or M and TOYOPEARL CM-650S or M are available from Toso Haas Co., Philadelphia, Pa.

Hydrophobic interactions are strongest at high ionic strength, therefore, this form of separation is conveniently performed following salt precipitations or ion exchange procedures. Adsorption of the proteins to a HIC column is favored by high salt concentrations, but the actual concentrations can vary over a wide range depending on the nature of the protein and the particular HIC ligand chosen. Various ions can be arranged in a so-called soluphobic series depending on whether they promote hydrophobic interactions (salting-out effects) or disrupt the structure of water (chaotropic effect) and lead to the weakening of the hydrophobic interaction. Cations are ranked in terms of increasing salting out effect as $Ba^{++}<; Ca^{++}<; Mg^{++}<; Li^+ <; Cs^+ <; Na^+ <; K^+ <; Rb^+ <; NH_4 4$, while anions may be ranked in terms of increasing chaotropic effect as $P0^{---} <; S0_4^{--} <; CH_3COOO^- <; Cl^- <; Br^- <; NO_3^- <; ClO_4^- <; I^- <; SCN^-$. HIC chromatography may be used in a bind and elute mode. Alternatively, HIC may be used in flowfhrough mode.

In general, Na, K or $NH_4$ sulfates effectively promote ligand-protein interaction in HIC. Salts may be formulated that influence the strength of the interaction as given by the following relationship: $(NH_4)_2SO_4 >; Na_2SO_4 >; NaCl >; NH_4Cl >; NaBr >; NaSCN$. In general, salt concentrations of between about 0.75 and about 2M ammonium sulfate or between about 1 and 4M NaCl are useful. In one embodiment, 0.3 to 0.6 M Na2SO4 is used for binding LT$\beta$R-Ig fusion protein to HIC. N.B elutions from HIC may also be achieved at salt concentrations lower than those stated here, *e.g.,* less than 0.3 Na2SO4. In one embodiment, 0.0 to 0.5 M ammonium sulfate is used to elute the protein product, including but not limited to, 0.3 to 0.5 M ammonium sulfate.

Additional purification protocols may be added including but not necessarily limited to: further ionic exchange chromatography, size exclusion chromatography, viral inactivation, concentration and freeze drying, hydroxylapatite chromatography, gel electrophoresis, dialysis, ethanol precipitation, reverse phase HPLC, chromatography on silica, chromatography on heparin SEQHAROSE™, chromatofocusing, PEG precipitation, or ammonium sulfate precipitation.

An additional purification step found in embodiments of the invention include anion exchange membrane filtration (*e.g.*, purification of protein preparations with a Q membrane adsorber). In some embodiments, protein purification with a Q membrane adsorber is performed at a pH above 6.2 and in a solution of high conductivity. As used herein, "high conductivity" refers to a conductivity of about 50 mS/cm or greater.

A number of chromatographic supports may be employed in the preparation of columns, the most extensively used are agarose, silica and organic polymer or copolymer resins. The hydrophobic interaction material is generally a base matrix (e.g., a hydrophilic carbohydrate (such as cross-linked agarose) or synthetic copolymer material) to which hydrophobic ligands (e.g., alkyl or aryl groups) are coupled. In one embodiment, the HIC material comprises an agarose resin substituted with phenyl groups. Exemplary HIC material includes: phenyl SEPHAROSE™, FAST FLOW with low or high substitution (GE Healthcare; Pharmacia LKB Biotechnology, AB, Sweden); phenyl SEPHAROSE™ High Performance column; phenyl or butyl-SEPHAROSE® CL-4B, butyl- SEPHAROSE® FF, octyl-SEPHAROSE® FF and phenyl-SEPHAROSE® FF (Pharmacia LKB Biotechnology AB, Sweden); FractogelTM EMD Propyl or FRACTOGEL™ EMC Phenyl columns (E. Merck, Germany); MACROPREP™ Methyl or MACRO-PREP™ t-Butyl Supports (Bio-Rad, California); WP HI-Propyl (C3)™ column (J.T. Baker, New Jersey).

Exemplary HIC materials are also available from Tosoh Corporation, Tokyo, Japan under the product names TOYOPEARL ether 650, phenyl 650, butyl 650 (EMD Merck), butyl 600 (EMD Merck), ether-5PW-HR, or phenyl-5PW-HR; Miles-Yeda, Rehovot, Israel under the product name alkyl-agarose, wherein the alkyl group contains from 2-10 carbon atoms, and J.T. Baker, Phillipsburg, N.J. under the product name Bakerbond WP-HI-propyl. It is also possible to prepare the desired HIC column using conventional chemistry. (See for example, Er-el. Z. et al, Biochem. Biophys. Res. Comm. 49:383 (1972) or Ulbrich,V. et al Coll. Czech. Chem. Commum. 9:1466 (1964)).

The choice of a particular gel can be determined by the skilled artisan. In general the strength of the interaction of the protein and the ligand increases with the chain length of the alkyl ligands but ligands having from about 4 to about 8 carbon atoms are suitable for most separations. A phenyl group has about the same hydrophobicity as a pentyl group, although the selectivity can be different owing to the possibility of pi-pi orbital interaction with aromatic groups on the protein. Selectively may also be affected by the chemistry of the supporting resin.

Ligand density is an important parameter in that it influences not only the strength and specificity the interaction but the capacity of the column as well. The ligand density of the commercially available phenyl or octyl phenyl gels is on the order of 40 pmoles/ml gel bed. Gel capacity is a function of the particular protein in question as well as pH, temperature and salt type and concentration but generally can be expected to fall in the range of 3-20 mg/ml of gel.

In general, a decrease in temperature decreases the interaction with the chromatography material. However, any benefit that would accrue by increasing the temperature must also be weighed against adverse effects such an increase may have on the stability of the protein.

In one embodiment, Ig fusion proteins can be eluted isocratically. In isocratic elution, all compounds begin migration through the column at onset. However, each migrates at a different rate, resulting in faster or slower elution rate.

In another embodiment, proteins of the invention can be bound to the column and eluted, e.g., using stepwise elution or gradient elution. Elution, whether stepwise or in the form of a gradient, can be accomplished in a variety of ways: (a) by changing the salt concentration, (b) by changing the polarity of the solvent or (c) by adding detergents. By decreasing salt concentration adsorbed proteins are eluted in order of increasing hydrophobicity. Changes in polarity may be affected by additions of solvents such as ethylene or propylene glycol or (iso)propanol, thereby decreasing the strength of the hydrophobic interactions. Detergents may function as displacers of proteins and may be used primarily in connection with the purification of membrane proteins

In performing the separation, the polypeptide mixture can be contacted with the chromatography material e.g., using a batch purification technique or using a column. Prior to purification it may be desirable to remove any chaotropic agents or very hydrophobic substances, e.g., by passing the mixture through a precolumn.

For example, for batch purification, chromatography material is prepared in or equilibrated to the desired starting buffer. A slurry of the material is obtained. The polypeptide solution is contacted with the slurry to adsorb at least one of the polypeptides to be separated to the chromatography material. The solution containing the polypeptides that do not bind to the chromatography material is separated from the slurry, e.g., by allowing the slurry to settle and removing the supernatant. The slurry can be subjected to one or more washing steps. If desired, the slurry can be contacted with a solution of lower conductivity to desorb polypeptides that have bound to the HIC material. In order to elute bound polypeptides, the salt concentration can be decreased.

In one embodiment, the chromatography material can be packed in a column. A mixture comprising the polypeptides to be separated can be applied to the column allowing at least one of the polypeptides to be separated to adsorb to the column. The polypeptides that do not adsorb to the column pass through and can be collected. In order to elute bound polypeptides, the salt concentration may be decreased, e.g., in a step-wise fashion or using a salt gradient, including a continuous or step salt gradient, to achieve separation of bound material through differential desorption.

Specific chromatography combinations which have been identified as being effective at separating inactive forms of an Ig fusion protein and/or protein aggregates are described further herein.

IV. Expression of Ig Fusion Proteins

The methods of the invention are used to purify mixtures of Ig fusion proteins which are first made using techniques, *e.g.*, cell culture techniques, known in the art.

Briefly, a nucleic acid encoding the Ig fusion protein is typically inserted in an expression vector for introduction into host cells that may be used to produce the desired quantity of polypeptide that, in turn, provides Ig fusion proteins for purification in accordance with the methods described herein.

The term "vector" or "expression vector" is used herein for the purposes of the specification and claims, to mean vectors used in accordance with the present invention as a vehicle for introducing into and expressing a desired gene in a cell. As known to those skilled in the art, such vectors may easily be selected from the group consisting of plasmids, phages, viruses and retroviruses. In general, vectors compatible with the instant invention will comprise a selection marker, appropriate restriction sites to facilitate cloning of the desired gene and the ability to enter and/or replicate in eukaryotic or prokaryotic cells.

For the purposes of this invention, numerous expression vector systems may be employed. For example, one class of vector utilizes DNA elements which are derived from animal viruses such as bovine papilloma virus, polyoma virus, adenovirus, vaccinia virus, baculovirus, retroviruses (RSV, MMTV or MOMLV) or SV40 virus. Others involve the use of polycistronic systems with internal ribosome binding sites. Additionally, cells which have integrated the DNA into their chromosomes may be selected by introducing one or more markers which allow selection of transfected host cells. The marker may provide for prototrophy to an auxotrophic host, biocide resistance (e.g., antibiotics) or resistance to heavy metals such as copper. The selectable marker gene can either be directly linked to the DNA sequences to be expressed, or introduced into the same cell by cotransformation. Additional elements may also be needed for optimal synthesis of mRNA. These elements may include signal sequences, splice signals, as well as transcriptional promoters, enhancers, and termination signals. In particularly preferred embodiments the cloned variable region genes are inserted into an expression vector along with the heavy and light chain constant region genes (preferably human) synthetic as discussed above. Preferably, this is effected using a proprietary expression vector of IDEC, Inc., referred to as NEOSPLA (U.S. patent 6,159,730). This vector contains the cytomegalovirus promoter/enhancer, the mouse beta globin mayor promoter, the SV40 origin of replication, the bovine growth hormone polyadenylation sequence, neomycin phosphotransferase exon 1 and exon 2, the dihydrofolate reductase gene and leader sequence. Vector systems are also taught in U.S. Pat. Nos. 5,736,137 and 5,658,570. This system provides for high expression levels, e.g., > 30 pg/cell/day. Other exemplary vector systems are disclosed e.g., in U.S. patent 6,413,777.

In other preferred embodiments, Ig fusion proteins may be expressed using polycistronic constructs such as those disclosed in copending United States provisional application No. 60/331,481 filed November 16,2001. In these novel expression systems, multiple gene products of interest such as heavy and light chains of antibodies may be produced from a single polycistronic construct. These systems advantageously use an internal ribosome entry site (IRES) to provide relatively high levels of polypeptides of the invention in eukaryotic host cells. Compatible IRES sequences are disclosed in U.S. Pat. No. 6,193,980. Those skilled in the art will appreciate that such expression systems may be used to effectively produce the full range of polypeptides disclosed in the instant application.

More generally, once the vector or DNA sequence encoding an Ig fusion protein has been prepared, the expression vector may be introduced into an appropriate host cell. That is, the host cells may be transformed. Introduction of the plasmid into the host cell can be accomplished by various techniques well known to those of skill in the art. These include, but are not limited to, transfection (including electrophoresis and electroporation), protoplast fusion, calcium phosphate precipitation, cell fusion with enveloped DNA, microinjection, and infection with intact virus. See, Ridgway, A. A. G. "Mammalian Expression Vectors" Chapter 24.2, pp. 470-472 Vectors, Rodriguez and Denhardt, Eds. (Butterworths, Boston, Mass. 1988). Most preferably, plasmid introduction into the host is via electroporation. The transformed cells are grown under conditions appropriate to the production of the light chains and heavy chains, and assayed for heavy and/or light chain protein synthesis. Exemplary assay techniques include enzyme-linked immunosorbent assay (ELISA), radioimmunoassay (RIA), or fluorescence-activated cell sorter analysis (FACS), immunohistochemistry and the like.

As used herein, the term "transformation" shall be used in a broad sense to refer to any introduction of DNA into a recipient host cell that changes the genotype and consequently results in a change in the recipient cell.

Along those same lines, "host cells" refers to cells that have been transformed with vectors constructed using recombinant DNA techniques and encoding at least one heterologous gene. In descriptions of processes for isolation of antibodies from recombinant hosts, the terms "cell" and "cell culture" are used interchangeably to denote the source of antibody unless it is clearly specified otherwise. In other words, recovery of polypeptide from the "cells" may mean either from spun down whole cells, or from the cell culture containing both the medium and the suspended cells.

The host cell line used for protein expression is most preferably of mammalian origin; those skilled in the art are credited with ability to preferentially determine particular host cell lines which are best suited for the desired gene product to be expressed therein. Exemplary host cell lines include, but are not limited to, DG44 and DUXB11 (Chinese Hamster Ovary lines, DHFR minus), HELA (human cervical carcinoma), CVI (monkey kidney line), COS (a derivative of CVI with SV40 T antigen), R1610 (Chinese hamster fibroblast) BALBC/3T3 (mouse fibroblast), HAK (hamster kidney line), SP2/O (mouse myeloma), P3.times.63-Ag3.653 (mouse myeloma), BFA-lclBPT (bovine endothelial cells), RAJI (human lymphocyte) and 293 (human kidney). CHO cells are particularly preferred. Host cell lines are typically available from commercial services, the American Tissue Culture Collection or from published literature.

*In vitro* production allows scale-up to give large amounts of the desired polypeptides. Techniques for mammalian cell cultivation under tissue culture conditions are known in the art and include homogeneous suspension culture, e.g. in an airlift reactor or in a continuous stirrer reactor, or immobilized or entrapped cell culture, e.g. in hollow fibers, microcapsules, on agarose microbeads or ceramic cartridges.

Genes encoding the polypeptide of the invention can also be expressed non-mammalian cells such as bacteria or yeast or plant cells. In this regard it will be appreciated that various unicellular non-mammalian microorganisms such as bacteria can also be transformed; i.e. those capable of being grown in cultures or fermentation. Bacteria, which are susceptible to transformation, include members of the enterobacteriaceae, such as strains of Escherichia coli or Salmonella; Bacillaceae, such as Bacillus subtilis; Pneumococcus; Streptococcus, and Haemophilus influenzae. It will further be appreciated that, when expressed in bacteria, the polypeptides typically become part of inclusion bodies. The polypeptides must be isolated, purified and then assembled into functional molecules.

In addition to prokaryotes, eukaryotic microbes may also be used. Saccharomyces cerevisiae, or common baker's yeast, is the most commonly used along eukaryotic microorganisms although a number of other strains are commonly available. For expression in Saccharomyces, the plasmid YRp7, for example, (Stinchcomb et al., Nature, 282:39 (1979); Kingsman et al., Gene, 7:141 (1979); Tschemper et al., Gene, 10:157 (1980)) is commonly used. This plasmid already contains the TRP1 gene which provides a selection marker for a mutant strain of yeast lacking the ability to grow in tryptophan, for example ATCC No. 44076 or PEP4-1 (Jones, Genetics, 85:12 (1977)). The presence of the trpl lesion as a characteristic of the yeast host cell genome then provides an effective environment for detecting transformation by growth in the absence of tryptophan.

In one embodiment, the Ig fusion proteins of the invention are expressed using mammalian cell culture at a low culturing temperature, *i. e.,* less than the conventional 37 °C or between 28 °C and 32 °C. Low culturing of Ig fusion proteins, particularly TNF receptor-Ig fusion proteins, decreases the overall percentage of inactive fusion protein.

Methods of culturing Ig fusion proteins at a low temperature are described in US 20020039580 (Browning et al.).

In one embodiment, the starting material for purification using the methods of the invention is a supernatant from CHO cells which express recombinant Ig fusion protein. In one embodiment, the CHO cells are cultured at a temperature of about 28°C to 32 °C. In one embodiment, the starting material for purification comprises approximately 20-40% aggregated material. In one embodiment, the starting material for purification comprises approximately 30% aggregated material. In one alternative, the starting material comprises approximately 50% or more than 50% aggregated material.

Prior to purification using the subject methods, the composition comprising the mixture of polypeptides to be separated will preferably be placed in a buffer of acidic or approximately neutral pH. This can be done, for example, by adding concentrated buffer, resuspending the sample in the buffer, exchanging the buffer (e.g., using dialysis or ultrafiltration). Alternatively, the pH of the sample buffer can simply be adjusted to be within the desired range.

V. Immunoglobulin Fusion Proteins

The methods of the invention achieve purified compositions of LTβR-Ig fusion protein.

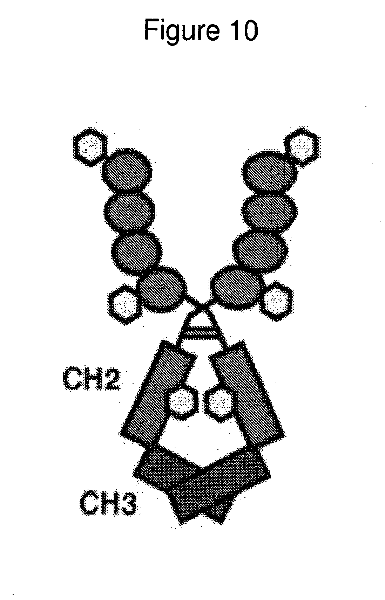

In one embodiment, LTβR-Ig fusion protein comprises the extracellular domain of human LTβR (form lacking the transmembrane domain) and the immunoglobulin Fc region (IgG1 Fc domain). SEQ ID NO: 1 describes the full length sequence of the human LTβR, including the extracellular domain. A schematic of the LT-β-R-Ig fusion is provided in Figure 10.

Fusion proteins can be prepared using methods that are well known in the art (see for example US Patent Nos. 5,116,964 and 5,225,538). Ordinarily, the ligand or ligand binding partner is fused C-terminally to the N-terminus of the constant region of the heavy chain (or heavy chain portion) and in place of the variable region. Any transmembrane regions or lipid or phospholipids anchor recognition sequences of ligand binding receptor are preferably inactivated or deleted prior to fusion. DNA encoding the ligand or ligand binding partner is cleaved by a restriction enzyme at or proximal to the 5' and 3'ends of the DNA encoding the desired ORF segment. The resultant DNA fragment is then readily inserted into DNA encoding a heavy chain constant region. The precise site at which the fusion is made may be selected empirically to optimize the secretion or binding characteristics of the soluble fusion protein. DNA encoding the fusion protein is then transfected into a host cell for expression.

VI. Pharmaceutical Compositions

The methods of the invention are particularly well suited for the purification of Ig fusion proteins for use in a Pharmaceutical formulation, as inactive and/or protein aggregates can have deleterious effects if administered to a subject in a therapy. For example, inactive forms of the fusion protein and protein aggregates may result in loss of efficacy of the Ig fusion protein or increased immunogenicity.

Methods of preparing and administering proteins obtained using the methods of the invention to a subject are well known to or are readily determined by those skilled in the art.

A soluble LT-β-R-Ig can be formulated as a pharmaceutical composition, e.g., for administration to a subject to treat an autoimmune disorder, e.g., a demyelinating disorder, such as Multiple Sclerosis. Typically, a pharmaceutical composition includes a pharmaceutically acceptable carrier. As used herein, "pharmaceutically acceptable carrier" includes solvents, dispersion media, coatings, antibacterial and antifungal agents, isotonic and absorption delaying agents, and the like that are physiologically compatible. The composition can include a pharmaceutically acceptable salt, e.g., an acid addition salt or a base addition salt (see e.g., Berge et al., J. Pharm. Sci. 66:1-19, 1977).

The soluble LT-β-R-Ig can be formulated according to standard methods. Pharmaceutical formulation is a well-established art, and is further described, e.g., in Gennaro (ed.), Remington: The Science and Practice of Pharmacy, 20th ed., Lippincott, Williams & Wilkins (2000) (ISBN: 0683306472); Ansel et al, Pharmaceutical Dosage Forms and Drug Delivery Systems, 7th Ed., Lippincott Williams & Wilkins Publishers (1999) (ISBN: 0683305727); and Kibbe (ed.), Handbook of Pharmaceutical Excipients American Pharmaceutical Association, 3rd ed. (2000) (ISBN: 091733096X).

A soluble LT-β-R-Ig can be formulated with excipient materials, such as sodium chloride, sodium dibasic phosphate heptahydrate, sodium monobasic phosphate, and a stabilizer. It can be provided, for example, in a buffered solution at a suitable concentration and can be stored at 2-8°C.

The pharmaceutical compositions may be in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form can depend on the intended mode of administration and therapeutic application. Typically compositions for the agents described herein are in the form of injectable or infusible solutions.

Such compositions can be administered by a parenteral mode (e.g., intravenous, subcutaneous, intraperitoneal, or intramuscular injection). The phrases "parenteral administration" and "administered parenterally" as used herein mean modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, intracerebral, intracranial, intracarotid and intrastemal injection and infusion.

The composition can be formulated as a solution, microemulsion, dispersion, liposome, or other ordered structure suitable for stable storage at high concentration.

Sterile injectable solutions can be prepared by incorporating an agent described herein in the required amount in an appropriate solvent with one or a combination of ingredients enumerated above, as required, followed by filtered sterilization. Generally, dispersions are prepared by incorporating an agent described herein into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of an agent described herein plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The soluble LT-β-R-Ig can be prepared with a carrier that will protect the compound against rapid release, such as a controlled release formulation, including implants, and microencapsulated delivery systems. Biodegradable, biocompatible polymers can be used, such as ethylene vinyl acetate, polyanhydrides, polyglycolic acid, collagen, polyorthoesters, and polylactic acid.

Many methods for the preparation of such formulations are patented or generally known. See, e.g., Sustained and Controlled Release Drug Delivery Systems, J.R. Robinson, ed., Marcel Dekker, Inc., New York, 1978.

A soluble LT-β-R-Ig can be modified, e.g., with a moiety that improves its stabilization and/or retention in circulation, e.g., in blood, serum, or other tissues, e.g., by at least 1.5, 2, 5, 10, or 50 fold. The modified agent can be evaluated to assess whether it can reach sites of inflammation (e.g., lesions or scleroses) such as can occur in a demyelinating disorder, such as Multiple Sclerosis (e.g., by using a labeled form of the agent).

For example, the LT-β-R-Ig can be associated with a polymer, e.g., a substantially non-antigenic polymer, such as a polyalkylene oxide or polyethylene oxide. Suitable polymers will vary substantially by weight. Polymers having molecular number average weights ranging from about 200 to about 35,000 Daltons (or about 1,000 to about 15,000, and 2,000 to about 12,500) can be used.

For example, a soluble LT-β-R-Ig can be conjugated to a water soluble polymer, e.g., a hydrophilic polyvinyl polymer, e.g., polyvinylalcohol or polyvinylpyrrolidone. A non-limiting list of such polymers include polyalkylene oxide homopolymers such as polyethylene glycol (PEG) or polypropylene glycols, polyoxyethylenated polyols, copolymers thereof and block copolymers thereof, provided that the water solubility of the block copolymers is maintained. Additional useful polymers include polyoxyalkylenes such as polyoxyethylene, polyoxypropylene, and block copolymers of polyoxyethylene and polyoxypropylene (Pluronics); polymethacrylates; carbomers; and branched or unbranched polysaccharides.

When the soluble LT-β-R-Ig is used in combination with a second agent, the two agents can be formulated

**EP 2 260 054 B1**

separately or together. For example, the respective pharmaceutical compositions can be mixed, e.g., just prior to administration, and administered together or can be administered separately, e.g., at the same or different times.

A soluble LT-β-R-Ig can be administered to a subject, e.g., a human subject, by a variety of methods. For many applications, the route of administration is one of: intravenous injection or infusion (IV), subcutaneous injection (SC), intraperitoneally (IP), or intramuscular injection. In some cases, administration may be directly into the CNS, e.g., intrathecal, intracerebroventricular (ICV), intracerebral or intracranial. The agent can be administered as a fixed dose, or in a mg/kg dose.

The dose can also be chosen to reduce or avoid production of antibodies against the agent.

The route and/or mode of administration of the soluble LT-β-R-Ig can also be tailored for the individual case, e.g., by determining the location, number or size of scleroses in a subject, e.g., using Magnetic Resonance Imaging (MRI) scans, Positron- Emission Tomography (PET) scans, Diffusion-Weighted Imaging (DW-I, or DW- MRI), Diffusion Tensor Imaging, Myelography, Magnetization Transfer. The severity or extent of a demyelinating disorder can also be determined from lumbar puncture (e.g., to check for elevated white cells in the cerebral-spinal fluid), evoked potential testing as a measure of nerve function, and/or any other standard parameters associated with a demyelinating disorder (e.g., Multiple Sclerosis), e.g., any of the assessment criteria described herein.

Dosage regimens are adjusted to provide the desired response, e.g., a therapeutic response or a combinatorial therapeutic effect. The dosage regimen will, for example, cause an increase in remyelination. Generally, a dose of a soluble LT-β-R-Ig optionally formulated separately or together with an appropriate dose of a second therapeutic agent can be used to provide a subject with the soluble LT-β-R-Ig.

Suitable dosages and/or dose ranges for the soluble LT-β-R-Ig include an amount sufficient to cause increased remyelination in a subject. Suitable dosages can be any of those described herein and include, for example, a dose of at least about 0.001 mg of a soluble LT-β-R-Ig per kg body weight of a subject (e.g., a human patient).

A dose of a soluble LT-β-R-Ig required to increase remyelination can depend on a variety of factors including, for example, the age, sex, and weight of a subject to be treated. Other factors affecting the dose administered to the subject include, e.g., the type or severity of the demyelinating disorder. For example, a patient with Acute Fulminant Multiple Sclerosis may require a administration of a different dosage of a soluble LT-β-R-Ig than a patient with a milder form of Multiple Sclerosis. Other factors can include, e.g., other disorders concurrently or previously affecting the patient, the general health of the patient, the genetic disposition of the patient, diet, time of administration, rate of excretion, drug combination, and any other additional therapeutics that are administered to the patient. It should also be understood that a specific dosage and treatment regimen for any particular patient will depend upon the judgment of the treating physician. The amount of active ingredients will also depend upon the particular described compound and the presence or absence and the nature of the additional anti-viral agent in the composition.

Dosage unit form or "fixed dose" as used herein refers to physically discrete units suited as unitary dosages for the subjects to be treated; each unit contains a predetermined quantity of active compound calculated to produce the desired therapeutic effect (e.g., an crease in remyelination in a subject) in association with the required pharmaceutical carrier and optionally in association with the other agent.

A pharmaceutical composition may include a therapeutically effective amount of a soluble LT-β-R-Ig described herein. Such effective amounts can be determined based on the effect of the administered agent, or the combinatorial effect of an agent and secondary agent if more than one agent is used. A therapeutically effective amount of an agent can also vary according to factors such as the disease state, age, sex, and weight of the individual, and the ability of the compound to elicit a desired response in the individual, e.g., amelioration of at least one disorder parameter, e.g., amelioration of at least one symptom of an autoimmune disorder, e.g., a demyelinating disorder, e.g., Multiple Sclerosis. For example, a therapeutically effective amount of soluble LT-β-RIg will increase remyelination and can also slow and/or ameliorate demyelination. A therapeutically effective amount is also one in which any toxic or detrimental effects of the composition is outweighed by the therapeutically beneficial effects.

Devices and Kits Pharmaceutical compositions that include a soluble LT-β-R-Ig can be administered with a medical device. The device can be designed with features such as portability, room temperature storage, and ease of use so that it can be used in emergency situations, e.g., by an untrained subject or by emergency personnel in the field, removed to medical facilities and other medical equipment. The device can include, e.g., one or more housings for storing pharmaceutical preparations that include a soluble LT-β-R-Ig, and can be configured to deliver one or more unit doses of the agent.

For example, the pharmaceutical composition can be administered with a transcutaneous delivery device, such as a syringe, including a hypodermic or multichamber syringe. Other suitable delivery devices include stents, catheters, transcutaneous patches, microneedles, and implantable controlled release devices. The device (e.g., a syringe) can include a soluble LT-β-R-Ig in a dry or liquid form at a dose sufficient to cause remyelination. The device can also be a dual-chambered device, wherein one chamber contains a unit dose of lyophilized soluble LT-β-R-Ig sufficient to cause increased remyelination in a subject, and a second chamber containing a liquid (e.g., a buffer) for reconstituting the lyophilized unit dose of a soluble LT-β-R-Ig.

16

In other examples, the pharmaceutical composition can be administered with a needleless hypodermic injection device, such as the devices described in US 5,399,163; 5,383,851; 5,312,335; 5,064,413; 4,941,880; 4,790,824; or 4,596,556. Examples of well-known implants and modules are described in, e.g., US 4,487,603, which discloses an implantable micro-infusion pump for dispensing medication at a controlled rate; US 4,486,194, which discloses a therapeutic device for administering medications through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,196, which discloses an osmotic drug delivery system. Many other devices, implants, delivery systems, and modules are also known.

A soluble LT-β-R-Ig can be provided in a kit. In one instance, the kit includes (a) a container that contains a composition that includes one or more unit doses of a soluble LT-β-R-Ig, and optionally (b) informational material.

The unit doses of soluble LT-β-R-Ig are sufficient to cause a desired result in a subject, e.g., slowed progression of a disorder or improvement in a subject. The informational material can be descriptive, instructional, marketing or other material that relates to the methods described herein and/or the use of the agents for therapeutic benefit. The kit can also include reagents and instructions useful in the testing (assaying) for remyelination. Such methods of assaying for remyelination include, but are not limited to, any of the testing methods described herein. In one instance, the kit includes one or more additional agents for treating a demyelinating disorder, such as one or more agents to treat Multiple Sclerosis. For example, the kit includes a first container that contains a composition that includes the soluble LT-β-R-Ig, and a second container that includes the one or more additional agents.

The informational material of the kits is not limited in its form. In one instance, the informational material can include information about production of the compound, molecular weight of the compound, concentration, date of expiration, batch or production site information, and so forth. In one instance, the informational material relates to methods of administering the soluble LT-β-R-Ig e.g., in a suitable dose, dosage form, or mode of administration (e.g., a dose, dosage form, or mode of administration described herein), to treat a subject who has a demyelinating disorder, or who is at risk of developing, or for experiencing an episode associated with a demyelinating disorder. The information can be provided in a variety of formats, including printed text, computer readable material, video recording, or audio recording, or a information that provides a link or address to substantive material.

In addition to the agent, the composition in the kit can include other ingredients, such as a solvent or buffer, a stabilizer, or a preservative. The agent can be provided in any form, e.g., liquid, dried or lyophilized form, preferably substantially pure and/or sterile. When the agents are provided in a liquid solution, the liquid solution preferably is an aqueous solution. When the agents are provided as a dried form, reconstitution generally is by the addition of a suitable solvent. The solvent, e.g., sterile water or buffer, can optionally be provided in the kit.

The kit can include one or more containers for the composition or compositions containing the agents. In some instances, the kit contains separate containers, dividers or compartments for the composition and informational material. For example, the composition can be contained in a bottle, vial, or syringe, and the informational material can be contained in a plastic sleeve or packet. In other instances, the separate elements of the kit are contained within a single, undivided container. For example, the composition is contained in a bottle, vial or syringe that has attached thereto the informational material in the form of a label. In some instances, the kit includes a plurality (e.g., a pack) of individual containers, each containing one or more unit dosage forms (e.g., a dosage form described herein) of the agents. The containers can include a combination unit dosage, e.g., a unit that includes both the soluble LT-β-R-Ig and the second agent, e.g., in a desired ratio. For example, the kit includes a plurality of syringes, ampules, foil packets, blister packs, or medical devices, e.g., each containing a single combination unit dose. The containers of the kits can be air tight, waterproof (e.g., impermeable to changes in moisture or evaporation), and/or light-tight.

The kit optionally includes a device suitable for administration of the composition, e.g., a syringe or other suitable delivery device. The device can be provided pre-loaded with one or both of the agents or can be empty, but suitable for loading.

VIII. Methods of Treatment

Formulations made using active LT-β-R-Ig fusion proteins purified in accordance with the methods described herein, may be used to treat a number of diseases which are treatable with an LT-β-R blocking agent. Examples of such diseases include autoimmune diseases, such as rheumatoid arthritis, intestinal diseases, such as Crohn's disease, and neurological diseases, such as multiple sclerosis. Other examples of diseases which may be treated using compositions described herein are described in US 20020197254 and US 7,255,854.

A pharmaceutical compositon comprising purified LT-β-R-Ig fusion proteins may be used to treat a demyelinating disorder. As used herein, a "demyelinating disorder" is any disease associated with the destruction or removal of myelin, the fatty sheath surrounding and insulating nerve fibers, from nerves. Demyelinating disorders include, for example, Multiple Sclerosis (e.g., Relapsing/Remitting Multiple Sclerosis, Secondary Progressive Multiple Sclerosis, Progressive Relapsing Multiple Sclerosis, Primary Progressive Multiple Sclerosis, and Acute Fulminant Multiple Sclerosis), Central Pontine Myelinolysis, Acute Disseminated Encephalomyelitis, Progressive Multifocal Leukoencephalopathy; Subacute Sclerosing Panencephalitis, Post-infectious Encephalomyelitis, Chronic Inflammatory Demyelinating Polyneuropathy, Guillain-Barre Syndrome, Progressive Multifocal Leucoencephalopathy, Devic's Disease, Balo's Concentric Sclerosis, and a leukodystrophy (e.g., Metachromatic Leukodystrophy, Krabbe disease, Adrenoleukodystrophy, Pelizaeus-Merzbacher disease, Canavan disease, Childhood Ataxia with Central Hypomyelination, Alexander disease, or Refsum disease). A human patient having a demyelinating disorder can have one or more symptoms of a demyelinating disorder such as, but not limited to, impaired vision, numbness, weakness in extremities, tremors or spasticity, heat intolerance, speech impairment, incontinence, dizziness, or impaired proprioception (e.g., balance, coordination, sense of limb position).

A human (e.g., a human patient) with a family history of a demyelinating disorder (e.g., a genetic predisposition for a demyelinating disorder), or who exhibits mild or infrequent symptoms of a demyelinating disorder described above can be, for the purposes of the method, considered at risk of developing a demyelinating disorder (e.g., Multiple Sclerosis) and may be treated with a pharmaceutical composition as described herein.

In some instances, the soluble LT-$\beta$-R-Ig can be administered to the subject in an amount, frequency, and/or for a time sufficient to improve a condition in a subject. In one instance, a composition of the disclosure can be used to induce or promote remyelination in a subject.

In one instance, the soluble LT-$\beta$-R-Ig is administered to the subject once. In other instances, the soluble LT-$\beta$-R-Ig is administered to the subject more than once, e.g., once every 3-10 days; at least twice and not more than once every 5-20 days; at least twice and not more than once every 28-3 1 days; weekly; biweekly; monthly; weekly over the course of at least 4 weeks; biweekly over the course of at least 6 weeks; monthly over the course of at least 3 months; or monthly over the course of at least 6 months.

In some instances, a suitable starting dose of soluble LT-$\beta$-R-Ig in trials to determine a dosage (e.g., the amount sufficient to induce remyelination in a subject) is 0.001 mg of soluble LT-$\beta$-R-Ig per kg body weight of the subject. In some instances, a suitable dose or starting dose is determined by a number of subjective, patient-specific factors such as, but not limited to, sex, age, weight, physical health, or any other factor described herein.

In some instances, the soluble LT-$\beta$-R-Ig is administered to a subject intravenously or parenterally (e.g., intrathecally, subcutaneously, intramuscularly, intranasally, or orally).

In some instances, the soluble LT-$\beta$-R-Ig can be administered to a subject as a monotherapy. In some instances, the soluble LT-$\beta$-R-Ig can be administered to a subject as a combination therapy with another treatment, e.g., another treatment for an autoimmune disorder, for example, a demyelinating disorder (e.g., any of the demyelinating disorders described herein (e.g., Multiple Sclerosis)). For example, the combination therapy can include administering to the subject (e.g., a human patient) one or more additional agents that provide a therapeutic benefit to the subject who has, or is at risk of developing, an autoimmune disorder, e.g., a demyelinating disorder.

In some instances, the soluble LT-$\beta$-R-Ig and the one or more additional agents are administered at the same time. In other instances, the soluble LT-$\beta$-R-Ig is administered first in time and the one or more additional agents are administered second in time. In some instances, the one or more additional agents are administered first in time and the soluble LT-$\beta$-R-Ig is administered second in time. The soluble LT-$\beta$-R-Ig can replace or augment a previously or currently administered therapy. For example, upon treating with LT-$\beta$-R-Ig, administration of the one or more additional agents can cease or diminish, e.g., be administered at lower levels. In other instances, administration of the previous therapy is maintained. In some instances, a previous therapy will be maintained until the level of LT-$\beta$-R-Ig reaches a level sufficient to provide a therapeutic effect. The two therapies can be administered in combination.

In one instance, a subject being treated for a demyelinating disorder using a pharmaceutical composition as described herein may be monitered for remyelination. Monitoring a subject (e.g., a human patient) for remyelination, as defined herein, means evaluating the subject for a change, e.g., an improvement in one or more parameters that are indicative of remyelination, e.g., one can monitor improvement in one or more symptoms of a demyelinating disorder. Such symptoms include any of the symptoms of a demyelinating disorder described herein. Remyelination can also be monitored by methods which include direct determination of the state of myelin in the subject, e.g., one can measure white matter mass using magnetic resonance imaging (MRI) or measure the thickness of myelin fibers using a magnetic resonance spectroscopy (MRS) brain scan. In some instances, the evaluation is performed at least 1 hour, e.g., at least 2,4, 6, 8, 12, 24, or 48 hours, or at least 1 day, 2 days, 4 days, 10 days, 13 days, 20 days or more, or at least 1week, 2 weeks, 4 weeks, 10 weeks, 13 weeks, 20 weeks or more, after an administration, preferably the first administration, of the soluble LT-$\beta$-R-Ig. The subject can be evaluated in one or more of the following periods: prior to beginning of treatment; during the treatment; or after one or more elements of the treatment have been administered. Evaluating can include evaluating the need for further treatment, e.g., evaluating whether a dosage, frequency of administration, or duration of treatment should be altered. It can also include evaluating the need to add or drop a selected therapeutic modality, e.g., adding or dropping any of the treatments for demyelinating disorders described herein. For example, continued administration of the soluble LT-$\beta$-R-Ig could be done with one or more additional treatment agents where necessary. If a preselected outcome of the evaluation is obtained, an additional step is taken, e.g., the subject is administered another treatment or another evaluation or test is performed..

For example, a lumbar puncture (i.e., a spinal tap) can be performed on a patient to obtain a sample of cerebrospinal fluid. The cerebrospinal fluid is then tested for the presence of, e.g., (i) abnormal proteins such as tiny fragments of myelin, (ii) elevated levels of or specific types of lymphocytes, and/or (iii) abnormal levels of immunoglobulin (IgG) molecules. Another example of a quantitative test for a demyelinating disorder is an evoked potential test, which measures nerve activity as a function of how long it takes nerve impulses from the eye, ear, or skin to reach the brain. A demyelinating disorder can also be assessed by evaluating the size and/or number of inflammatory lesions (i.e., scleroses) present at the central nervous system using any of several methods of imaging including, but not limited to, Magnetic Resonance Imaging (MRI) scans, Positron-Emission Tomography (PET) scans, Diffusion-Weighted Imaging (DW-I, or DW-MRI), Diffusion Tensor Imaging, Myelography, Magnetization Transfer. Patients can also be diagnosed using a variety of semiquantitative or qualitative assessments of their neuropsychology (e.g., the status of various abilities such as memory, arithmetic, attention, judgment and reasoning) or symptoms (clinical parameters) presented by the patient including, e.g., any of the symptoms of Multiple Sclerosis described above. Additionally, the extent or progression of a demyelinating disorder can be detected by testing a patient's urine for elevated levels of myelin basic protein- like material (MBPLM), which substance becomes elevated as axonal damage occurs during disease progression (see, for example, Whitaker et al. (1995) Ann. Neural. 38(4):635-632). Certain tests for color blindness can also be helpful in tracking the effect of demyelinating disorders on the eyes.

Such diagnostic methods described above can also be used to evaluate increased remyelination in a subject (e.g., a patient) following treatment with a soluble LT-β-R-Ig For example, remyelination can coincide with a reduction in the size or number of scleroses present in a patient as determined through any of the imaging methods described herein. Also, remyelination in a subject could be measured as an increase in the speed of transmission of a signal from the ears, eyes, or skin to the brain, as determined through evoked potential testing. In some cases, remyelination can be evaluated as an increase in white matter volume (e.g., nerve mass of the spine or brain), particularly where the demyelinating disorder has resulted in nerve atrophy. In some instances, the extent or occurrence of remyelination in a subject can be assessed by directly measuring the thickness of myelin in a subject using, e.g., magnetic resonance spectroscopy scans.

The present disclosure also includes the use of the methods and compositions described herein in combination with therapies or medicaments that can cause demyelinating conditions. For example, anti-TNF therapy for treatment of rheumatoid arthritis, as a side-effect, can result in a type of demyelinating condition. Thus, a soluble LT-β-R-Ig can be administered (e.g, co-administered) in combination with an anti-TNF therapy to prevent, ameliorate, or reverse the demyelination side-effects and to promote remyelination. Anti-TNF therapies include, but are not limited to, adalimumab (Humira), etanercept (Enbrel), or infliximab (Remicade).

In some instances, a soluble LT-β-R (e.g., LT-β-R-Fc) is used as a second line therapy. For example, a patient who is determined to be unresponsive to one or more therapies for a demyelinating disorder (e.g., Multiple Sclerosis) will stop receiving the one or more treatments and will begin treatment with a soluble LT-β-R-Ig.

The foregoing disclosure teaches to those of skill in the art the aspects of the invention including how to make and use the invention. The following examples are meant to provide further elucidation of the invention but are not meant as limitations thereof.

**EXAMPLES**

The following examples describe methods for purifying an antibody based biologic, *i.e.,* LT-β-R-Ig, from product related impurities. Three product related impurities are co-expressed with LT-β-R-Ig when expressed in CHO cells. These product related impurities present a challenge in the purification process. The impurities include two monomeric but structurally-distinct inactive forms of LT-β-R-Ig (termed "Inactive-1" and "Inactive-2") and aggregates, which included dimeric and higher molecular weight material. An example of the percentages of impurities versus protein product following expression of the Fc fusion protein in CHO cells is about 46% product (active, monomeric protein), about 35% aggregate, about 13% Inactive-2, and about 6% Inactive-1.

**EXAMPLE 1: DEVELOPMENT OF A HIGH RESOLUTION HYDROPHOBIC INTERACTION CHROMATOGRAPHY PROCESS CAPABLE OF REMOVING MULTIPLE PRODUCT RELATED IMPURITIES DURING THE PURIFICATION OF AN ANTIBODY BASED BIOLOGIC (CYCLE 1)**

An HIC process step capable of removing two monomeric, but inactive, product-related impurities from an antibody-based product was developed for phase 1 clinical manufacturing. During subsequent development, improvements in capacity and robustness were sought while maintaining chromatographic resolution. Developmental studies will be described in which alternative HIC resins and binding salts were explored, and parameters critical for the separation efficiency were identified by a design-of-experiments approach. A second HIC step with distinct resolving power was employed downstream in the same process for the removal of a third product-related impurity. Issues and challenges encountered in the establishment of a robust process appropriate for manufacturing-scale clinical production will be discussed.

The following example describes a hydrophobic interaction chromatography (HIC) process that is capable of removing product-related impurities from an antibody-based product, *e.g.,* two inactive forms of the protein, that was developed for clinical manufacturing. The following example describes HIC butyl and phenyl resins, binding salts, and parameters identified as important to separation efficiency.

Overall, the following process (referred to as "Cycle 1-1" and "Cycle 1-2") used a first HIC butyl column, *e.g.,* Butyl-650M resin (Tosoh Biosciences) followed by a second HIC phenyl column. The combination of HIC processes resulted in a decrease in inactive and aggregated molecules. The Butyl-650M resin was determined to be effective at separating active LT-β-R-Ig from the other forms, *i.e.*, inactive-1, inactive-2, and aggregate forms. Cycle 1-1 was used for 2000 L manufacturing of LT-β-R-Ig, whereas Cycle 1-2 was optimized for a higher titer starting material (*i.e.,* 800 mg/mL). Cycles 1-1 and 1-2 both included a first butyl HIC separation and a second phenyl HIC separation.

MATERIALS AND METHODS

Resins used in this example included the Toyopearl Butyl-650M (Tosoh Bioscience) and the Phenyl Sepharose 6 Fast Flow (Pharmacia). The Butyl-650M chromatography process included an equilibration and wash step using a solution comprising 20 mM Na Phosphate, pH 7.5, 1.65 M NaCl, and an elution step using a solution comprising 20 mM Na Phosphate, pH 7.5, 0.7 M NaCl.

Column effluent was monitored by Absorbance 280 nm ($A_{280}$) using Pharmacia UV-1 detectors. The AKTA FPLC from Pharmacia and Unicorn software version 3.2.6 were used for various experiments.

Except where noted, chromatography was performed at temperatures ranging from 18 - 28 °C. Column temperatures were regulated in specific cases using Torrey Pines Scientific HPLC Column Chiller/Heater model C030. Temperatures for the purification solutions were controlled in some experiments using Neslab water bath model RTE-211.

Statistical analysis was performed using JMP® software (SAS Institute) version 3.2.6 was utilized for experimental design and result analysis.

LT-β-R-Ig protein concentrations throughout this report are based on an extinction coefficient of 1.25.

**1.1 CYCLE 1-1**

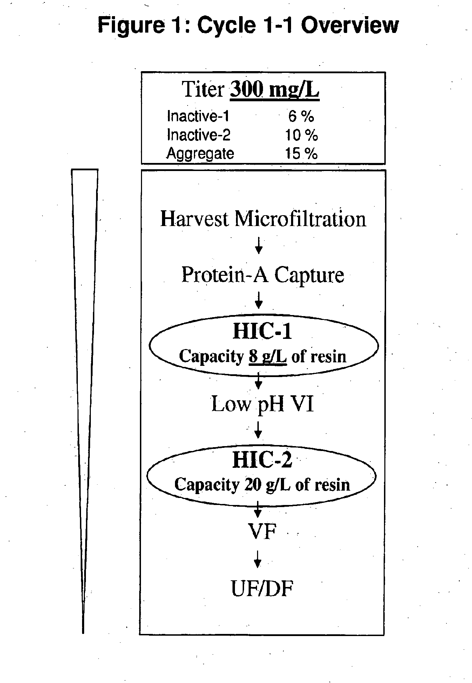

Cycle 1-1 was developed to remove impurities from LT-β-R-Ig expressed in mammalian cell culture. Impurities which needed to be removed included two different inactive forms of the LT-β-R-Ig protein, *i.e.,* inactive-1 and inacative-2 forms, as well as aggregates. A summary of the process of cycle 1-1 is described in Figure 1.

LTBR-Ig was first expressed in recombinant Chinese Hamster Ovary (CHO) cells using a fed-batch process. In "Cycle-1-1, titer was ~300 mg/L with aggregate at ~12%, Inactive-1 at ~5% and Inactive-2 at ~9%. Following expression, the cell culture was harvested and centrifuged. The clarified conditioned media was processed over a Protein-A Capture column. The resulting LT-β-R-Ig mixture was processed over a butyl resin HIC column (referred to as HIC-1) (Butyl-650M; Tosoh Bioscience), where the resin had a capacity of 8 g/L. The HIC-1 step cleared the mixture of inactive forms 1 and 2 of LT-β-R-Ig and was optimized for protein capacity.

The Butyl-650M resin was chosen for a number of reasons over other tested resins, including the following preferable characteristics: 1) a rigid methacrylate backbone; 2) 40 - 90 μm particle size; 3) operating velocity of 80 - 300 cm/hr; and 4) stringent cleaning conditions possible, *e.g.,* 1 N NaOH, 50 % Methanol. The butyl resin was able to remove more than 99% of the inactive forms of LT-β-R-Ig.

A number of salts were screened to determine which was most effective at resolving the LT-β-R-Ig protein product from the inactive and aggregate forms. NaCl and $Na_2SO_4$ were both identified as being able to resolve the impurities from the LTBR-Ig product. Inactive-1 and Inactive-2 forms for LTBR-Ig were eluted from the butyl resin in the wash and strip NaCl solutions, respectively. The butyl HIC-1 step reduced Inactive-1 and Inactive-2 to < 1.0 % in the eluate fraction.

Figure 3 describes an HPLC analysis of process intermediates following HIC-1, including HIC-HLPC that resolves both inactive forms of LT-β-R-Ig from the active LT-β-R-Ig and aggregates, and size exclusion HPLC (SE-HPLC) that resolves aggregate from active, monomeric LT-β-R-Ig fusion protein.

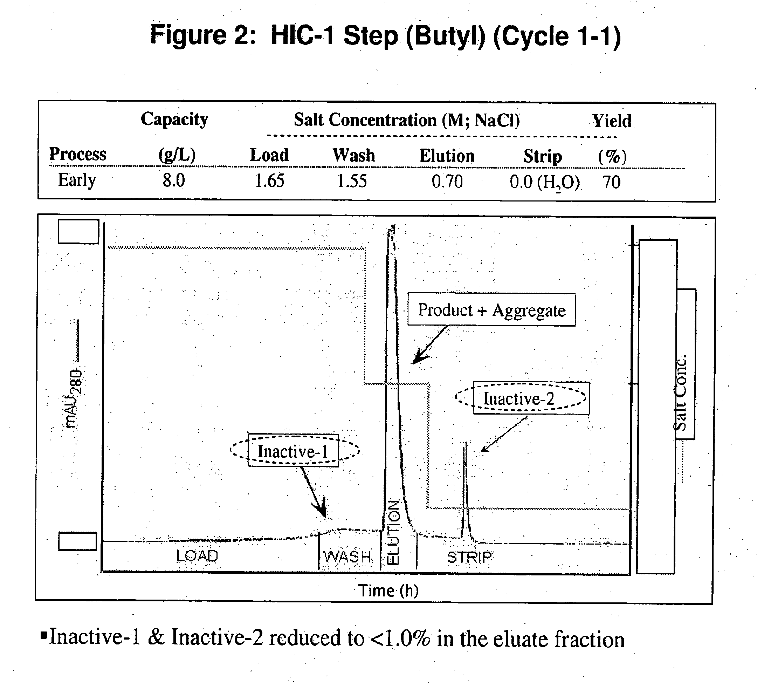

A summary of the results following the HIC-1 butyl step is provided in Figure 2, where the two inactive forms of LT-β-R-Ig were separated from the active LT-β-R-Ig. As described in Figure 2, the LT-β-R-Ig product was eluted from the HIC-1 butyl resin using 0.7 M NaCl. Final Cycle-1-1 conditions for HIC-1 were: bind at 1.65 M NaCl (everything bound), then wash at 1.55 M NaCl (Inactive-1 was washed off the column) then eluate at 0.7 M NaCl (Product and aggregate eluated) then strip at 0 M NaCl (Inactive-2 emerged from the column) (also see Figure 2).

The eluate from the HIC-1 step was then virally inactivated using a low pH process, and subsequently processed over a second HIC chromatography column using phenyl sepharose (Phenyl Sepharose 6 Fast Flow Hi Sub).

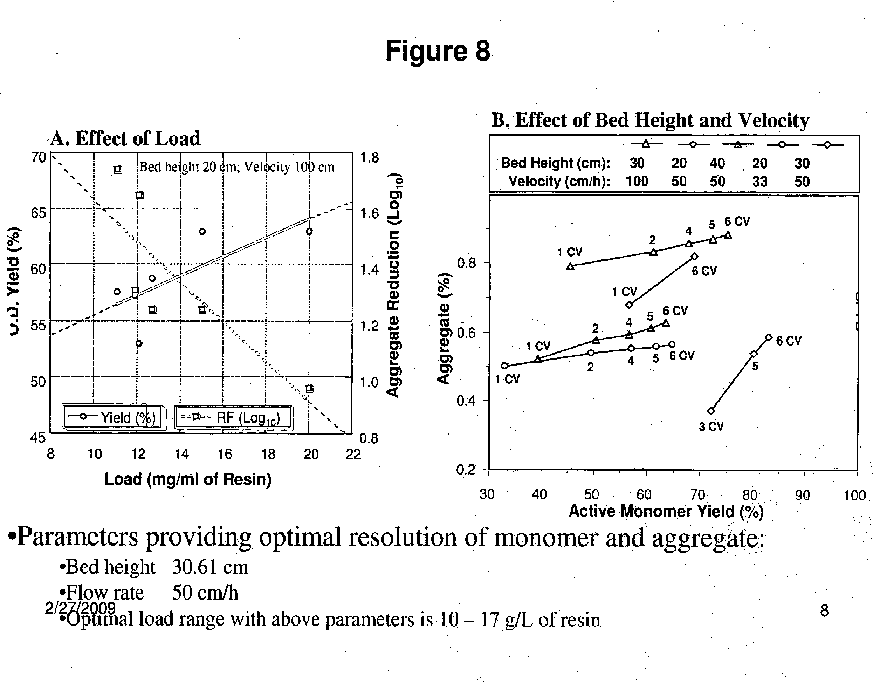

The second HIC step (phenyl) resulted in clearance of protein aggregates and optimized resolution of the eluate. Results showing aggregate clearance by the HIC-2 (phenyl) step are described in Figure 8A, including the effect of load.

Following the second HIC (HIC-2) step, the eluate was further processed through virus removal filters (VF) and ultrafiltration/diafiltration (UF/DF).

### 1.2 CYCLE 1-2

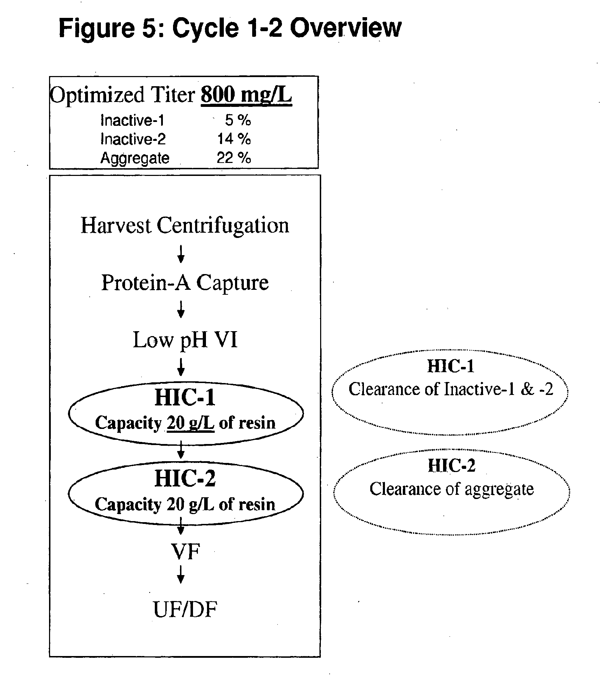

Cycle 1-2 was a modified version of Cycle 1-1 described above, where Cycle 1-2 had an improved ability to increase capacity (protein titer in the starting material increased). An overview of Cycle 1-2 is provided in Figure 5. As described below, in Cycle 1-2 sodium sulfate was used (instead of sodium chloride) to increase capacity on the Butyl column from 8 to 20 g/L of resin.

Different lyotrophic salts were screened for both improved capacity characteristics and the ability to resolve active vs. inactive (forms 1 and 2) of LT-$\beta$-R-Ig. A screen was performed to determine salts and resins that could effectively remove the inactive and aggregated forms of LT-$\beta$-R-Ig following the increase in titer. The results from the screen of lyotrophic salts is described in Table 1.

Table 1: Screen of Lyoptrophic Salts

| Salt | Conc. (M) | Capacity | Resolution: Monomer / Inactive 1 / Inactive 2 |
|---|---|---|---|
| NaCl | 1.6 | Low | High |
| $(NH_4)_2SO_4$ | 0.6 | High | Low |
| $K_n(H_n)PO_4$ | 1.0 | High | Low |
| $Na_2SO_4$ | 0.6 | High | High |

Sodium sulphate was chosen for its ability to promote a high capacity at a low salt concentration, is comparable in cost to NaCl and is waste-management friendly, despite the fact that it is solubility sensitive to temperature and other salts, and has a maximum solubility of about 1.3 M at ambient temperature.

Results from the resin screen are provided in Table 2. A screen of HIC resins using various binding salts suggested that butyl resin-2 (Tosoh Biosciences Toyopearl Butyl 600M resin) loaded at 0.6 M $Na_2SO_4$ was an optimized combination for the HIC-1 step of cycle 1-2, although other combinations tested proved effective at resolving impurities as well. Thus, butyl resin-2 (having a pore size of 750 A) loaded at 0.6M sodium sulfate proved to be the best candidate.

Table 2: Resin screen

| | Butyl Resin-1 (650A pore) | Butyl resin-2 (750A pore) | Phenyl resin-1 (30 $\mu$M bead) | Phenyl resin-2 (90 $\mu$M bead) |
|---|---|---|---|---|
| Binding Salt | Dynamic Binding Capacity (g/L) | | | |
| 1.6 M NaCl | 10.0[A] | N.D | < 2.0 | 0 |
| 0.6 M $Na_2SO_4$ | > 16.0 | 28.0[A] | > 23 | > 8.0 |
| | Resolution: Monomer / Inactive 1 / Inactive 2 | | | |
| 0.6 M $Na_2SO_4$ | Yes | Yes | Yes | No |
| [A] 10% breakthrough | | | | |

Generally, the process steps of Cycle 1-2 were similar to those described above for Cycle 1-1. with the exception of the order of viral inactivation and the use of a sodium sulfate based wash for the Butyl step. LT-$\beta$-R-Ig was first expressed in recombinant Chinese Hamster Ovary (CHO) cells using a fed-batch process. The titer from the cell expression was about 800 mg/L of LT-$\beta$-R-Ig, including 5% inactive-1 LT-$\beta$-R-Ig, 14% inactive-2 LT-$\beta$-R-Ig, and 22% LT-$\beta$-R-Ig aggregate. Following expression, the cell culture was harvested and centrifuged. The clarified conditioned media was processed over a Protein-A Capture column and virally inactivated using a low pH process. The resulting LT-β-R-Ig mixture was processed over a butyl resin HIC column (referred to as HIC-1) (Butyl-600M; Tosoh Bioscience), where the resin had a capacity of greater than 20 g/L. The HIC-1 step cleared the mixture of inactive forms 1 and 2 of LT-β-R-Ig and was optimized for protein capacity.

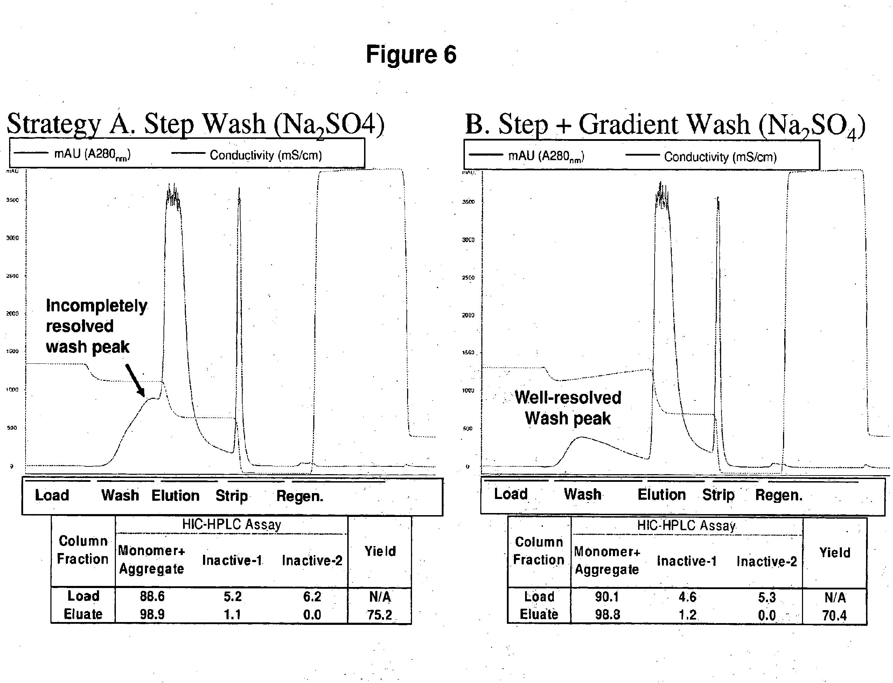

Two wash strategies were used to determine the optimal parameters for washing the butyl resin to increase capacity, *i.e.,* sodium sulfate step wash and a sodium sulfate step / followed by a reverse gradient wash. The results of these two strategies are described in Figure 6, where Strategy B - Step and Gradient Wash proved to provide a better resolved elution. Different amounts of load, sodium sulfate wash concentration, and sodium sulfate elution solution concentrations were studied, as described below in Tables 3 and 4.

Table 3

| Factors | | |
|---|---|---|
| Load (g/litre of resin) | Wash [$Na_2SO_4$] (mM) | Elution [$Na_2SO_4$] (mM) |
| 26 | 460 | 230 |
| 26 | 540 | 230 |
| 22 | 500 | 200 |
| 18 | 460 | 230 |
| 18 | 540 | 170 |
| 26 | 460 | 170 |
| 18 | 540 | 230 |
| 18 | 460 | 170 |
| 26 | 540 | 170 |
| 22 | 500 | 200 |
| Responses (%) | | |
| Active Monomer in Eluate | | |
| Inactive-1 in Eluate | | |
| Inactive-2 in Eluate | | |
| Aggregate in Eluate | | |
| Active Monomer Yield in Eluate | | |
| O.D. in eluate (Yield) | | |
| O.D. in Wash | | |

Table 4

| Statistical Analysis | Parameter and Range | Response | | | |
|---|---|---|---|---|---|
| | | Active Monomer Yield (%) | Inactive-1 in Eluate (%) | Inactive-2 in Elate (%) | O.D in Wash (%) |
| $R^2$ | Multiple regression model with 2-way interactions | 0.88 | 0.92 | 0.81 | 0.98 |
| P > ρ | Load Amount | 0.0190 | 0.0064 | 0.9225 | <0.0001 |
| | Wash [$Na_2SO_4$] | 0.0045 | 0.0064 | 0.4191 | <0.0001 |
| | Elution [$Na_2SO_4$] | 0.2684 | 0.5971 | 0.0061 | 0.1640 |
| | Load Amount X Wash [$Na_2SO_4$] | 0.6849 | 0.0064 | 0.7850 | 0.6306 |

Contour plots were analyzed to determine critical factors for the HIC-1 butyl purification step of cycle 1-2. Critical factors identified for Inactive-1 clearance included column load (in a range of 18-26 g/L) and the $Na_2SO_4$ wash concentration (range of 0.455-0.465 M, using narrow conductivity and osmolality specifications). A key factor for inactive-2 clearance was the $Na_2SO_4$ elution concentration maintained at over 0.22 M. Factors that were determined to not be important in the purification step included the load volume (when $Na_2SO_4$ concentration was > 0.56 M) and elution volume (when $Na_2SO_4$ concentration was > 0.22 M. Other potentially critical factors included wash volume, temperature, resin lot consistency, and load protein concentration.

SUMMARY OF CYCLES 1-1 AND 1-2

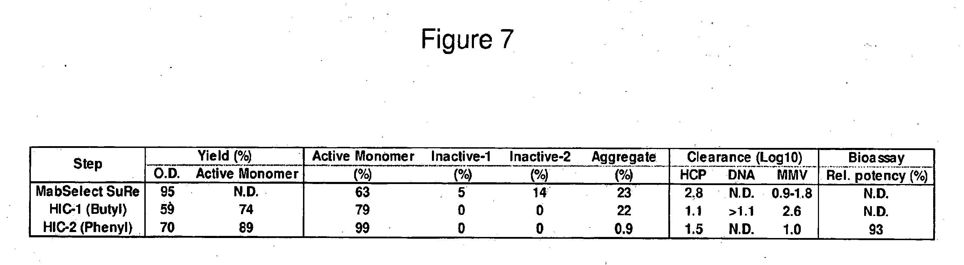

Thus, purification of the LT-β-R-Ig protein was achieved using a combination of HIC columns which, in turn, separated the active LT-β-R-Ig protein (drug product) from the three different product-related impurities, *i.e.,* two different inactive forms of the LT-β-R-Ig protein and aggregates of LT-β-R-Ig (for summary of results see Figure 7).

The HIC-1 column step (butyl resin)was optimized for increased capacity while maintaining resolution of the active LTBR-Ig protein. The butyl HIC column was able to separate the active LT-β-R-Ig protein from the two different inactive forms of the protein, *i.e.,* was able to clear disulphide scrambled monomeric species of LT-β-R-Ig (Inactive-1 and Inactive-2). Product yield was weighed versus clearance of inactive species of LT-β-R-Ig with the HIC-1 column step. Sodium sulphate promoted high capacity with high resolution of species. The critical factors in maximizing clearance of the inactive forms versus product yield included the column load and the wash concentration of $Na_2SO_4$ (for cycle 1-2).

The two processes also included optimization of a third-column step, *i.e.,* HIC-2 (phenyl resin), which yielded a product that was clear of aggregate forms of the LT-β-R-Ig protein, as well as residual inactive-2. Critical factors that were identified for the HIC-2 step included the column load, bed height, and flow rate.

**EXAMPLE 2: PURIFICATION OF BIOLOGICALLY ACTIVE LT-β-R-IG FUSION PROTEINS: CYCLE 2**

The following example describes a process which purifies Ig-fusion biologics, *i.e.,* LT-β-R-Ig fusion protein, from a feedstock containing a high percentage of product-related impurities, *i.e.,* about 50% product-related impurities. As described above, feedstock of LT-β-R-Ig contains different types of impurities, *i.e.,* aggregated species and two distinct, disulfide-scrambled forms of the product - termed Inactive-1 and Inactive-2 forms of LT-β-R. As these impurities are closely related to LT-β-R-Ig (the product), purification of the desired product presents a challenge.

The manufacturing process for LT-β-R-Ig was initially developed for early clinical studies and was later modified to increase productivity to levels sufficient for commercialization. To achieve this, cell culture productivity was increased by a factor of four. This change, however, was associated with an increase in the product variants from 20% to approximately 50% of the total product-related protein. Most notably, aggregate levels were more than doubled, as they increased from about 12% to about 35% of the total. The feed material included a titer of up to 1350 mg/L, which included about 6% inactive-1 LT-β-R-Ig, about 14% inactive-2 LT-β-R-Ig protein, and about 28 to 35% aggregate. The increase in LT-β-R-Ig protein production not only increased the active LT-β-R-1g (drug product) but also increased the three impurities associated with this fusion protein.

Thus, Cycle-2 was designed in response to an increase in LT-β-R-Ig fusion protein titer from mammalian cell expression, particularly with respect to the large increase in protein aggregates which present a challenge for purification. To remove these impurities from the cell culture harvest, an optimized process was developed and was based on a two part separation using a first mixed mode HIC resin and a second phenyl HIC resin. An overview of the increase in impurities is described below in Table 5.

Table 5:

| Process Modifications Associated with the Enhancement of Productivity[A] | | | | | |
|---|---|---|---|---|---|
| **Day of Harvest** | **Percent Increase Relative to Unmodified Process** | | | | |
| | **Bioreactor** | | **Product-Related Impurities** | | **Purified Product** |
| | Volume | Titer B | Aggregate | Inactive Monomer | Mass per Batch |
| 14 | 750% | 290% | 210% | 120% | 1100% |
| 16 | 750% | 400% | 250% | 130% | 1500% |
| [A] Following early clinical studies, increased productivity was required to support commercial manufacturing | | | | | |
| [B] Total product-related; equivalent to 260% increase in active monomeric product at Day-16 | | | | | |

Product related impurities were present in more than 50% of the total protein in the feed for downstream processing (46% product; 35% aggregate; 6% inactive-1; and 13% inactive-2).

Cycle 2 was successful at purifying product when more than 50% of the starting feedstock consisted of undesirable inactive and aggregated variants. The Cycle-2 process was used to successfully purify biologically active, LT-β-R-Ig fusion protein at a 15000 L manufacturing scale. While Inactive-1 co-purified with product (non-aggregated active LT-β-R-Ig) in several modes of chromatography, an efficient separation of these two forms of the LT-β-R-Ig protein was achieved using a mixed mode resin. Inactive-2 was separated from product in a robust manner using hydrophobic interaction chromatography (HIC). Clearance of aggregate to less than 1% in the drug substance was achieved by precise control of the mixed mode and HIC chromatography, as well as a Wash-1 step on the mixed mode resin, that was guided by factorial design experiments to identify and investigate critical parameters.

In the cycle-2 process, the mixed mode chromatography reduced aggregate from ~28% (in the load) to ~10-24% (in the eluate). The subsequent Phenyl step cleared the remaining aggregate, *i.e.,* cleared aggregate from at least 23% (in the Phenyl load) to <1% in the Phenyl eluate

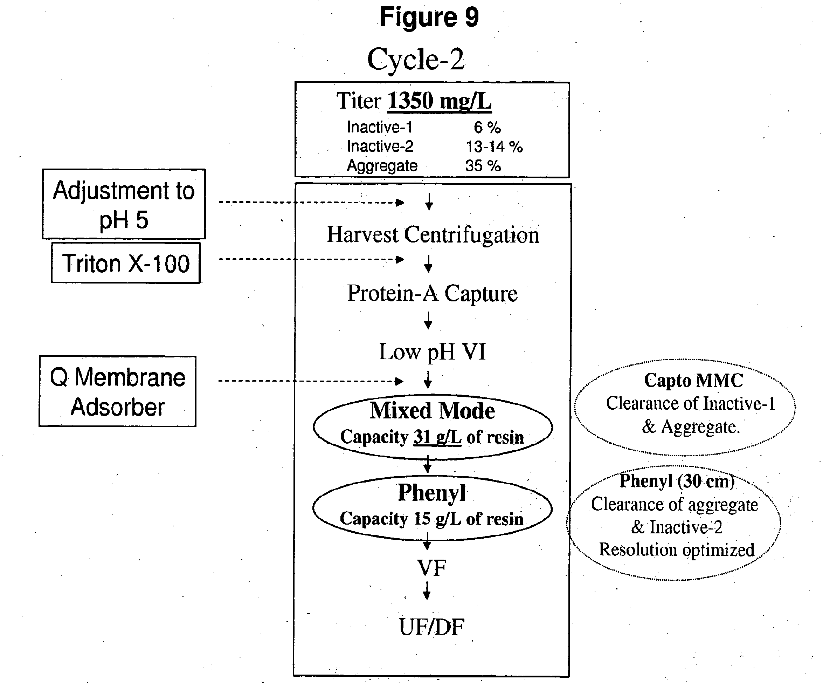

An overview of Cycle-2 is provided in Figure 9, and is summarized as follows. Prior to centrifugation, the bioreactor material (14, 15, or 16 days) was adjusted to a pH of 4.7 to 5.3 to promote removal of process related impurities. Following harvest centrifugation, Triton X-100 was added at 0.05% to the load material for the Protein A capture to promote retroviral inactivation. Subsequent capture of the LT-β-R-Ig fusion protein was performed using Protein A columns at a capacity of 21 g/L. Following Protein A capture, low pH viral inactivation was performed, followed by use of a Q membrane adsorber for further parvovirus clearance. Mixed mode HIC column was then used at a capacity of 31 g/L to clear the mixture of the inactive-1 form of LT-β-R-1g and a portion of the aggregate. Following the mixed mode column step, the eluate was subjected to a HIC column containing phenyl at a capacity of 15 g/L and a bed height of about 30 cm+. This HIC column separated the active LT-β-R-Ig protein from the aggregate and inactive-2 forms. The phenyl column was increased to about 30 cm to optimize clearance of the impurities. The optimal loading range for this height was about 10-15 g/L.

Thus, downstream changes used to process the higher protein titer / high impurity feedstock included modifications to the chromatographic steps. Compared to the previous cycle-1 process steps; cycle-2 was modified such that there was a low pH adjustment of the bioreactor prior to the harvest. This allowed for improved precipitation and removal of process related impurities, and provided enhanced DNA clearance and "clean" feed for the columns. Rapid processing was required with the low pH modification due to limited product stability in the harvest material. A high capacity Protein-A affinity resin was also used, which provided about 140% increase in capacity and decreased the number of cycles required to process the feedstock while increasing productivity. An ion exchange membrane adsorber was also added to the process to provide extra capability for parvovirus clearance. A new second column (mixed mod) was added to the process to improve capacity and clearance of product-related impurities. This new second column increased capacity over 380% and provided robust inactive-1 removal, as well as clearance of aggregates. Additional details regarding the Cycle-2 steps are described below.

HARVEST AND VIRAL INACTIVATION

Prior to purification of monomeric, active LT-β-R-Ig (product), the protein was produced in mammalian CHO cells, harvested, and subsequently clarified. At harvest, the conditioned media was cooled to 2 - 8°C and adjusted to pH 4.7 - 5.3. The pH adjusted conditioned media was fed to the disc stack centrifuge bowl. Cells and cellular debris were collected in the bowl sediment hold space and removed intermittently during the harvest operation. The clarified cell-free centrate rose to the bowl top and was continuously discharged under pressure by a centrifugal pump. Upon completion of the centrifugation process, the collected centrate was mixed and neutralized to pH 6.9 - 7.5.

Following harvest and centrifugation of the mixture containing both active LT-β-R-Ig and associated impurities, triton X-100 was added to inactivate adventitious viruses without damaging the product or generating unacceptably high levels of aggregate. The temperature of the neutralized centrate was adjusted to 15 - 25°C and Triton X-100 was added to a final concentration of 0.05% (w/v). The Triton-X adjusted centrate was held for a minimum of 2 hours before being processed further.

The mixture (containing inactive and active forms of LT-β-R-Ig and aggregates) was run over a recombinant protein A column. Recombinant Protein A (rProtein A) MabSelect SuRe resin was chosen for the capture step due to the high binding affinity and specificity that this resin exhibits for LT-β-R-Tg. Purification of LT-β-R-Ig from process related impurities took place at this chromatography step in the process and LT-β-R-Ig. In addition, the MabSelect SuRe step provided clearance of potential viral contaminants, host cell DNA, host cell proteins, and reduced the process volume by eight-fold. This chromatography step was operated at 18 - 26°C.

Introduction of a novel chromatography resin for the 2nd-column step (mixed mode) improved capacity and clearance of product-related impurities, but decreased capability for clearance of potential parvovirus. To restore excess clearance of adventitious parvovirus with optimal facility-fit, a Q Membrane Adsorber unit operation was implemented in the process. Development identified the optimal pH (about pH 6.4) and conductivity (high conductivity) for clearance of the model parvovirus - Mouse Minute Virus (MMV) - while providing ~100% product yield. The Q membrane was implemented between the Protein A affinity and 2nd-column steps, and provided > 3 Log10 of MMV in scale-down viral-spiking studies using GMP materials.

Each cycle of MabSelect SuRe eluate was processed through low pH viral inactivation and Q membrane filtration prior to pooling. This step was designed to inactivate pH sensitive viruses without damaging the product or generating unacceptably high levels of aggregate. The MabSelect eluate was adjusted to be pH 3.5 - 3.9, then held for 2 - 2.5 hours at 18 - 26°C. The solution was then adjusted to pH 6.2 - 6.6 and processing continued into the next step, involving an anion exchanger (Sartobind Q-Membrane Adsorber (QMA)) which is an adsorber for parvovirus clearance.

MIXED MODE RESIN

A mixed mode resin, *i.e.,* Capto MMC chromatography (GE/Healthcare), was the second chromatography step in the LT-β-R-Ig purification process of Cycle-2 (first step being Protein A). Although Capto MMC is a mixed mode resin, it was operated in the cation-exchange mode. The mixed mode resin was selected due to its high binding capacity for LT-β-R-Ig and its ability to separate active, monomeric LT-β-R-Ig from process- and product-related impurities. In addition, the Capto MMC step provided clearance of host cell proteins, MabSelect SuRe column rProtein A leachate, and potential viral contaminants. Capto MMC chromatography also reduced product related impurities such as aggregates and inactive species of LTBR-Ig. This step was carried out at 16 - 24°C. Conditions for the Capto MMC step and removal of both inactive-1 and the aggregate impurities included: load ratio of 26-31 g/L with a load pH of 4.8-5.2. In addition, the first wash had a pH of 7.0 to 7.2. The column load and wash steps impacted the yield and aggregate clearance.

The primary role of the second column step (first column being the Protein A) in the cycle-1 LT-β-R-Ig purification process was to clear the disulfide-scrambled product related impurity, *i.e..,* the Inactive-1 species, from the active, monomeric LT-β-R-Ig product. In cycle-1, this separation was performed using the Tosoh Bioscience Butyl 650M resin. However, the Butyl 650M resin was limited in that it had relatively low binding capacity and was not suitable for increased titer. Thus the challenge for the LT-β-R-Ig cycle-2 process development was to identify conditions to increase the capacity of the second column step (to accommodate the increased titer while minimizing column cycling) while maintaining comparable Inactive-1 clearance to that achieved in cycle-1. A high capacity IEX resin was used and was capable of robust Inactive-1 clearance using an intermediate wash step.

The Cation exchange/HIC Mixed Mode resin that was selected was the Capto MMC (GE/Healthcare). 0.66 cm (I.D.) x 15-20 cm bed height glass columns (Omnifit) were used, and experiments were performed at room temperature (22-26°C).

The Capto MMC resin is composed of a cross-linked agarose bead that has been derivatized with a mixed-mode ligand (GE Healthcare). The ligand contains several multiple functionalities for interaction with the target protein, which include ionic and hydrophobic-type interactions. Thus, the resin can be operated in both a hydrophobic and cationic mode of chromatography. For LT-β-R'-Ig purification process, the Capto MMC column was operated in the cationic mode, in which the LT-β-R-Ig was eluted from the resin by increasing the buffer pH and/or buffer conductivity.

The assays performed during the Capto MMC development were analytical HIC and analytical SEC.

In addition to using $OD_{280}$ to determine yield, LTBR-Ig recovery was also quantified using the "active monomer yield", as defined in Eq. (1):

$$ActiveMonomerYield = \frac{[V_{elution}][OD_{280,elution}][\%monomer_{elution}][\%Active_{elution}]}{[V_{Load}][OD_{280,Load}][\%monomer_{Load}][\%Active_{Load}]} \quad \text{Eq. (1)}$$

where: V is the solution volume (mL), and $OD_{280}$ is the absorbance at 280 nm/mL. The % monomer and % "Active" species were measured using HPLC-SEC and analytical-HIC, respectively. LT-β-R-Ig aggregates included both dimer (defined as a multimer containing four chains of disulfide bonded molecules) and highmolecular weight species.

Dynamic binding capacity (DBC) at 5% breakthrough for Capto MMC was calculated using Eq. (2) :

$$DBC = \frac{C_o(V_{BT} - V_{DV})}{V_B} \quad \text{Eq. (2)}$$

where $C_0$ equals the concentration of protein in the load (mg/mL), $V_{BT}$ equals the volume loaded at the point of 5% breakthrough (mL), $V_B$ equals the total volume of the resin bed and $V_{DV}$ equals the system and column dead volume.

The Capto MMC resin (mixed mode) was able to effectively separate the inactive-1 form, and partially separate the aggregated forms from active, monomeric LT-β-R-Ig (desired product). Capto MMC was able to separate both the active form of LT-β-R-Ig, as well as a portion of the aggregate species.

Three experiments were performed to define operating conditions for the Capto MMC resin, as well as examine the sensitivity of the resin to changes in the operating conditions using feed from early cell culture development work. Results of these studies showed the performance of Capto MMC was sensitive to the column loading, wash buffer pH, and wash buffer concentration. The column loading range was determined to be 20-31 mg LTBR-Ig/mL resin, the Wash-1 buffer pH set to pH 7.0-7.2 and Wash-1 buffer concentration set to about 80 mM Bis Tris. The second Wash (Wash-2) had a pH of 5.5-5.7 (e.g., pH 5.6) and a concentration of about 20- 30 mM sodium acetate. LT-β-R-Ig was eluted from the mixed mode resin using 50 mM phosphate and 150 mM NaCl (pH 7.0).

Factorial analysis of chromatography conditions identified that the column load ratio and wash solution conditions were important for the separation.

During development, increases in bioreactor titer were accompanied by an increase in aggregate level necessitating partial clearance of aggregate by the mixed mode 2nd-column step. Aggregate clearance was obtained by increasing the stringency of the wash conditions. However, a complete separation of aggregate from product was not achieved - aggregate clearance was obtained at the expense of partial product loss during the wash. Because product yield and aggregate clearance were dependent on column load ratio and wash conditions, optimal operating ranges were required for both parameters.

Mixed mode column operating conditions and performance separating inactive-1 and/or aggregates is described in Figure 11. Inactive-1 clearance was robust with high product yield over a broad range of load and wash conditions. In contrast, aggregate clearance required a more stringent wash condition that negatively impacted product yield and caused a dependence of yield and aggregate clearance on the column load and wash conditions, even within optimal ranges. During manufacturing the optimal range specifications required for the wash solution revealed a lot-to-lot variability in the buffering component, requiring revision of the solution lot record.

A summary of the decrease in the inactive-1 form of LT-β-R-Ig and the aggregate using the mixed mode resin is described below in Table 6. Table 6 provides an analysis of various runs of LT-β-R-Ig production and purification under similar conditions. Use of the mixed mode resin decreased the inactive-1 form of LTBR-Ig-from about 6% in the original mixture obtained from the cell culturing process to less than 1%, or undetectable amounts. In addition, as described in Table 6, the percentage of aggregates was also decreased from about 30% (28-35%) to about 10-24%.

Table 6: Summary of example runs using mixed mode resin (Capto MMC)

| | Run 1 | Run 2 | Run 3 | Run 4 | Run 5 | Run 6 | Run 7 |
|---|---|---|---|---|---|---|---|
| Initial titer | > 800 | 820 | 1240 | 1200 | 980 | 1050 | 1396 |
| Aggregate (%) | 28 | 29 | 33 | 35 | 33 | 28 | 26 |
| Inactive-1 (%) | 7 | 6 | 6 | 6 | 6 | 6 | 6 |
| Inactive-2 (%) | 10 | 9 | 9 | 14 | 10 | 7 | 6 |
| Capto MMC step | | | | | | | |
| Active monomer (%) | 75 | 77 | 67 | 67 | 75 | -74 | 74 |
| Aggregate in eluate (%) | 25[A] | 14 | 23[A] | 19 | 13 | 21 | 15 |
| Inactive-1 (%) | < LLOQ[B] | < LLOQ | < LLOQ | < LLOQ | < LLOQ | < LLOQ | < LLOQ |
| Inactive-2 (%) | 10 | 8 | 10 | 10 | 11 | 8 | 6 |

[A]Aggregate high - Capto MMC Wash-1 pH was subsequently increased to provide higher aggregate clearance
[B]< LLOQ = less than lowest level of quantification for the HIC-HPLC assay

DESORBTION OF PRODUCT RELATED IMPURITIES DURING THE CAPTO MMC WASH-1 AND ELUTION STEPS

To investigate the chromatographic behavior of product-related impurities during the Capto MMC step, fractions were collected throughout the Capto MMC Wash-1 and Elution. Each fraction was analyzed by absorbance, SEC- and HIC-HPLC and the total amount of each individual product-related species determined. This analysis revealed that, under the conditions employed, the Inactive-1 species was removed efficiently during the Wash-1 step and was undetectable in the elution fraction. In contrast, high molecular weight variants and dimer (collectively termed aggregate) were recovered in the Wash-1 and Elution pools. Notably, desorbtion of aggregate declined before the end of the Wash-1 step at pH 7.0 and prior to the commencement of Wash-2 (pH 5.6). Thus, a limited bolus of aggregate emerges from the column during Wash-1 (in 80 mM Bis-Tris, pH 7.0) while further aggregate remains bound until the column is eluted with 50 mM sodium phosphate, 150 mM NaCl, pH 7.0. Active monomer and Inactive-2 desorbed from the column during the Wash-1 and Elution steps. Analysis of the elution pool reveal that it was enriched for active monomer. This was mainly to preferential loss of Inactive-1 and aggregate during the Wash-1 step because the percent Inactive-2 in the load material and eluate pool was similar.

PHENYL SEPHAROSE RESIN

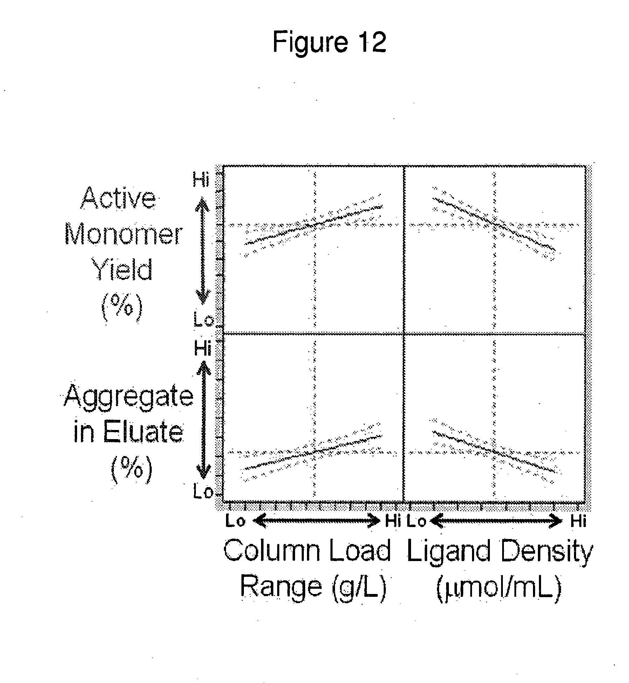

Phenyl Sepharose 6 Fast Flow High Sub (Phenyl Sepharose) was the final (3rd) chromatography step in the LT-β-R-Ig purification process. Phenyl Sepharose is a hydrophobic interaction chromatography resin chosen as the final polishing column due to its high binding capacity for LT-β-R-Ig and ability to separate the product from process and product related impurities. The step was found to remove aggregated and inactive-2 forms of LT-β-R-Ig. To clear the high level of aggregate present in the feedstock, the resolving power of the HIC 3rd column was increased by extending the bed height from 20 cm to 30 cm. Additionally, the phenyl step provided further clearance of host cell DNA, host cell proteins, MabSelect SuRe column rProtein A leachate, and potential viral contaminants. Factorial analysis of chromatography conditions, combined with risk assessment, identified that column load and resin lot (ligand density) were important factors for the separation, as described in Figure 12. After load adjustment with ammonium sulfate, the Capto MMC eluate pool was loaded onto the Phenyl Sepharose column (Phenyl Sepharose 6 Fast Flow (hi sub); 0.66 cm (I.D.) x 20-40 cm bed height glass columns). The load range and bed height were optimized for clearance of aggregates and inactive-2, where the aggregate percentage was < 1% and inactive-2 was < LLOQ.

The assays used during the HIC screening and development of the Phenyl Sepharose F.F. chromatography steps were analytical HIC and analytical SEC.

In addition to using $OD_{280}$ to determine yield, LT-β-R recovery was also quantified using the "active monomer yield", as defined in Eq. (I):

$$ActiveMonomerYield = \frac{[V_{elution}][OD_{280,elution}][\%monomer_{elution}][\%Active_{elution}]}{[V_{Load}][OD_{280,Load}][\%monomer_{Load}][\%Active_{Load}]} \quad (1)$$

where: V is the solution volume (mL), and $OD_{280}$ is the absorbance at 280 nm/mL.

The % monomer and % "Active" species were measured using HPLC-SEC and analytical-HIC, respectively. LT-β-R-Ig aggregate species included both dimer and high molecular weight species.

In order to examine the effect of bed height on aggregate clearance, Phenyl Sepharose was packed at three bed heights (20, 30 and 40 cm). LT-β-R-Ig loaded onto Phenyl sepharose column to a target of approximately 15 mg LT-β-R-Ig/mL resin and then washed with 3 CV of equilibrating solution. The column was washed and LT-β-R-Ig was eluted using an ammonium sulfate buffer. Fractions were collected during the elution and analyzed for yield and monomer content. The column loading and product recovery were determined by measurement of the absorbance ($OD_{280}$).

Table 7 shows the effect of bed height and operating velocity on LT-β-R aggregate removal and recovery. (Feed: 15% Aggregate, 9% Inactive-2; Column Loading: 15 mg LT-β-R/mL resin, ammonium sulfate Elution Buffer concentration: 0.325 M, Elution volume: 6 CV). Parameters which provided optimal resolution of LT-β-R-Ig and aggregate proteins included a bed height of 30 cm (versus 20 cm in the Cycle-1 phenyl bed height) and a flow rate of 50 cm/h. In addition, the optimal load range with these parameters was 10 to 20 g/L of resin. Alternatively, a flow rate of 100 cm/hr and an elution solution of 0.44 M ammonium sulfate (range of 0.3 to 0.5) were also determined to be effective.

Table 7

| Bed Height (cm) | Operating Velocity (cm/hr) | Residence Time (min) | $OD_{280}$ LTBR Recovery (%) | Active Monomer Yield (%) | Aggregate Monomer % (%) | Inactive-2 (%) |
|---|---|---|---|---|---|---|
| 30 | 100 | 18 | 57 | 75 | 0.88 | 0 |
| 20 | 50 | 24 | 50 | 69 | 0.82 | 0 |
| 40 | 50 | 48 | 47 | 64 | 0.63 | 0 |
| 20 | 33 | 36 | 47 | 65 | 0.56 | 0 |
| 30 | 50 | 36 | 59 | 83 | 0.58 | 0 |

As can be seen in Table 7, increasing the bed height of the phenyl column improved overall yield of the active LT-β-R-Ig (referred to above as "active monomer") and decreased aggregate percentages.

Because yield and aggregate clearance were strongly dependent on column load and resin ligand density, optimal operating ranges were required for those parameters (described in more detail below). The difficulty of the separation (removal of >20% aggregate) revealed a lot-to-lot variability in resin performance that was correlated with the ligand density specification

Generally, the phenyl step was carried out at room temperature and with a packed bed height of 30 cm. Following loading, the column was washed with 2.6 mM acetic acid, 1.0 M ammonium sulfate, 50 mM acetate, pH 5.8 (Wash-1).

LT-β-R-Ig was subsequently eluted following the washes using 2.6 mM acetic acid, 0.44 M ammonium sulfate, 50 mM acetate, pH 5.8.

A summary of the decrease in the aggregate impurity form of LT-β-R-Ig using the phenyl resin is described in Table 8, which provides an analysis of various runs of LT-β-R-Ig production and purification under similar conditions.

Table 8: Summary of example runs using phenyl resin (Phenyl Sepharose)

| | Run 1 | Run 2 | Run 3 | Run 4 | Run 5 | Run 6 | Run 7 |
|---|---|---|---|---|---|---|---|
| Initial titer | > 800 | 820 | 1240 | 1200 | 980 | 1050 | 1396 |
| Aggregate (%) | 28 | 29 | 33 | 35 | 33 | 28 | 26 |
| Inactive-1 (%) | 7 | 6 | 6 | 6 | 6 | 6 | 6 |
| Inactive-2 (%) | 10 | 9 | 9 | 14 | 10 | 7 | 6 |
| Phenyl Sepharose step | | | | | | | |
| Active monomer(%) | 97 | 99 | 98 | 98 | 99 | 97 | 99 |
| Aggregate in eluate (%) | 3* | 2* | 0.4 | 0.6 | 0.4 | 0.4 | 0.3 |
| Inactive-1 (%) | < LLOQ | < LLOQ | < LLOQ | < LLOQ | < LLOQ | < LLOQ | < LLOQ |
| Inactive-2 (%) | 2.3 | < LLOQ | < LLOQ | < LLOQ | < LLOQ | < LLOQ | < LLOQ |
| * Aggregate high. For Subsequent PT's the resin lot and elution solution ammonium sulfate conc. were harmonized (resin lot variability issue) | | | | | | | |

In sum, use of the phenyl HIC step decreased the percentage of aggregates to less than 1%. The phenyl HIC step also decreased the inactive-2 form of LTBR-Ig to less than 1%.

USE OF CYCLE-2 FOR 15,000 L SCALE

Cycle-2 was used successfully at large scale, *i.e.,* 15,000 L, to purify LTBR-Ig from unwanted impurities, including aggregates and two inactive forms of the protein. Upon initial transfer to 15,000 L scale, the process was operated conservatively to minimize risk to product quality. Harvest occurred at day-14 or 15, and contained 30% aggregate in the bioreactor. Cycle-2 included a high load on the 2nd column to promote Inactive-1 and aggregate clearance and a low load on the 3rd column to promote aggregate and Inactive-2 clearance.

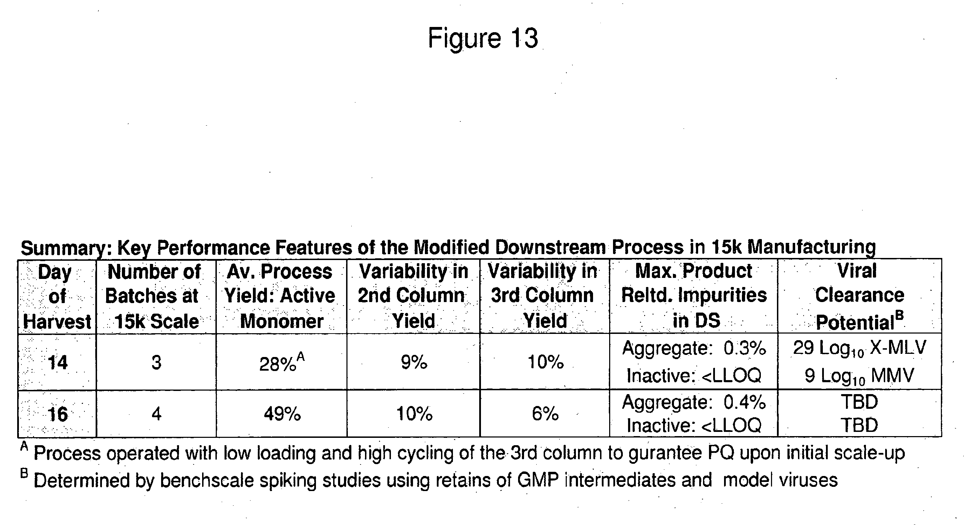

Based on process performance in batches 1-3, the process was subsequently operated under higher yielding conditions (Batches 4-7), where harvest occurred at day 16 and contained 35% aggregate in the bioreactor. High loading on the 3rd column was performed to promote high yield. Figure 13 provides a summary of key performance features of the modified downstream process in 15k manufacturing.

Summary

To summarize the overall process, clarification of the cell cultured harvest was with disc stack centrifugation. Triton X-100 was added to the clarified conditioned media for viral inactivation. The clarified conditioned media was processed over a Recombinant Protein A (rProtein A), MabSelect SuRe chromatography column to capture the product. Low pH viral inactivation was performed next. The viral inactivated rProtein A MabSelect eluate was then filtered through a Sartobind Q membrane adsorber (QMA) for adventitious parvovirus clearance. The product was further purified by Capto MMC mixed mode chromatography followed by hydrophobic interaction chromatography using Phenyl Sepharose 6 Fast Flow Hi Sub (Phenyl Sepharose). The Phenyl Sepharose eluate was then filtered through Planova 15N virus removal filters, then concentrated and diafiltered by ultrafiltration (UF). The UF retentate is subsequently formulated into the final formulation buffer at a product concentration of 100 g/L and stored at -70°C.

The Cycle 2 process (particularly, mixed mode followed by phenyl HIC) was proven to be effective for scaling up production and purification of LT-β-R-Ig for manufacturing purposes, *e.g.,* 15000 L scale. A summary of the percentages for decreasing inactive-1, inactive-2, and aggregate percentages using the Cycle-2 process is provided below in Table 9.

Table 9.

| % | Mixture following cell culture and harvest | Following Protein A column | Following mixed mode HIC column | Following phenyl HIC column |
|---|---|---|---|---|
| Aggregate | 30-35% | ~ 28% | 8-24% | <1 % |
| Inactive-1 | ~ 6% | ~ 6% | <1% | <1 % |
| Inactive-2 | ~ 13% | ~ 13% | ~ 13% | <1 % |

## EQUIVALENTS

The present invention provides among other things improved methods relating to purified, active forms of Ig fusion proteins, including LT-β-R-Fc fusions. While specific embodiments of the subject invention have been discussed, the above specification is illustrative and not restrictive. Many variations of the invention will become apparent to those skilled in the art upon review of this specification. The full scope of the invention should be determined by reference to the claims, along with their full scope of equivalents, and the specification, along with such variations.

## Claims

1. A method for separating biologically active lymphotoxin-β receptor immunoglobulin (LT-β-R-Ig) fusion proteins from biologically inactive LT-β-R-Ig fusion proteins inclusive of the first inactive form of the LT-β-R-Ig fusion protein (inactive-1) and the second inactive form of the LT-β-R-Ig fusion protein (inactive-2), comprising

   loading a mixture comprising biologically active LT-β-R-Ig fusion proteins and biologically inactive LT-β-R-Ig fusion proteins onto a butyl hydrophobic interaction chromatography (HIC) resin and

   contacting the resin with a solution comprising a salt gradient to obtain a first eluate comprising biologically inactive-1 LT-β-R-Ig fusion proteins and a second eluate comprising biologically active LT-β-R-Ig fusion proteins, and

   wherein the inactive-1 LT-β-R-Ig fusion protein elutes from the butyl hydrophobic interaction chromatography (HIC) resin at a higher salt concentration than the biologically active LT-β-R-Ig fusion protein, and

   wherein the inactive-2 LT-β-R-Ig fusion protein remains bound to the butyl resin until stripped off with water.

2. The method of claim 1, wherein:

   (a) the salt gradient is a step or continuous gradient;
   (b) the salt gradient comprises 1.6 M to 0.0 M NaCl;
   (c) the second eluate comprising biologically active LT-β-R-Ig fusion proteins is obtained by washing the HIC resin with a 1.5 M NaCl wash;
   (d) the salt gradient comprises 0.6 M to 0.0 M $Na_2SO_4$; or
   (e) the salt gradient is a step gradient comprising increments of $Na_2SO_4$ ranging from 0.6 M to 0.0 M.

3. The method of claim 1 or claim 2, wherein:

   (i) the butyl HIC resin has a pore size of 600 to 750 A;
   (ii) the butyl HIC resin has a load capacity of about 8 mg to about 20 mg of protein/L of resin;
   (iii) the butyl HIC resin comprises a hydroxylated methacrylate backbone;
   (iv) the second eluate comprising biologically active LT-β-R-Ig fusion proteins comprises less than 2% biologically inactive LT-β-R-Ig fusion proteins; or
   (iv) the second eluate comprising biologically active LT-β-R-Ig fusion proteins comprises less than 1% biologically inactive LT-β-R-Ig fusion proteins.

4. The method of any one of claims 1-3, wherein the method further comprises:

   loading the second eluate comprising biologically active LT-β-R-Ig fusion proteins onto a second HIC resin under conditions that allow the biologically active LT-β-R-Ig fusion proteins to bind the second HIC resin; and contacting the second HIC resin with a solution to obtain a third eluate further enriched for the presence of biologically active LT-β-R-Ig fusion proteins.

**5.** The method of any one of claims 1-3, wherein the method further comprises
loading the second eluate onto a phenyl hydrophobic interaction chromatography (HIC) resin, wherein the resin has a load capacity of between about 10-20 g of protein/L of resin and a bed height of about 30 to 31 cm, and contacting the resin with a solution at a flow rate of about 50 to 150 cm/hr to obtain a third eluate comprising biologically unaggregated, biologically active LT-β-R-Ig fusion proteins and a fourth eluate comprising aggregated LT-β-R-Ig fusion proteins.

**6.** The method of claim 5, wherein:

(a) the third eluate comprising unaggregated, biologically active LT-β-R-Ig fusion proteins comprises less than 2% aggregated LT-β-R-Ig fusion proteins; or
(b) the third eluate comprising unaggregated, biologically active LT-β-R-Ig fusion proteins comprises less than 1% aggregated LT-β-R-Ig fusion proteins.

**7.** A method for separating biologically active lymphotoxin receptor immunoglobulin (LT-β-R-Ig) fusion proteins from biologically inactive LT-β-R-Ig fusion proteins inclusive of the first inactive form of the LT-β-R-Ig fusion protein (inactive-1) and the second inactive form of the LT-β-R-Ig fusion protein (inactive-2), said method comprising the steps of:

i) loading a mixture comprising biologically active LT-β-R-Ig fusion proteins and biologically inactive LT-β-R-Ig fusion proteins onto a butyl hydrophobic interaction chromatography (HIC) resin under conditions that allow the biologically active LT-β-R-Ig fusion proteins to bind the butyl resin;
ii) contacting the butyl HIC resin with a solution comprising a salt gradient to obtain a first eluate enriched for the presence of biologically active LT-β-R-Ig fusion proteins;
iii) loading the first eluate of step ii) onto a second HIC resin under conditions that allow the biologically active LT-β-R-Ig fusion proteins to bind the second HIC resin; and
iv) contacting the second HIC resin with a solution to obtain a second eluate further enriched for the presence of biologically active LT-β-R-Ig fusion proteins,
to thereby separate the biologically active LT-β-R-Ig fusion proteins from the biologically inactive LT-β-R-Ig fusion proteins, and
wherein the inactive-1 LT-β-R-Ig fusion protein elutes from the butyl hydrophobic interaction chromatography (HIC) resin at a higher salt concentration than the biologically active LT-β-R-Ig fusion protein, and
wherein the inactive-2 LT-β-R-Ig fusion protein remains bound to the butyl resin until stripped off with water.

**8.** The method of claim 7, wherein:

(a) the salt gradient of (ii) is either a step or a continuous gradient; or
(b) the butyl HIC resin comprises a hydroxylated methacrylate backbone.

**9.** The method of claim 7 or claim 8, wherein:

(i) the butyl HIC resin has a pore size of 600 to 750 A;
(ii) the first eluate comprises less than 2% of a first form of the inactive LT-β-RIg fusion protein (Inactive-1);
(iii) the first eluate comprises less than 1% of a first form of the inactive LT-β-RIg fusion protein (Inactive-1);
(iv) the second eluate comprises less than 2% of a second form of the inactive LT-β-R-Ig fusion protein (Inactive-2); or
(v) the second eluate comprises less than 1% of a second form of the inactive LT-β-R-Ig fusion protein (Inactive-2).

**10.** The method of claim 2, wherein:

(a) the salt gradient comprises 1.6 M to 0.0 M NaCl and the concentration of LT-β-R-Ig fusion proteins in the mixture is at least 300 mg/L; or
(b) the salt gradient comprises 0.6 M to 0.0 M $Na_2SO_4$ and the concentration of LT-β-R-Ig fusion proteins in the mixture is at least 800 mg/L.

**11.** A method for separating biologically active LT-β-R-Ig fusion proteins from biologically inactive LT-β-R-Ig fusion proteins, said method comprising the steps of:

i) loading a mixture comprising biologically active LT-β-R-Ig fusion proteins and biologically inactive LT-β-R-Ig fusion proteins onto a mixed mode resin under conditions that allow the biologically active LT-β-R-Ig fusion proteins to bind the mixed mode resin;

ii) eluting the biologically active LT-β-R-Ig fusion proteins from the mixed mode resin of step i) with a solution to obtain a first eluate enriched for the presence of biologically active LT-β-R-Ig fusion proteins;

iii) loading the first eluate of step ii) onto a hydrophobic interaction chromatography (HIC) resin under conditions that allow the biologically active LT-β-R-Ig fusion proteins to bind the HIC resin; and

iv) eluting the biologically active LT-(3-R-Ig fusion proteins of step iii) with a solution to obtain a second eluate further enriched for the presence of biologically active LT-β-R-Ig fusion proteins,

to thereby separate biologically active LT-β-R-Ig fusion proteins from biologically inactive LT-β-R-Ig fusion proteins.

12. A method for separating unaggregated, biologically active LT-β-R-Ig fusion proteins from aggregated LT-β-R-Ig fusion proteins the method comprising the steps of:

i) loading a mixture comprising unaggregated, biologically active LT-β-R-Ig fusion proteins and aggregated LT-β-R-Ig fusion proteins, wherein more than about 30% or about 30% of LT-β-R-Ig fusion proteins in the mixture are aggregated, onto a mixed mode resin under conditions that allow the biologically active LT-β-R-Ig fusion proteins to bind the mixed mode resin;

ii) eluting the biologically active LT-β-R-Ig fusion proteins from the mixed mode resin of step i) using a solution to obtain a first eluate enriched for the presence of biologically active LT-β-R-Ig fusion proteins;

iii) loading the first eluate of step ii) onto a hydrophobic interaction chromatography (HIC) resin under conditions that allow the biologically active Ig fusion proteins to bind the HIC resin; and

iv) eluting the biologically active LT-β-R-Ig fusion proteins of step iii) using a solution to obtain a second eluate further enriched for the presence of biologically active LT-β-R-Ig fusion proteins,

thereby separating the unaggregated biologically active LT-β-R-Ig fusion proteins from the aggregated LT-β-R-Ig fusion proteins.

13. The method of claim 11 or 12, wherein the mixed mode resin is characterized as having both ionic interaction capability and hydrophobic interaction capability.

14. The method of claim 11 or 12, wherein the mixture is loaded onto the mixed mode resin at a pH of about 5.0.

15. The method of claim 14, wherein the loaded mixed mode resin is washed with a buffer having a pH of about 7.0 to 7.2 prior to step (ii).

16. The method of claim 13, wherein the buffer is Bis Tris.

17. The method of any one of claims 11-16, wherein the HIC resin is a phenyl resin.

18. The method of claim 17, wherein:

(a) the phenyl resin is phenyl sepharose;
(b) the biologically active LT-β-R-Ig is eluted from the phenyl resin with ammonium sulfate; and preferably with 0.3 to 0.5 M ammonium sulfate; or
(c) the flow rate of the phenyl resin is 50 to 150 cm/hr, and preferably about 100 cm/hr.

19. A method for removing at least 97% aggregated LT-β-R-Ig fusion proteins from a mixture comprising unaggregated, biologically active LT-β-R-Ig fusion proteins and aggregated LT-β-R-Ig fusion proteins, said method comprising the steps of:

i) loading the mixture onto a mixed mode resin under conditions that allow the unaggregated, biologically active LT-β-R-Ig fusion proteins to bind the mixed mode resin;

ii) eluting the unaggregated, biologically active LT-β-R-Ig fusion proteins from the mixed mode resin of step i) with a solution to obtain a first eluate enriched for the presence of unaggregated, biologically active LT-β-R-Ig fusion proteins;

iii) loading the first eluate of step ii) onto a hydrophobic interaction chromatography (HIC) resin under conditions that allow the unaggregated, biologically active LT-β-R-Ig fusion proteins to bind the HIC resin; and
iv) eluting the unaggregated, biologically active LT-β-R-Ig fusion proteins of step
iii) with a solution to obtain a second eluate further enriched for the presence of unaggregated, biologically active LT-β-R-Ig fusion proteins,

to thereby remove at least 97% aggregated LT-β-R-Ig fusion proteins from the mixture comprising unaggregated, biologically active LT-β-R-Ig fusion proteins and aggregated LT-β-R-Ig fusion proteins.

20. The method of claim 19, wherein the first eluate comprises less than 25%, less than 20%, less than 15%, or about 10% aggregated LT-β-R-Ig fusion proteins.

21. The method of claim 19, wherein the second eluate comprises less than 2% aggregated LT-β-R-Ig fusion proteins or less than 1% aggregated LT-β-R-Ig fusion proteins.

22. The method of any one of claims 19-21, further comprising contacting the mixture with recombinant Protein A (rProtein A) prior to step (i).

23. The method of claim 22, wherein the mixture contacted with recombinant Protein A is a mammalian cell culture supernatant.

24. The method of claim 23, wherein:

(a) the mammalian cell culture supernatant is obtained from mammalian cells cultured at 28 °C; or
(b) the cell culture supernatant comprises a nonionic surfactant, and preferably the nonionic surfactant is Triton X-100.

25. A method for preparing a composition comprising biologically active LT-β-R-Ig fusion proteins, wherein the composition comprises less than 1% of a first inactive form of the LT-β-R-Ig fusion protein (Inactive-1), less than 1% of a second inactive form of the LT-β-R-Ig fusion protein (Inactive-2), and less than 1% aggregated LT-β-R-Ig fusion proteins, said method comprising the steps of

i) obtaining a cell culture supernatant from a mammalian cell culture system comprising a mammalian host cell transformed with DNA encoding the LT-β-R-Ig-fusion protein;
ii) contacting the cell culture supernatant with recombinant Protein A (protein A) to obtain an LT-β-R-Ig fusion protein mixture;
iii) loading the LT-β-R-Ig fusion protein mixture of ii) onto a mixed mode resin at a pH of about 5.0 under conditions such that the LT-β-R-Ig fusion proteins bind the mixed mode resin;
iv) washing the mixed mode resin with a buffer having a pH of about 7.0 to 7.2;
v) contacting the mixed mode resin of step iii) with a solution to obtain a first eluate enriched for the presence of biologically active LT-β-R-Ig fusion protein;
vi) loading the first eluate of step iv) onto a hydrophobic interaction chromatography (HIC) resin under conditions that allow the LT-β-R-Ig fusion protein to bind the HIC resin; and
vii) contacting the HIC resin of step vi) with a solution to obtain a second eluate further enriched for the presence of biologically active LT-β-R-Ig fusion protein,

wherein the second eluate comprises less than 1% of the first inactive form of the LT-β-R-Ig fusion protein (Inactive-1), less than 1% of the second inactive form of the LT-β-R-Ig fusion protein (Inactive 2), and less than 1% aggregated LT-β-R-Ig fusion proteins.

26. The method of any one of claims 19-25, further comprising contacting the mixture with an anion exchange membrane adsorber prior to step (i).

**Patentansprüche**

1. Verfahren zum Trennen von biologisch aktiven Lymphotoxin-β-Rezeptor-Immunglobulin-(LT-β-R-Ig)-Fusionsproteinen von biologisch inaktiven LT-β-R-Ig-Fusionsproteinen einschließlich der ersten inaktiven Form des LT-β-R-Ig-Fusionsproteins (inactive-1) und der zweiten inaktiven Form des LT-β-R-Ig-Fusionsproteins (inactive-2), das Folgendes beinhaltet:

Laden eines Gemischs aus biologisch aktiven LT-β-R-Ig-Fusionsproteinen und biologisch inaktiven LT-β-R-Ig-Fusionsproteinen auf ein Butyl-hydrophobe Interaktionschromatographie(HIC)-Harz, und

Inkontaktbringen des Harzes mit einer Lösung, die einen Salzgradienten umfasst, um ein erstes Eluat, das biologisch inaktive (inactive-1) LT-β-R-Ig-Fusionsproteine umfasst, und ein zweites Eluat zu gewinnen, das biologisch aktive LT-β-R-Ig-Fusionsproteine umfasst, und

wobei das inactive-1 LT-β-R-Ig-Fusionsprotein von dem Butyl-HIC-(hydrophobe Interaktionschromatographie)-Harz mit einer höheren Salzkonzentration eluiert als das biologisch aktive LT-β-R-Ig-Funktionsprotein, und

wobei das inactive-2 LT-β-R-Ig-Fusionsprotein an das Butylharz gebunden bleibt, bis es mit Wasser entfernt wird.

2. Verfahren nach Anspruch 1, wobei:

(a) der Salzgradient ein stufenweiser oder kontinuierlicher Gradient ist;

(b) der Salzgradient 1,6 M bis 0,0 M NaCl umfasst;

(c) das zweite Eluat, das biologisch aktive LT-β-R-Ig-Fusionsproteine umfasst, durch Waschen des HIC-Harzes mit einer 1,5 M NaCl Wäsche gewonnen wird;

(d) der Salzgradient 0,6 M bis 0,0 M Na$_2$SO$_4$ umfasst; oder

(e) der Salzgradient ein stufenweiser Gradient ist, der Na$_2$SO$_4$-Inkremente im Bereich von 0,6 M bis 0,0 M umfasst.

3. Verfahren nach Anspruch 1 oder Anspruch 2, wobei:

(i) das Butyl-HIC-Harz eine Porengröße von 600 bis 750 Å hat;

(ii) das Butyl-HIC-Harz eine Beladungskapazität von etwa 8 mg bis etwa 20 mg Protein/l Harz hat;

(iii) das Butyl-HIC-Harz ein hydroxyliertes Methacrylatgerüst umfasst;

(iv) das zweite Eluat, das biologisch aktive LT-β-R-Ig-Fusionsproteine umfasst, weniger als 2 % biologisch inaktive LT-β-R-Ig-Fusionsproteine umfasst; oder

(iv) das zweite Eluat, das biologisch aktive LT-β-R-Ig-Fusionsproteine umfasst, weniger als 1 % biologisch inaktive LT-β-R-Ig-Fusionsproteine umfasst.

4. Verfahren nach einem der Ansprüche 1 - 3, wobei das Verfahren ferner Folgendes beinhaltet:

Laden des zweiten Eluats, das biologisch aktive LT-β-R-Ig-Fusionsproteine umfasst, auf ein zweites HIC-Harz unter Bedingungen, die es zulassen, dass die biologisch aktiven LT-β-R-Ig-Fusionsproteine das zweite HIC-Harz binden; und

Inkontaktbringen des zweiten HIC-Harzes mit einer Lösung, um ein drittes Eluat zu gewinnen, das ferner in Bezug auf die Anwesenheit von biologisch aktiven LT-β-R-Ig-Fusionsproteinen angereichert ist.

5. Verfahren nach einem der Ansprüche 1 - 3, wobei das Verfahren ferner Folgendes beinhaltet:

Laden des zweiten Eluats auf ein Phenyl-HIC-(hydrophobe Interaktionschromatographie)-Harz, wobei das Harz eine Beladungskapazität von etwa 10-20 g Protein/l Harz und eine Betthöhe von etwa 30 bis 31 cm hat, und Inkontaktbringen des Harzes mit einer Lösung mit einer Strömungsrate von 50 bis 150 cm/h, um ein drittes Eluat, das biologisch unaggregierte, biologisch aktive LT-β-R-Ig-Fusionsproteine umfasst, und ein viertes Eluat zu gewinnen, das aggregierte LT-β-R-Ig-Fusionsproteine umfasst.

6. Verfahren nach Anspruch 5, wobei:

(a) das dritte Eluat, das unaggregierte, biologisch aktive LT-β-R-Ig-Fusionsproteine umfasst, weniger als 2 % aggregierte LT-β-R-Ig-Fusionsproteine umfasst; oder

(b) das dritte Eluat, das unaggregierte, biologisch aktive LT-β-R-Ig-Fusionsproteine umfasst, weniger als 1 % aggregierte LT-β-R-Ig-Fusionsproteine umfasst.

7. Verfahren zum Trennen von biologisch aktiven Lymphotoxin-β-Rezeptor-Immunglobulin-(LT-β-R-Ig)-Fusionsproteinen von biologisch inaktiven LT-β-R-Ig-Fusionsproteinen einschließlich der ersten inaktiven Form des LT-β-R-Ig-Fusionsproteins (inactive-1) und der zweiten inaktiven Form des LT-β-R-Ig-Fusionsproteins (inactive-2), wobei das genannte Verfahren die folgenden Schritte beinhaltet:

i) Laden eines Gemischs, das biologisch aktive LT-β-R-Ig-Fusionsproteine und biologisch inaktive LT-β-R-Ig-Fusionsproteine umfasst, auf ein Butyl-HIC-(hydrophobe Interaktionschromatographie)-Harz unter Bedingungen, die es zulassen, dass die biologisch aktiven LT-β-R-Ig-Fusionsproteine das Butylharz binden;

ii) Inkontaktbringen des Butyl-HIC-Harzes mit einer Lösung, die einen Salzgradienten umfasst, um ein erstes Eluat zu gewinnen, das in Bezug auf die Anwesenheit von biologisch aktiven LT-β-R-Ig-Fusionsproteinen angereichert ist;

iii) Laden des ersten Eluats von Schritt ii) auf ein zweites HIC-Harz unter Bedingungen, die es zulassen, dass die biologisch aktiven LT-β-R-Ig-Fusionsproteine das zweite HIC-Harz binden; und

iv) Inkontaktbringen des zweiten HIC-Harzes mit einer Lösung, um ein zweites Eluat zu gewinnen, das weiter in Bezug auf die Anwesenheit von biologisch aktiven LT-β-R-Ig-Fusionsproteinen angereichert ist,

um dadurch die biologisch aktiven LT-β-R-Ig-Fusionsproteine von den biologisch inaktiven LT-β-R-Ig-Fusionsproteinen zu trennen, und

wobei das inactive-1 LT-β-R-Ig-Fusionsprotein von dem Butyl-hydrophobe Interaktionschromatographie(HIC)-Harz mit einer höheren Salzkonzentration eluiert als das biologisch aktive LT-β-R-Ig-Fusionsprotein, und

wobei das inactive-2 LT-β-R-Ig-Fusionsprotein an das Butylharz gebunden bleibt, bis es mit Wasser entfernt wird.

8. Verfahren nach Anspruch 7, wobei:

(a) der Salzgradient von (ii) entweder ein stufenweiser oder ein kontinuierlicher Gradient ist; oder
(b) das Butyl-HIC-Harz ein hydroxyliertes Methacrylatgerüst umfasst.

9. Verfahren nach Anspruch 7 oder Anspruch 8, wobei:

(i) das Butyl-HIC-Harz eine Porengröße von 600 bis 750 Å hat;
(ii) das erste Eluat weniger als 2 % einer ersten Form des inaktiven LT-β-R-Ig-Fusionsproteins (Inactive-1) umfasst;
(iii) das erste Eluat weniger als 1 % einer ersten Form des inaktiven LT-β-R-Ig-Fusionsproteins (Inactive-1) umfasst;
(iv) das zweite Eluat weniger als 2 % einer zweiten Form des inaktiven LT-β-R-Ig-Fusionsproteins (Inactive-2) umfasst; oder
(v) das zweite Eluat weniger als 1 % einer zweiten Form des inaktiven LT-β-R-Ig-Fusionsproteins (Inactive-2) umfasst.

10. Verfahren nach Anspruch 2, wobei:

(a) der Salzgradient 1,6 M bis 0,0 M NaCl umfasst und die Konzentration von LT-β-R-Ig-Fusionsproteinen in dem Gemisch wenigstens 300 mg/l beträgt; oder
(b) der Salzgradient 0,6 M bis 0,0 M $Na_2SO_4$ umfasst und die Konzentration von LT-β-R-Ig-Fusionsproteinen in dem Gemisch wenigstens 800 mg/l beträgt.

11. Verfahren zum Trennen von biologisch aktiven LT-β-R-Ig-Fusionsproteinen von biologisch inaktiven LT-β-R-Ig-Fusionsproteinen, wobei das genannte Verfahren die folgenden Schritte beinhaltet:

i) Laden eines Gemischs, das biologisch aktive LT-β-R-Ig-Fusionsproteine und biologisch inaktive LT-β-R-Ig-Fusionsproteine umfasst, auf ein Mischharz unter Bedingungen, die es zulassen, dass die biologisch aktiven LT-β-R-Ig-Fusionsproteine das Mischharz binden;

ii) Eluieren der biologisch aktiven LT-β-R-Ig-Fusionsproteine von dem Mischharz von Schritt i) mit einer Lösung, um ein erstes Eluat zu gewinnen, das in Bezug auf die Anwesenheit von biologisch aktiven LT-β-R-Ig-Fusionsproteinen angereichert ist;

iii) Laden des ersten Eluats von Schritt ii) auf HIC-(hydrophobe Interaktionschromatographie)-Harz unter Bedingungen, die es zulassen, dass die biologisch aktiven LT-β-R-Ig-Fusionsproteine das HIC-Harz binden; und

iv) Eluieren der biologisch aktiven LT-β-R-Ig-Fusionsproteine von Schritt iii) mit einer Lösung, um ein zweites Eluat zu gewinnen, das weiter in Bezug auf die Anwesenheit von biologisch aktiven LT-β-R-Ig-Fusionsproteinen angereichert ist,

um dadurch biologisch aktive LT-β-R-Ig-Fusionsproteine von biologisch inaktiven LT-β-R-Ig-Fusionsproteinen zu trennen.

**12.** Verfahren zum Trennen von unaggregierten, biologisch aktiven LT-β-R-Ig-Fusionsproteinen von aggregierten LT-β-R-Ig-Fusionsproteinen, wobei das Verfahren die folgenden Schritte beinhaltet:

i) Laden eines Gemischs, das unaggregierte, biologisch aktive LT-β-R-Ig-Fusionsproteine und aggregierte LT-β-R-Ig-Fusionsproteine umfasst, wobei mehr als etwa 30 % oder etwa 30 % von LT-β-R-Ig-Fusionsproteinen in dem Gemisch aggregiert sind, auf ein Mischharz unter Bedingungen, die es zulassen, dass die biologisch aktiven LT-β-R-Ig-Fusionsproteine das Mischharz binden;

ii) Eluieren der biologisch aktiven LT-β-R-Ig-Fusionsproteine von dem Mischharz von Schritt i) mit einer Lösung, um ein erstes Eluat zu gewinnen, das in Bezug auf die Anwesenheit von biologisch aktiven LT-β-R-Ig-Fusionsproteinen angereichert ist;

iii) Laden des ersten Eluats von Schritt ii) auf ein HIC-(hydrophobe Interaktionschromatographie)-Harz unter Bedingungen, die es zulassen, dass die biologisch aktiven Ig-Funktionsproteine das HIC-Harz binden; und

iv) Eluieren der biologisch aktiven LT-β-R-Ig-Fusionsproteine von Schritt iii) mit einer Lösung, um ein zweites Eluat zu gewinnen, das weiter in Bezug auf die Anwesenheit von biologisch aktiven LT-β-R-Ig-Fusionsproteinen angereichert ist,

um dadurch die unaggregierten, biologisch aktiven LT-β-R-Ig-Fusionsproteine von den aggregierten LT-β-R-Ig-Fusionsproteinen zu trennen.

**13.** Verfahren nach Anspruch 11 oder 12, wobei das Mischharz **dadurch gekennzeichnet ist, dass** es sowohl ionische Interaktionsfähigkeit als auch hydrophobe Interaktionsfähigkeit hat.

**14.** Verfahren nach Anspruch 11 oder 12, wobei das Gemisch auf das Mischharz mit einem pH-Wert von etwa 5,0 geladen wird.

**15.** Verfahren nach Anspruch 14, wobei das geladene Mischharz mit einem Puffer mit einem pH-Wert von etwa 7,0 bis 7,2 vor Schritt (ii) gewaschen wird.

**16.** Verfahren nach Anspruch 13, wobei der Puffer Bis Tris ist.

**17.** Verfahren nach einem der Ansprüche 11 - 16, wobei das HIC-Harz ein Phenylharz ist.

**18.** Verfahren nach Anspruch 17, wobei:

(a) das Phenylharz Phenylsepharose ist;

(b) das biologisch aktive LT-β-R-Ig von dem Phenylharz mit Ammoniumsulfat, vorzugsweise mit 0,3 bis 0,5 M Ammoniumsulfat, eluiert wird; oder

(d) die Strömungsrate des Phenylharzes 50 bis 150 cm/h vorzugsweise etwa 100 cm/h beträgt.

**19.** Verfahren zum Entfernen von wenigstens 97 % aggregierten LT-β-R-Ig-Fusionsproteinen von einem Gemisch, das unaggregierte, biologisch aktive LT-β-R-Ig-Fusionsproteine und aggregierte LT-β-R-Ig-Fusionsproteine umfasst, wobei das genannte Verfahren die folgenden Schritte beinhaltet:

i) Laden des Gemischs auf ein Mischharz unter Bedingungen, die es zulassen, dass die unaggregierten, biologisch aktiven LT-β-R-Ig-Fusionsproteine das Mischharz binden;

ii) Eluieren der unaggregierten, biologisch aktiven LT-β-R-Ig-Fusionsproteine von dem Mischharz von Schritt i) mit einer Lösung, um ein erstes Eluat zu gewinnen, das in Bezug auf die Anwesenheit von unaggregierten, biologisch aktiven LT-β-R-Ig-Fusionsproteinen angereichert ist;

iii) Laden des ersten Eluats von Schritt ii) auf ein HIC-(hydrophobe Interaktionschromatographie)-Harz unter Bedingungen, die es zulassen, dass die unaggregierten, biologisch aktiven LT-β-R-Ig-Fusionsproteine das HIC-Harz binden; und

iv) Eluieren der unaggregierten, biologisch aktiven LT-β-R-Ig-Fusionsproteine von Schritt iii) mit einer Lösung, um ein zweites Eluat zu gewinnen, das weiter in Bezug auf die Anwesenheit von unaggregierten, biologisch aktiven LT-β-R-Ig-Fusionsproteinen angereichert ist,

um dadurch wenigstens 97 % aggregierte LT-β-R-Ig-Fusionsproteine von dem Gemisch zu entfernen, das unaggregierte, biologisch aktive LT-β-R-Ig-Fusionsproteine und aggregierte LT-β-R-Ig-Fusionsproteine umfasst.

**20.** Verfahren nach Anspruch 19, wobei das erste Eluat weniger als 25 %, weniger als 20 %, weniger als 15 % oder etwa 10 % aggregierte LT-β-R-Ig-Fusionsproteine umfasst.

**21.** Verfahren nach Anspruch 19, wobei das zweite Eluat weniger als 2 % aggregierte LT-β-R-Ig-Fusionsproteine oder weniger als 1 % aggregierte LT-β-R-Ig-Fusionsproteine umfasst.

**22.** Verfahren nach einem der Ansprüche 19 - 21, das ferner das Inkontaktbringen des Gemischs mit rekombinantem Protein A (rProtein A) vor Schritt (i) beinhaltet.

**23.** Verfahren nach Anspruch 22, wobei das mit dem rekombinanten Protein A in Kontakt gebrachte Gemisch ein Säugetierzellkulturüberstand ist.

**24.** Verfahren nach Anspruch 23, wobei:

(a) der Säugetierzellkulturüberstand von bei 28°C kultivierten Säugetierzellen gewonnen wird; oder
(b) der Zellkulturüberstand ein nichtionisches Tensid umfasst, wobei das nichtionische Tensid vorzugsweise Triton X-100 ist.

**25.** Verfahren zur Herstellung einer Zusammensetzung, die biologisch aktive LT-β-R-Ig-Fusionsproteine umfasst, wobei die Zusammensetzung weniger als 1 % einer ersten inaktiven Form des LT-β-R-Ig-Fusionsproteins (Inactive-1), weniger als 1 % einer zweiten inaktiven Form des LT-β-R-Ig-Fusionsproteins (Inactive-2) und weniger als 1% aggregierte LT-β-R-Ig-Fusionsproteine umfasst, wobei das genannte Verfahren die folgenden Schritte beinhaltet:

i) Gewinnen eines Zellkulturüberstands von einem Säugetierzellkultursystem, das eine Säugetierwirtszelle umfasst, die mit DNA transformiert ist, die das LT-β-R-Ig-Fusionsprotein transformiert;
ii) Inkontaktbringen des Zellkulturüberstands mit rekombinantem Protein A (rProtein A), um ein LT-β-R-Ig-Funktionsproteingemisch zu erhalten;
iii) Laden des LT-β-R-Ig-Fusionproteingemischs von ii) auf ein Mischharz mit einem pH-Wert von etwa 5,0 unter solchen Bedingungen, dass die LT-β-R-Ig-Fusionsproteine das Mischharz binden;
iv) Waschen des Mischharzes mit einem Puffer mit einem pH-Wert von etwa 7,0 bis 7,2;
v) Inkontaktbringen des Mischharzes von Schritt iii) mit einer Lösung, um ein erstes Eluat zu gewinnen, das in Bezug auf die Anwesenheit von biologisch aktivem LT-β-R-Ig-Fusionsprotein angereichert ist;
vi) Laden des ersten Eluats von Schritt iv) auf ein HIC-(hydrophobe Interaktionschromatographie)-Harz unter Bedingungen, die es zulassen, dass das LT-β-R-Ig-Fusionsprotein das HIC-Harz bindet; und
vii) Inkontaktbringen des HIC-Harzes von Schritt vi) mit einer Lösung, um ein zweites Eluat zu gewinnen, das weiter in Bezug auf die Anwesenheit von biologisch aktivem LT-β-R-Ig-Fusionsprotein angereichert ist, wobei das zweite Eluat weniger als 1% der ersten inaktiven Form des LT-β-R-Ig-Fusionsproteins (Inactive-1), weniger als 1 % der zweiten inaktiven Form des LT-β-R-Ig-Fusionsproteins (Inactive-2) und weniger als 1 % aggregierte LT-β-R-Ig-Fusionsproteine umfasst.

**26.** Verfahren nach einem der Ansprüche 19 - 25, das ferner das Inkontaktbringen des Gemischs mit einem Anionenaustauschmembran-Adsorber vor Schritt (i) beinhaltet.

**Revendications**

**1.** Procédé de séparation de protéines hybrides de récepteur de lymphotoxine β et d'immunoglobuline (LT-β-R-Ig) biologiquement actives de protéines hybrides LT-β-R-Ig biologiquement inactives, y compris la première forme inactive de la protéine hybride LT-β-R-Ig (inactive-1) et la deuxième forme inactive de la protéine hybride LT-β-R-Ig (inactive-2), comprenant :

le chargement d'un mélange comprenant des protéines hybrides LT-β-R-Ig biologiquement actives et des protéines hybrides LT-β-R-Ig biologiquement inactives sur une résine butylique de chromatographie par interactions hydrophobes (HIC) et
la mise en contact de la résine avec une solution comprenant un gradient de sel pour obtenir un premier éluat comprenant des protéines hybrides LT-β-R-Ig biologiquement inactives (inactive-1) et un deuxième éluat comprenant des protéines hybrides LT-β-R-Ig biologiquement actives, et
la protéine hybride LT-β-R-Ig inactive-1 étant éluée à partir de la résine butylique de chromatographie par interactions hydrophobes (HIC) à une concentration de sel plus élevée que la protéine hybride LT-β-R-Ig biologiquement active, et

la protéine hybride LT-β-R-Ig inactive-2 restant liée à la résine butylique jusqu'à ce qu'elle soit éliminée par extraction avec de l'eau.

2. Procédé selon la revendication 1, dans lequel :

(a) le gradient de sel est un gradient discontinu ou un gradient continu ;
(b) le gradient de sel comprend 1,6 M à 0,0 M de NaCl ;
(c) le deuxième éluat comprenant les protéines hybrides LT-β-R-Ig biologiquement actives est obtenu par lavage de la résine HIC avec un produit de lavage contenant 1,5 M de NaCl ;
(d) le gradient de sel comprend 0,6 M à 0,0 M de $Na_2SO_4$ ; ou
(e) le gradient de sel est un gradient discontinu comprenant des incréments de $Na_2SO_4$ de 0,6 M à 0,0 M.

3. Procédé selon la revendication 1 ou la revendication 2, dans lequel :

(i) la résine butylique HIC a un taille de pore de 600 à 750 A ;
(ii) la résine butylique HIC a une capacité de charge d'environ 8 mg à environ 20 mg de protéine/l de résine ;
(iii) la résine butylique HIC comprend un squelette de méthacrylate hydroxylé ;
(iv) le deuxième éluat comprenant des protéines hybrides LT-β-R-Ig biologiquement actives comprend moins de 2 % de protéines hybrides LT-β-R-Ig biologiquement inactives ; ou (iv) le deuxième éluat comprenant des protéines hybrides LT-β-R-Ig biologiquement actives comprend moins de 1 % de protéines hybrides LT-β-R-Ig biologiquement inactives.

4. Procédé selon l'une quelconque des revendications 1 à 3, le procédé comprenant en outre :

le chargement du deuxième éluat comprenant des protéines hybrides LT-β-R-Ig biologiquement actives sur une deuxième résine HIC sous des conditions qui permettent aux protéines hybrides LT-β-R-Ig biologiquement actives de se lier à la deuxième résine HIC ; et
la mise en contact de la deuxième résine HIC avec une solution pour obtenir un troisième éluat enrichi davantage par la présence de protéines hybrides LT-β-R-Ig biologiquement actives.

5. Procédé selon l'une quelconque des revendications 1 à 3, le procédé comprenant en outre :

le chargement du deuxième éluat sur une résine phénylique de chromatographie par interactions hydrophobes (HIC), la résine ayant une capacité de charge d'environ 10 à 20 g de protéine/l de résine et une hauteur de lit d'environ 30 à 31 cm, et
la mise en contact de la résine avec une solution à un débit d'environ 50 à 150 cm/h pour obtenir un troisième éluat comprenant des protéines hybrides LT-β-R-Ig biologiquement actives, biologiquement non agrégées, et un quatrième éluat comprenant des protéines hybrides LT-β-R-Ig agrégées.

6. Procédé selon la revendication 5, dans lequel :

(a) le troisième éluat comprenant des protéines hybrides LT-β-R-Ig biologiquement actives, non agrégées, comprend moins de 2 % de protéines hybrides LT-β-R-Ig agrégées ; ou
(b) le troisième éluat comprenant des protéines hybrides LT-β-R-Ig biologiquement actives, non agrégées, comprend moins de 1 % de protéines hybrides LT-β-R-Ig agrégées.

7. Procédé de séparation de protéines hybrides de récepteur de lymphotoxine β et d'immunoglobuline (LT-β-R-Ig) biologiquement actives de protéines hybrides LT-β-R-Ig biologiquement inactives, y compris la première forme inactive de la protéine hybride LT-β-R-Ig (inactive-1) et la deuxième forme inactive de la protéine hybride LT-β-R-Ig (inactive-2), ledit procédé comprenant les étapes qui consistent à :

i) charger un mélange comprenant des protéines hybrides LT-β-R-Ig biologiquement actives et des protéines hybrides LT-β-R-Ig biologiquement inactives sur une résine butylique de chromatographie par interactions hydrophobes (HIC) sous des conditions qui permettent aux protéines hybrides LT-β-R-Ig biologiquement actives de se lier à la résine butylique ;
ii) mettre en contact la résine butylique HIC avec une solution comprenant un gradient de sel pour obtenir un premier éluat enrichi pour la présence de protéines hybrides LT-β-R-Ig biologiquement actives ;

iii) charger le premier éluat de l'étape ii) sur une deuxième résine HIC sous des conditions qui permettent aux protéines hybrides LT-β-R-Ig biologiquement actives de se lier à la deuxième résine HIC ; et

iv) mettre en contact la deuxième résine HIC avec une solution pour obtenir un deuxième éluat enrichi davantage par la présence de protéines hybrides LT-β-R-Ig biologiquement actives,

pour séparer ainsi les protéines hybrides LT-β-R-Ig biologiquement actives des protéines hybrides LT-β-R-Ig biologiquement inactives, et

la protéine hybride LT-β-R-Ig inactive-1 étant éluée à partir de la résine butylique de chromatographie par interactions hydrophobes (HIC) à une concentration de sel plus élevée que la protéine hybride LT-β-R-Ig biologiquement active, et

la protéine hybride LT-β-R-Ig inactive-2 restant liée à la résine butylique jusqu'à ce qu'elle soit éliminée par extraction avec de l'eau.

8. Procédé selon la revendication 7, dans lequel :

(a) le gradient de sel de (ii) est soit un gradient discontinu, soit un gradient continu ; ou
(b) la résine butylique HIC comprend un squelette de méthacrylate hydroxylé.

9. Procédé selon la revendication 7 ou la revendication 8, dans lequel :

(i) la résine butylique HIC a une taille de pore de 600 à 750 A ;
(ii) le premier éluat comprend moins de 2 % d'une première forme de la protéine hybride LT-β-R-Ig inactive (inactive-1) ;
(iii) le premier éluat comprend moins de 1 % d'une première forme de la protéine hybride LT-β-R-Ig inactive (inactive-1) ;
(iv) le deuxième éluat comprend moins de 2 % d'une deuxième forme de la protéine hybride LT-β-R-Ig inactive (inactive-2) ; ou
(v) le deuxième éluat comprend moins de 1 % d'une deuxième forme de la protéine hybride LT-β-R-Ig inactive (inactive-2).

10. Procédé selon la revendication 2, dans lequel :

(a) le gradient de sel comprend 1,6 M à 0,0 M de NaCl et la concentration de protéines hybrides LT-β-R-Ig dans le mélange est d'au moins 300 mg/l ; ou
(b) le gradient de sel comprend 0,6 M à 0,0 M de $Na_2SO_4$ et la concentration de protéines hybrides LT-β-R-Ig dans le mélange est d'au moins 800 mg/l.

11. Procédé de séparation de protéines hybrides LT-β-R-Ig biologiquement actives de protéines hybrides LT-β-R-Ig biologiquement inactives, ledit procédé comprenant les étapes qui consistent à :

i) charger un mélange comprenant des protéines hybrides LT-β-R-Ig biologiquement actives et des protéines hybrides LT-β-R-Ig biologiquement inactives sur une résine mode mixte sous des conditions qui permettent aux protéines hybrides LT-β-R-Ig biologiquement actives de se lier à la résine mode mixte ;
ii) éluer les protéines hybrides LT-β-R-Ig biologiquement actives à partir de la résine mode mixte de l'étape i) avec une solution pour obtenir un premier éluat enrichi pour la présence de protéines hybrides LT-β-R-Ig biologiquement actives ;
iii) charger le premier éluat de l'étape ii) sur une résine de chromatographie par interactions hydrophobes (HIC) sous des conditions qui permettent aux protéines hybrides LT-β-R-Ig biologiquement actives de se lier à la résine HIC ; et
iv) éluer les protéines hybrides LT-β-R-Ig biologiquement actives de l'étape iii) avec une solution pour obtenir un deuxième éluat enrichi davantage par la présence de protéines hybrides LT-β-R-Ig biologiquement actives, pour séparer ainsi les protéines hybrides LT-β-R-Ig biologiquement actives des protéines hybrides LT-β-R-Ig biologiquement inactives.

12. Procédé de séparation de protéines hybrides LT-β-R-Ig biologiquement actives, non agrégées, de protéines hybrides LT-β-R-Ig agrégées, le procédé comprenant les étapes qui consistent à :

i) charger un mélange comprenant des protéines hybrides LT-β-R-Ig biologiquement actives, non agrégées, et des protéines hybrides LT-β-R-Ig agrégées, plus d'environ 30 % ou environ 30 % des protéines hybrides LT-β-R-Ig dans le mélange étant agrégées, sur une résine mode mixte sous des conditions qui permettent aux protéines hybrides LT-β-R-Ig biologiquement actives de se lier à la résine mode mixte ;

ii) éluer les protéines hybrides LT-β-R-Ig biologiquement actives à partir de la résine mode mixte de l'étape i) en utilisant une solution pour obtenir un premier éluat enrichi pour la présence de protéines hybrides LT-β-R-Ig biologiquement actives ;

iii) charger le premier éluat de l'étape ii) sur une résine de chromatographie par interactions hydrophobes (HIC) sous des conditions qui permettent aux protéines hybrides d'Ig biologiquement actives de se lier à la résine HIC ; et

iv) éluer les protéines hybrides LT-β-R-Ig biologiquement actives de l'étape iii) en utilisant une solution pour obtenir un deuxième éluat enrichi davantage par la présence de protéines hybrides LT-β-R-Ig biologiquement actives,

en séparant ainsi les protéines hybrides LT-β-R-Ig biologiquement actives non agrégées des protéines hybrides LT-β-R-Ig agrégées.

13. Procédé selon la revendication 11 ou 12, dans lequel la résine mode mixte est **caractérisée en ce qu'**elle présente à la fois une capacité d'interactions ioniques et une capacité d'interactions hydrophobes.

14. Procédé selon la revendication 11 ou 12, dans lequel le mélange est chargé sur la résine mode mixte à un pH d'environ 5,0.

15. Procédé selon la revendication 14, dans lequel la résine mode mixte chargée est lavée avec un tampon ayant un pH d'environ 7,0 à 7,2 avant l'étape (ii).

16. Procédé selon la revendication 13, dans lequel le tampon est un tampon bis-tris.

17. Procédé selon l'une quelconque des revendications 11 à 16, dans lequel la résine HIC est une résine phénylique.

18. Procédé selon la revendication 17, dans lequel :

(a) la résine phénylique est la phényl-sépharose ;
(b) la LT-β-R-Ig biologiquement active est éluée à partir de la résine phénylique avec du sulfate d'ammonium, et préférablement avec 0,3 à 0,5 M de sulfate d'ammonium ; ou
(c) le débit de la résine phénylique est de 50 à 150 cm/h, et préférablement d'environ 100 cm/h.

19. Procédé d'élimination d'au moins 97 % des protéines hybrides LT-β-R-Ig agrégées d'un mélange comprenant des protéines hybrides LT-β-R-Ig biologiquement actives, non agrégées, et des protéines hybrides LT-β-R-Ig agrégées, ledit procédé comprenant les étapes qui consistent à :

i) charger le mélange sur une résine mode mixte sous des conditions qui permettent aux protéines hybrides LT-β-R-Ig biologiquement actives, non agrégées, de se lier à la résine mode mixte ;

ii) éluer les protéines hybrides LT-β-R-Ig biologiquement actives, non agrégées, à partir de la résine mode mixte de l'étape i) avec une solution pour obtenir un premier éluat enrichi pour la présence de protéines hybrides LT-β-R-Ig biologiquement actives, non agrégées ;

iii) charger le premier éluat de l'étape ii) sur une résine de chromatographie par interactions hydrophobes (HIC) sous des conditions qui permettent aux protéines hybrides LT-β-R-Ig biologiquement actives, non agrégées, de se lier à la résine HIC ; et

iv) éluer les protéines hybrides LT-β-R-Ig biologiquement actives, non agrégées, de l'étape iii) avec une solution pour obtenir un deuxième éluat enrichi davantage par la présence de protéines hybrides LT-β-R-Ig biologiquement actives, non agrégées,

pour éliminer ainsi au moins 97 % des protéines hybrides LT-β-R-Ig agrégées du mélange comprenant des protéines hybrides LT-β-R-Ig biologiquement actives, non agrégées, et des protéines hybrides LT-β-R-Ig agrégées.

20. Procédé selon la revendication 19, dans lequel le premier éluat comprend moins de 25 %, moins de 20 %, moins de 15 %, ou environ 10 % de protéines hybrides LT-β-R-Ig agrégées.

21. Procédé selon la revendication 19, dans lequel le deuxième éluat comprend moins de 2 % de protéines hybrides LT-β-R-Ig agrégées ou moins de 1 % de protéines hybrides LT-β-R-Ig agrégées.

**22.** Procédé selon l'une quelconque des revendications 19 à 21, comprenant en outre la mise en contact du mélange avec une Protéine A recombinante (Protéine Ar) avant l'étape (i).

**23.** Procédé selon la revendication 22, dans lequel le mélange mis en contact avec la Protéine A recombinante est un surnageant de culture de cellules mammifères.

**24.** Procédé selon la revendication 23, dans lequel :

(a) le surnageant de culture de cellules mammifères est obtenu à partir de cellules mammifères mises en culture à 28 ° C ; ou
(b) le surnageant de culture de cellules comprend un tensioactif non ionique, et le tensioactif non ionique est préférablement le Triton X-100.

**25.** Procédé de préparation d'une composition comprenant des protéines hybrides LT-β-R-Ig biologiquement actives, la composition comprenant moins de 1 % d'une première forme inactive de la protéine hybride LT-β-R-Ig (inactive-1), moins de 1 % d'une deuxième forme inactive de la protéine hybride LT-β-R-Ig (inactive-2), et moins de 1 % de protéines hybrides LT-β-R-Ig agrégées, ledit procédé comprenant les étapes qui consistent à :

(i) obtenir un surnageant de culture de cellules à partir d'un système de culture de cellules mammifères comprenant une cellule hôte mammifère transformée avec un ADN codant pour la protéine hybride LT-β-R-Ig ;
(ii) mettre en contact le surnageant de culture de cellules avec une Protéine A recombinante (Protéine Ar) pour obtenir un mélange de protéines hybrides LT-β-R-Ig ;
(iii) charger le mélange de protéines hybrides LT-β-R-Ig de l'étape ii) sur une résine mode mixte à un pH d'environ 5,0 sous des conditions telles que les protéines hybrides LT-β-R-Ig se lient à la résine mode mixte ;
(iv) laver la résine mode mixte avec un tampon ayant un pH d'environ 7,0 à 7,2 ;
(v) mettre en contact la résine mode mixte de l'étape iii) avec une solution pour obtenir un premier éluat enrichi pour la présence d'une protéine hybride LT-β-R-Ig biologiquement active ;
(vi) charger le premier éluat de l'étape iv) sur une résine de chromatographie par interactions hydrophobes (HIC) sous des conditions qui permettent à la protéine hybride LT-β-R-Ig de se lier à la résine HIC ; et
(vii) mettre en contact la résine HIC de l'étape vi) avec une solution pour obtenir un deuxième éluat enrichi davantage par la présence d'une protéine hybride LT-β-R-Ig biologiquement active,
le deuxième éluat comprenant moins de 1 % de la première forme inactive de la protéine hybride (inactive-1), moins de 1 % de la deuxième forme inactive de la protéine hybride LT-β-R-Ig (inactive-2), et moins de 1 % de protéines hybrides LT-β-R-Ig agrégées.

**26.** Procédé selon l'une quelconque des revendications 19 à 25, comprenant en outre la mise en contact du mélange avec un adsorbeur à membrane échangeuse d'anions avant l'étape (i).

# Figure 1: Cycle 1-1 Overview

| Titer **300 mg/L** | |
|---|---|
| Inactive-1 | 6 % |
| Inactive-2 | 10 % |
| Aggregate | 15 % |

Harvest Microfiltration
↓
Protein-A Capture
↓

**HIC-1**
Capacity **8 g/L** of resin

↓
Low pH VI
↓

**HIC-2**
Capacity 20 g/L of resin

↓
VF
↓
UF/DF

EP 2 260 054 B1

# Figure 2: HIC-1 Step (Butyl) (Cycle 1-1)

| Process | Capacity (g/L) | Salt Concentration (M; NaCl) | | | | Yield (%) |
| | | Load | Wash | Elution | Strip | |
|---------|----------------|------|------|---------|------------|-----------|
| Early | 8.0 | 1.65 | 1.55 | 0.70 | 0.0 ($H_2O$) | 70 |

■Inactive-1 & Inactive-2 reduced to <1.0% in the eluate fraction

EP 2 260 054 B1

# Figure 3: HPLC Analysis of Process Intermediates (Cycle 1-1)

## HIC-HPLC:

Resolves Inactive –1 & Inactive-2
from monomer + aggregate

## SE-HPLC:

Resolves aggregate from monomer
and LMW material

$$\%\text{Active Monomer} = (\text{Main Peak Area})_{\text{HIC-HPLC}} \times (\text{Main Peak Area})_{\text{SE-HPLC}}$$

# Figure 4

# Figure 5: Cycle 1-2 Overview

| Optimized Titer **800 mg/L** | |
|---|---|
| Inactive-1 | 5 % |
| Inactive-2 | 14 % |
| Aggregate | 22 % |

Harvest Centrifugation
↓
Protein-A Capture
↓
Low pH VI
↓

**HIC-1**
**Capacity 20 g/L of resin**
↓
**HIC-2**
**Capacity 20 g/L of resin**
↓
VF
↓
UF/DF

**HIC-1**
Clearance of Inactive-1 & -2

**HIC-2**
Clearance of aggregate

EP 2 260 054 B1

# Figure 6

## Strategy A. Step Wash (Na₂SO4)

Legend: —— mAU (A280 nm)    —— Conductivity (mS/cm)

Incompletely resolved wash peak

Load    Wash    Elution    Strip    Regen.

| Column Fraction | HIC-HPLC Assay | | | Yield |
| --- | --- | --- | --- | --- |
| | Monomer+ Aggregate | Inactive-1 | Inactive-2 | |
| Load | 88.6 | 5.2 | 6.2 | N/A |
| Eluate | 98.9 | 1.1 | 0.0 | 75.2 |

## B. Step + Gradient Wash (Na₂SO₄)

Legend: —— mAU (A280 nm)    —— Conductivity (mS/cm)

Well-resolved Wash peak

Load    Wash    Elution    Strip    Regen.

| Column Fraction | HIC-HPLC Assay | | | Yield |
| --- | --- | --- | --- | --- |
| | Monomer+ Aggregate | Inactive-1 | Inactive-2 | |
| Load | 90.1 | 4.6 | 5.3 | N/A |
| Eluate | 98.8 | 1.2 | 0.0 | 70.4 |

# Figure 7

| Step | Yield (%) | | Active Monomer | Inactive-1 | Inactive-2 | Aggregate | Clearance (Log10) | | | Bioassay |
|---|---|---|---|---|---|---|---|---|---|---|
| | O.D. | Active Monomer | (%) | (%) | (%) | (%) | HCP | DNA | MMV | Rel. potency (%) |
| MabSelect SuRe | 95 | N.D. | 63 | 5 | 14 | 23 | 2.8 | N.D. | 0.9-1.8 | N.D. |
| HIC-1 (Butyl) | 59 | 74 | 79 | 0 | 0 | 22 | 1.1 | >1.1 | 2.6 | N.D. |
| HIC-2 (Phenyl) | 70 | 89 | 99 | 0 | 0 | 0.9 | 1.5 | N.D. | 1.0 | 93 |

# Figure 8

## A. Effect of Load

## B. Effect of Bed Height and Velocity

| Bed Height (cm): | 30 | 20 | 40 | 20 | 30 |
|---|---|---|---|---|---|
| Velocity (cm/h): | 100 | 50 | 50 | 33 | 50 |

- Parameters providing optimal resolution of monomer and aggregate:
    - Bed height   30.61 cm
    - Flow rate   50 cm/h
    - Optimal load range with above parameters is 10 – 17 g/L of resin

## Figure 9

### Cycle-2

| Titer **1350 mg/L** | |
|---|---|
| Inactive-1 | 6 % |
| Inactive-2 | 13-14 % |
| Aggregate | 35 % |

Adjustment to pH 5 - - - - - - ▶

Triton X-100 - - - - - - ▶

↓ Harvest Centrifugation

↓ Protein-A Capture

↓ Low pH VI

Q Membrane Adsorber - - - - - - ▶ ↓

**Mixed Mode**
**Capacity 31 g/L of resin**

**Capto MMC**
Clearance of Inactive-1 & Aggregate.

↓

**Phenyl**
**Capacity 15 g/L of resin**

**Phenyl (30 cm)**
Clearance of aggregate & Inactive-2
Resolution optimized

↓ VF

↓ UF/DF

Figure 10

## Figure 11

A

Aggregate in Eluate (%)

Material in Wash (%)

Active Monomer in Eluate (% Yield)

Lo ←——→ Hi   Lo ←——→ Hi

Wash Spec. Range   Column Load Range (g/L)

B IEX 2nd-Column Operating Conditions and Performance

| Clearance Required | Column Loading Spec. | Wash Solution Spec. | Product Yield |
|---|---|---|---|
| Inactive-1 alone | ■ Broad range | ■ Broad range | ■ High<br>■ Consistent |
| Inactive-1 and Aggregate | ■ 50% reduction in operating range | ■ 50% reduction in operating range<br>■ Targeted to lower yielding conditions | ■ 10-50% reduction<br><br>■ Variable |

EP 2 260 054 B1

Figure 12

EP 2 260 054 B1

# Figure 13

EP 2 260 054 B1

**Summary: Key Performance Features of the Modified Downstream Process in 15k Manufacturing**

| Day of Harvest | Number of Batches at 15k Scale | Av. Process Yield: Active Monomer | Variability in 2nd Column Yield | Variability in 3rd Column Yield | Max. Product Reltd. Impurities in DS | Viral Clearance Potential[B] |
|---|---|---|---|---|---|---|
| 14 | 3 | 28%[A] | 9% | 10% | Aggregate: 0.3%<br>Inactive: <LLOQ | 29 $Log_{10}$ X-MLV<br>9 $Log_{10}$ MMV |
| 16 | 4 | 49% | 10% | 6% | Aggregate: 0.4%<br>Inactive: <LLOQ | TBD<br>TBD |

[A] Process operated with low loading and high cycling of the 3rd column to gurantee PQ upon initial scale-up

[B] Determined by benchscale spiking studies using retains of GMP intermediates and model viruses

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20020039580 A, Browning
- WO 0036092 A
- WO 2008112325 A
- US 08505606 B
- US 60029060 B
- US 20020197254 A
- US 7001598 B
- US 3917527 A
- US 4000098 A
- US 6159730 A
- US 5736137 A
- US 5658570 A
- US 6413777 B
- US 60331481 B
- US 6193980 B

- US 5116964 A
- US 5225538 A
- US 5399163 A
- US 5383851 A
- US 5312335 A
- US 5064413 A
- US 4941880 A
- US 4790824 A
- US 4596556 A
- US 4487603 A
- US 4486194 A
- US 4447233 A
- US 4447224 A
- US 4439196 A
- US 4475196 A
- US 7255854 B

### Non-patent literature cited in the description

- **BROWNING et al.** *J. Immunol.,* 1997, vol. 159 (7), 3288-3298
- **MORIMOTO, K. et al.** *L Biochem. Biophys. Meth.,* 1992, vol. 24, 107
- **ER-EL. Z. et al.** *Biochem. Biophys. Res. Comm.,* 1972, vol. 49, 383
- **ULBRICH,V. et al.** *Coll. Czech. Chem. Commum.,* 1964, vol. 9, 1466
- **RIDGWAY, A. A. G.** Mammalian Expression Vectors. 1988, 470-472
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157

- **JONES.** *Genetics,* 1977, vol. 85, 12
- **BERGE et al.** *J. Pharm. Sci.,* 1977, vol. 66, 1-19
- Remington: The Science and Practice of Pharmacy. Lippincott, Williams &amp, 2000
- **ANSEL et al.** Pharmaceutical Dosage Forms and Drug Delivery Systems. Lippincott Williams & Wilkins Publishers, 1999
- Handbook of Pharmaceutical Excipients. American Pharmaceutical Association, 2000
- Sustained and Controlled Release Drug Delivery Systems. Marcel Dekker, Inc, 1978
- **WHITAKER et al.** *Ann. Neural.,* 1995, vol. 38 (4), 635-632